(19) Europäisches Patentamt / European Patent Office / Office européen des brevets

(11) **EP 4 301 868 B1**

(12) **EUROPEAN PATENT SPECIFICATION**

(45) Date of publication and mention
of the grant of the patent:
**06.05.2026 Bulletin 2026/19**

(21) Application number: **22763911.9**

(22) Date of filing: **01.03.2022**

(51) International Patent Classification (IPC):
*C12Q 1/68* (2018.01)    *C12N 15/00* (2006.01)
*C12N 5/00* (2006.01)    *C12Q 1/34* (2006.01)
*C07H 21/04* (2006.01)    *C12N 9/22* (2006.01)
*C12N 15/10* (2006.01)    *C12N 15/86* (2006.01)
*C12Q 1/6806* (2018.01)

(52) Cooperative Patent Classification (CPC):
(C-Sets available)
**C12N 15/1065; C12N 9/22; C12N 15/102;
C12N 15/1075; C12N 15/1096; C12N 15/86;
C12Q 1/6806;** C12N 2310/20; C12N 2740/16043
(Cont.)

(86) International application number:
**PCT/US2022/018364**

(87) International publication number:
**WO 2022/187262 (09.09.2022 Gazette 2022/36)**

(54) **METHODS AND COMPOSITIONS FOR COMBINATORIAL TARGETING OF THE CELL TRANSCRIPTOME**

VERFAHREN UND ZUSAMMENSETZUNGEN FÜR KOMBINATORISCHES TARGETING DES ZELLTRANSKRIPTOMS

PROCÉDÉS ET COMPOSITIONS POUR LE CIBLAGE COMBINATOIRE DU TRANSCRIPTOME CELLULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.03.2021 US 202163154985 P**
**03.02.2022 US 202263306343 P**

(43) Date of publication of application:
**10.01.2024 Bulletin 2024/02**

(73) Proprietors:
• **New York Genome Center, Inc.**
**New York, NY 10013 (US)**
• **New York University**
**New York, NY 10012-1091 (US)**

(72) Inventors:
• **SANJANA, Neville, E.**
**New York, NY 10012 (US)**
• **WESSELS, Hans-Hermann**
**Brooklyn, NY 11205 (US)**
• **MENDEZ-MANCILLA, Alejandro**
**Brooklyn, NY 11216 (US)**
• **SATIJA, Rahul**
**New York, NY 10012 (US)**

(74) Representative: **Appleyard Lees IP LLP**
**G Mill**
**Dean Clough Industrial Park**
**Halifax HX3 5AH (GB)**

(56) References cited:
**WO-A1-2016/182959    WO-A1-2018/005691
US-A1- 2020 318 172**

**(Cont. next page)**

- **DIXIT ATRAY ET AL: "Perturb-Seq: Dissecting Molecular Circuits with Scalable Single-Cell RNA Profiling of Pooled Genetic Screens", CELL, ELSEVIER, AMSTERDAM NL, vol. 167, no. 7, 15 December 2016 (2016-12-15), pages 1853, XP029850713, ISSN: 0092-8674, DOI: 10.1016/ J.CELL.2016.11.038**
- **WESSELS HANS-HERMANN ET AL: "Efficient combinatorial targeting of RNA transcripts in single cells with Cas13 RNA Perturb-seq", BIORXIV, 2 February 2022 (2022-02-02), XP093022884, Retrieved from the Internet <URL:https://www.biorxiv.org/content/10.1101/ 2022.02.02.478894v1.full.pdf> [retrieved on 20230210], DOI: 10.1101/2022.02.02.478894**
- **MCCARTY NICHOLAS S., GRAHAM ALICIA E., STUDENÁ LUCIE, LEDESMA-AMARO RODRIGO: "Multiplexed CRISPR technologies for gene editing and transcriptional regulation", NATURE COMMUNICATIONS, vol. 11, no. 1281, March 2020 (2020-03-01), pages 1 - 13, XP055736214**
- **REPLOGLE ET AL.: "Combinatorial single- cell CRISPR screens by direct guide RNA capture and targeted sequencing", NAT BIOTECHNOL., vol. 38, no. 8, August 2020 (2020-08-01), pages 954 - 961, XP037211717, DOI: 10.1038/ s41587-020-0470-y**
- **LIAO ET AL.: "Modular one-pot assembly of CRISPR arrays enables library generation and reveals factors influencing crRNA biogenesis", NAT COMMUN, vol. 10, no. 2948, 2019, pages 1 - 14, XP055738525, DOI: 10.1038/ s41467-019-10747-3**
- **BURGESS DARREN J.: "Genetic screens: Combining CRISPR perturbations and RNA-seq", NAT REV GENET, vol. 18, no. 2, 2017, pages 67, XP055968301**
- **ZHU SHIYOU, CAO ZHONGZHENG, LIU ZHIHENG, HE YUAN, WANG YINAN, YUAN PENGFEI, LI WEI, TIAN FENG, BAO YING, WEI WENSHENG: "Guide RNAs with embedded barcodes boost CRISPR-pooled screens", GENOME BIOLOGY, vol. 20, no. 1, 2019, pages 1 - 12, XP055968304**

Remarks:

The complete document including Reference Table(s) and the Sequence Listing(s) can be downloaded from the EPO website

(52) Cooperative Patent Classification (CPC): (Cont.)

C-Sets
**C12N 15/1075, C12Q 2521/301, C12Q 2521/107, C12Q 2525/173, C12Q 2525/155, C12Q 2525/301, C12Q 2537/143, C12Q 2535/122, C12Q 2563/179; C12N 15/1096, C12Q 2521/301, C12Q 2521/107, C12Q 2525/173, C12Q 2525/155, C12Q 2525/301, C12Q 2537/143, C12Q 2535/122, C12Q 2563/179; C12Q 1/6806, C12Q 2521/301, C12Q 2521/107, C12Q 2525/173, C12Q 2525/155, C12Q 2525/301, C12Q 2537/143, C12Q 2535/122, C12Q 2563/179**

**Description**

STATEMENT OF GOVERNMENT SUPPORT

This invention was made with government support under DP2HG010099, DP2HG009623-01, RM1HG011014-01, and R01CA218668 awarded by the National Institutes of Health. The government has certain rights in the invention.

BACKGROUND

Recent technological advances that couple pooled genetic perturbations with scRNA-seq or multimodal characterization (i.e. Perturb-Seq, CROP-seq, CRISP-seq, and ECCITE-seq) promise to transform our understanding of gene function. In particular, the ability to perform combinatorial perturbations represents an opportunity to decode complex regulatory networks, with pioneering work demonstrating the ability to identify epistasis and other genetic interactions. However, there are specific technical and analytical challenges associated with pooled single-cell screens which are exacerbated when considering combinatorial perturbations. For example, undetected or incorrectly assigned sgRNAs can affect up to 20% of cells, but this is compounded when multiple independent sgRNAs are introduced and independently detected in each cell. Moreover, perturbations introduced by Cas9 are not uniformly efficient, and a considerable fraction of targeted cells may exhibit no phenotypic effects of perturbation. Therefore, when performing two or more simultaneous perturbations, the fraction of cells where all perturbations are both successfully introduced and successfully detected can decrease dramatically.

WO2018005691A1 discloses a genetic screening method using guide RNAs that contain a barcode and a poly(A) tail, which allows CRISPR-based genetic perturbations to be tracked at the single-cell level by RNA sequencing. The method enables identification of the effects of specific genetic modifications on gene expression in individual cells.

Dixit, A., Parnas, O., Li, B., et al., Cell, 167(7):1853-1866.e17, 2016, describes the Perturb-seq technique, which combines pooled CRISPR-mediated gene perturbations with single-cell RNA sequencing. This approach assigns transcriptional profiles to individual genetic perturbations and allows analysis of genetic interactions in cell populations.

WO2016182959A1 discloses methods for CRISPR/Cas9-mediated gene editing in stem cells, involving transient treatment of the cells with viability-enhancing compounds before and/or after delivery of CRISPR/Cas9 components. The document also describes the use of guide RNAs with 5' caps and 3' poly(A) tails to affect editing efficiency, cell viability, and engraftment.

What is needed is improved compositions and methods for introducing multiple genetic perturbations in a cell.

SUMMARY OF THE INVENTION

The subject-matter for which protection is sought is defined by the attached claims; any embodiments described herein that are not encompassed by the claims are provided for illustrative purposes only.

In one aspect, provided herein is a nucleic acid comprising a CRISPR array comprising one or more crRNA sequences, each crRNA comprising a direct repeat (DR) sequence and a gRNA sequence, and a 5' direct repeat (DR) sequence linked to a barcode guide RNA (bcgRNA), the bcgRNA comprising from 5' to 3' (a) a barcode sequence; and (b) a reverse-transcription handle.

In certain embodiments, the bcgRNA comprises from 5' to 3' (a) a PCR handle; (b) a barcode sequence; and (c) a reverse-transcription handle. In certain embodiments, the CRISPR array comprises one, two, three, or more crRNA sequences. In certain embodiments, each of the one or more crRNA sequences comprise a gRNA that comprises an at least 20 nucleotide sequence that is complementary to a target RNA sequence. In certain embodiments, each of the one or more crRNA sequences comprise a gRNA that is a 23-nucleotide sequence that is complementary to a target RNA sequence. In certain embodiments, each of the crRNA present in the CRISPR array has a different gRNA sequence. In certain embodiments, two or more of the crRNA present in the CRISPR array comprise the same gRNA sequence. In certain embodiments, each of the crRNA sequences present in the CRISPR array is specific for a different region of a target transcript. In certain embodiments, the CRISPR array comprises crRNA sequences having guide RNA (gRNA) sequences that target multiple transcripts. In certain embodiments, the bcgRNA is downstream (3') of the one or more crRNA sequences of the CRISPR array. In certain embodiments, the bcgRNA is upstream (5') of the one or more crRNA sequences of the CRISPR array. In certain embodiments, the direct repeat is capable of binding a CRISPR-Cas13 enzyme, optionally Cas13d. In certain embodiments, the reverse-transcription handle comprises a polyA sequence, a CS1, or a CS2. In certain embodiments, the barcode comprises 8 to 15 nucleotides. In certain embodiments, CRISPR array further comprises a stabilizing RNA element at its 3' end. In certain embodiments, the stabilizing RNA element is a MALAT1, NEAT1 (MENß), spnpreQ$_1$, ZIKV xrRNA1, mpknot, or evopreQ$_1$ element.

In another aspect, provided herein is an expression cassette comprising a nucleic acid according comprising a CRISPR array comprising one or more crRNA sequences, each crRNA comprising a direct repeat (DR) sequence and a gRNA sequence, and a 5' direct repeat (DR) sequence linked to a barcode guide RNA (bcgRNA), the bcgRNA comprising from 5' to 3' (a) a barcode sequence; and (b) a reverse-transcription handle. In certain embodiments, the expression cassette includes a bcgRNA comprising from 5' to 3' (a) a PCR handle; (b) a barcode sequence; and (c) a reverse-transcription handle. The vector comprising the expression cassette can be a non-viral vector or a viral vector. In certain embodiments, the non-viral vector is a plasmid. In a further embodiment, the plasmid comprises a sequence encoding a CRISPR-Cas enzyme, optionally a Cas13 or a Cas12 enzyme. In certain embodiments, the Cas enzyme is a type VI CRISPR-Cas enzyme. In certain embodiments the vector is viral vector that is a retrovirus vector, a lentivirus vector, an adenovirus vector, or an adeno-associated virus vector.

In one aspect, provided herein is a host cell comprising a nucleic acid comprising a CRISPR array as described and a CRISPR-Cas enzyme, optionally a Cas13 or a Cas12 enzyme.

In another aspect, provided herein is a method of introducing one or more gene perturbations in a single cell transcriptome, comprising culturing the host cell. In certain embodiments, a method of performing gene perturbation profiling is provided, wherein the method comprises (a) obtaining the host cell; (b) isolating RNA from the cell; (c) performing reverse-transcription comprising contacting the RNA with a primer specific for the reverse-transcription handle; (d) identifying the barcode sequence; and (e) detecting expression of one or more transcripts or gene products, wherein CRISPR-Cas enzyme introduces one or more perturbations in the cell transcriptome. In certain embodiments, a method of performing gene perturbation profiling is provided, wherein the method comprises (a) obtaining the host cell according to claim 21, labeling the cell with a fluorophore-conjugated antibody, and sorting the cell using flow cytometry; (b) isolating RNA from the cell; (c) performing reverse-transcription comprising contacting the RNA with a primer specific for the reverse-transcription handle; (d) identifying the barcode sequence; and (e) detecting expression of one or more transcripts or gene products, wherein CRISPR-Cas enzyme introduces one or more perturbations in the cell transcriptome. In a further embodiment, the method comprises a step (d) identifying the barcode sequence that includes amplifying the barcode sequence using a primer specific for the PCR handle. In certain embodiments, the method includes (e) detecting expression of one or more transcripts or gene products that includes one or more of flow cytometric analysis, cell-hashing, single-cell sequencing analysis, single cell RNA sequencing (scRNA-seq), Perturb-seq, CROP-seq, CRISP-seq, ECCITE-seq, cellular indexing of transcriptomes and epitopes (CITE-seq).

Other aspects and advantages of the invention will be readily apparent from the following detailed description of the invention.

BRIEF DESCRIPTION OF THE DRAWINGS

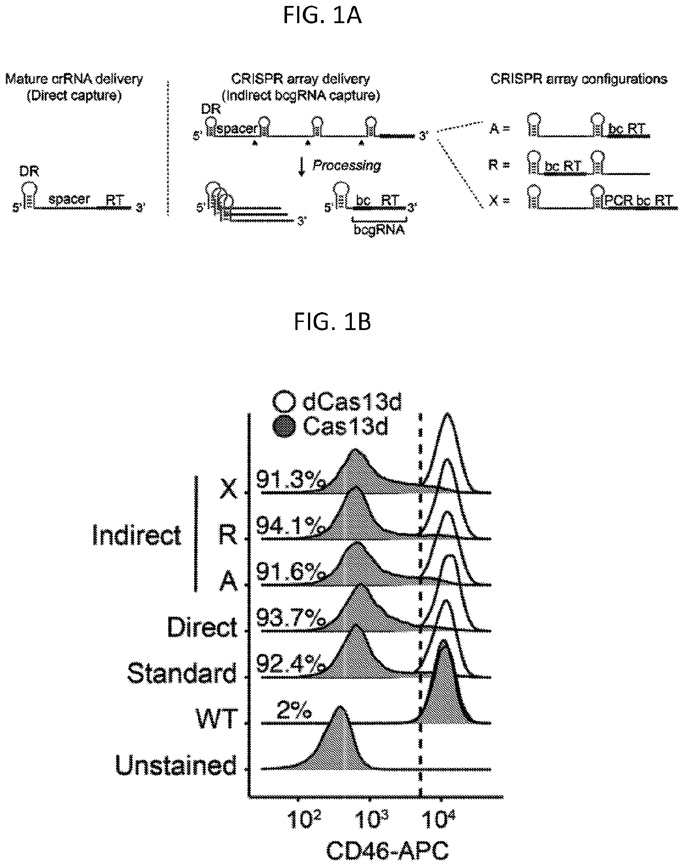

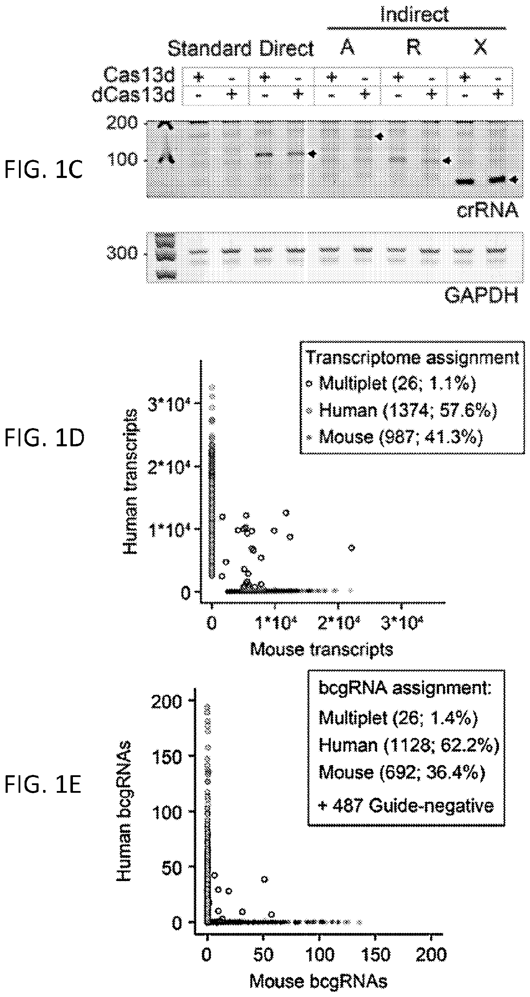

FIG. 1A - FIG. 1E show efficient capture of gRNAs for Cas13 RNA Perturb-seq (CaRPool-seq). (FIG. 1A) Scheme of direct and indirect array-based gRNA capture approaches. Four approaches have been assessed. Direct capture uses a reverse transcription (RT) handle added directly downstream to the spacer RNA. For the indirect capture method, a barcode guide RNA (bcgRNA) is captured as part of a CRISPR array. Three different CRISPR array configurations (A, R and X) have been tested (RT = Reverse transcription handle, bc = Barcode, PCR = PCR primer annealing site, bcgRNA = barcode guide RNA, A = Array, R = Reversed Array configuration, X = Extra PCR handle). (FIG. 1B) Density plots showing the CD46-APC signal upon Cas13d-mediated CD46 knockdown and dCas13d-mediated controls using the four CaRPool-seq configurations described in (FIG. 1A), as well as standard gRNA. The CS1 RT handle was used in all cases. (FIG. 1C) PCR amplicons of reverse-transcribed crRNAs from lentivirally infected cells used in FIG. 1B. Direct capture (expected 109 bp), R-type array (expected 99 bp), and X-type array (expected 52 bp) allowed for reverse transcription and amplification at varying efficiency. X-type arrays are independent of template switching success and show the highest detection sensitivity. Type-X band intensities were equal between active RfxCas13 and inactive RfxdCas13d suggesting that Cas13 RNA targeting activity does not reduce available bcgRNA amounts. A-type arrays showed a faint band at the length of unprocessed CRISPR arrays (159 bp). (FIG. 1D) Species mixing experiment profiling 2,387 HEK293FT-Cas13d or mouse NIH/3T3-Cas13d cells lentivirally transduced with CRISPR array virus. The CRISPR array includes a non-targeting gRNA and a bcgRNA in X-type configuration. The plot shows the number of transcripts associated with each cell barcode. Datapoint shading and boxed labels are assigned based on transcriptome classification (>90% species-specificity required for assignment). (FIG. 1E) Number of bcgRNAs associated with each cell barcode. Datapoint shading are based on transcriptome classification, and boxed labels are based on observed gRNA (>90% species-specificity required for assignment).

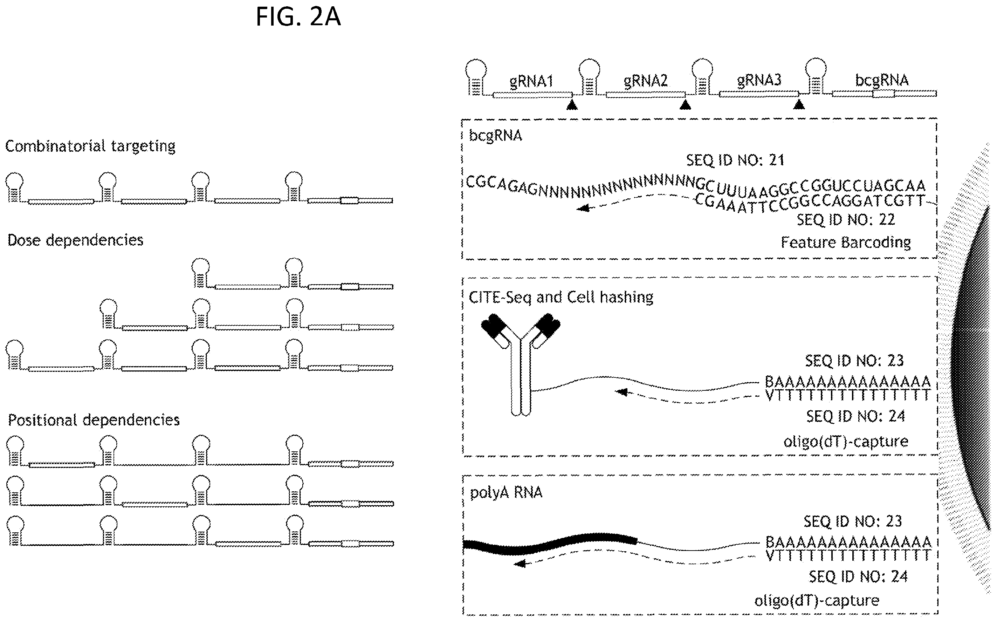

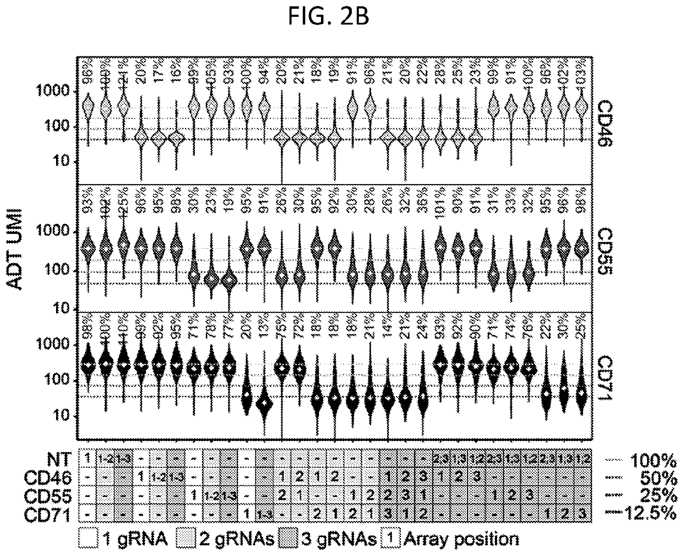

FIG. 2A - FIG. 2D show CaRPool-seq enables combinatorial gene targeting with a multimodal single-cell readout. (FIG. 2A) CaRPool-seq can be combined with CITE-seq and Cell Hashing modalities. (FIG. 2B) Violin plots depicting protein expression of target genes (ADT UMI counts for CD46, CD55, CD71), grouped by CRISPR arrays (n=29).

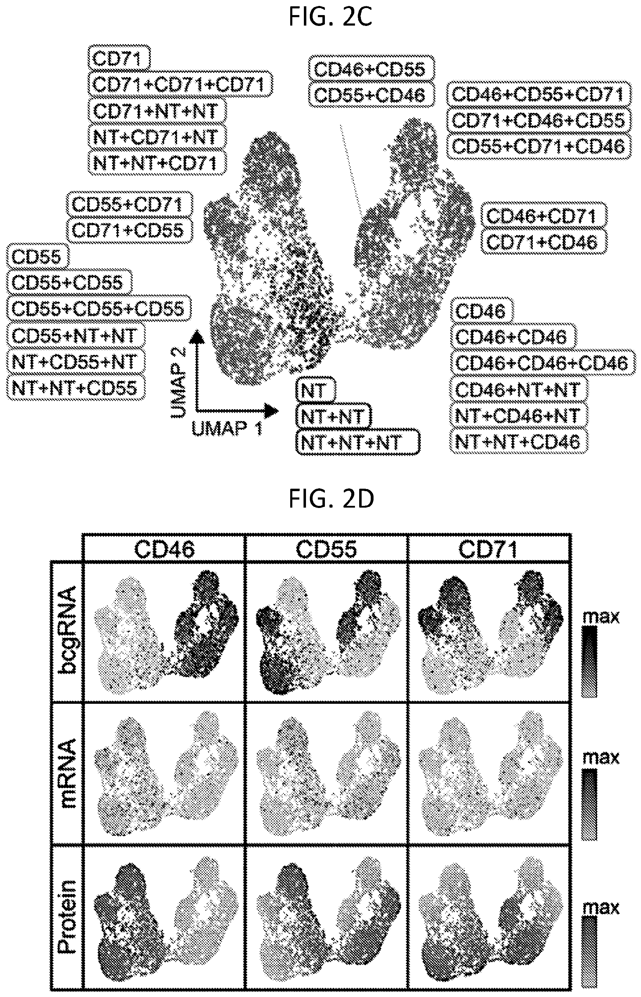

Three dashed lines indicate 50%, 25%, and 12.5% UMI count relative to the median of all non-targeting cells. Diamonds indicate the median UMI count. The number above each violin plot indicates the mean level of reduction across single cells. CD71+CD71 was not included in the experiment. (FIG. 2C) UMAP visualization of single-cell protein expression profiles of CaRPool-seq experiment (n = 6,986 cells). Cells are shaded based on the single or combinatorial perturbations they received. (FIG. 2D) Expression levels of bcgRNA, mRNA, and protein (ADT) for CD46, CD55, CD71 superimposed on the UMAP visualization (n =6,986 total cells).

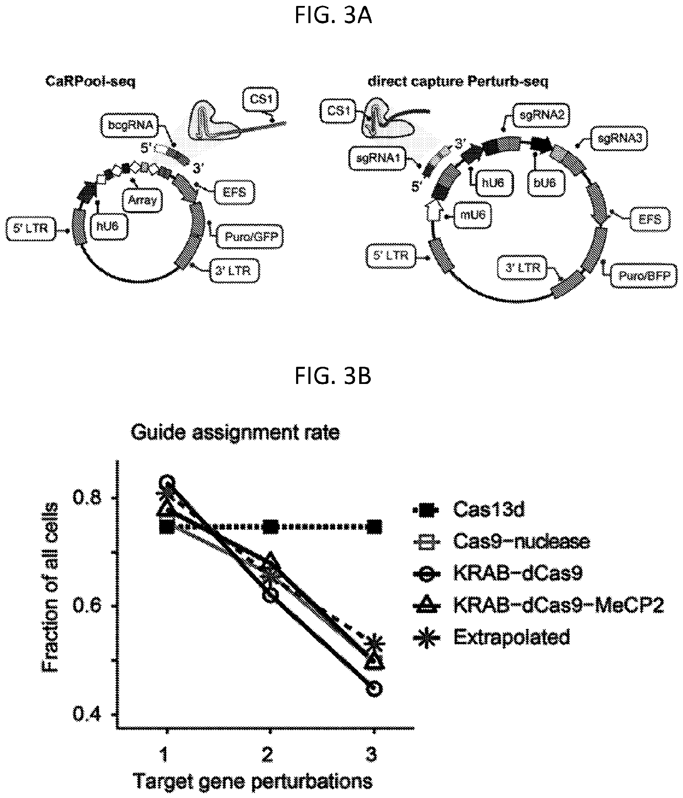

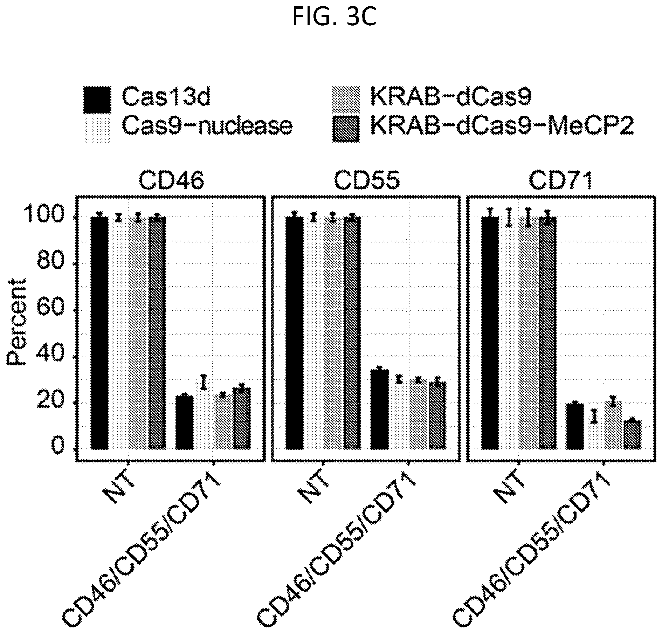

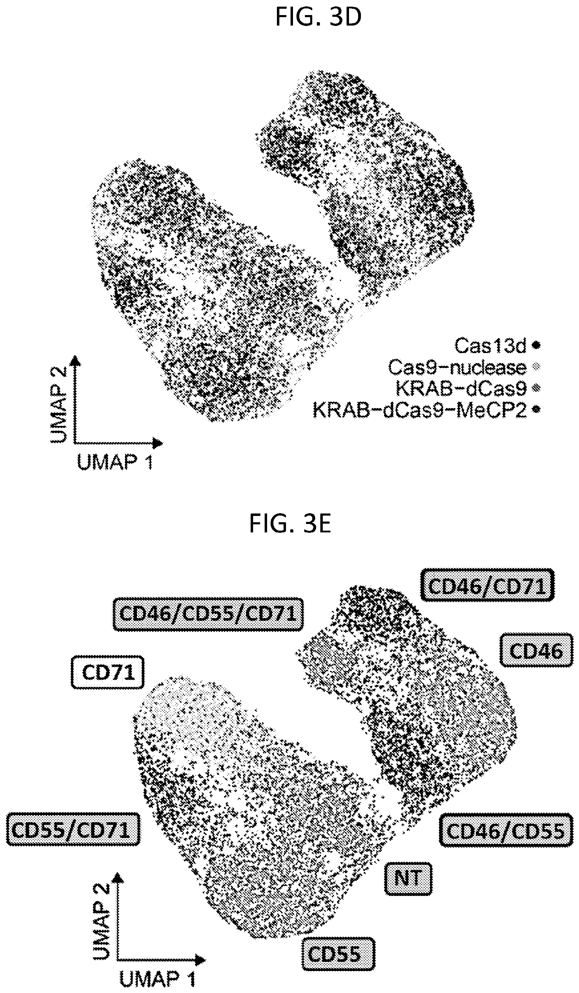

FIG. 3A - FIG. 3E show benchmarking CarPool-seq against alternative combinatorial perturbation approaches. (FIG. 3A) Plasmid vectors for lentivirus production for triple perturbation scenarios comparing CaRPool-seq and Direct Capture Perturb-seq. In both cases, the feature of interest is captured using 10x Genomics feature barcoding technology. In CaRPool-seq a single barcode sequence (bcgRNA) represents a combinatorial perturbation, while Perturb-seq requires the independent capture of multiple gRNA. (FIG. 3B) Fraction of cells where the correct combination of gRNA was detected for single, double, and triple perturbations. The dashed line represents a theoretical extrapolation based on an assumption of independent sgRNA detection with p=0.81, as reported in Replogle et al. (Nat. Biotechnol. 38, 954-961 (2020)). (FIG. 3C) Relative expression of cell surface proteins CD46, CD55, and CD71 in cells with assigned non-targeting (s)gRNAs (NT) or a combination of three targeting (s)gRNAs. The expression level of each target is normalized to NT control. Bars indicate mean across cells with s.e.m. error bars. (FIG. 3D) Protein level ADT-based clustering of single-cell expression profiles of merged CaRPool-seq, Perturb-seq experiments using Cas9, KRAB-dCas9, or KRAB-dCas9-MeCP2. Cells are shaded by perturbation technology, and in (FIG. 3E) cells are shaded based on the single or combinatorial perturbation received. Cells cluster together across technologies, indicating that all approaches introduce perturbations of similar phenotypic strength.

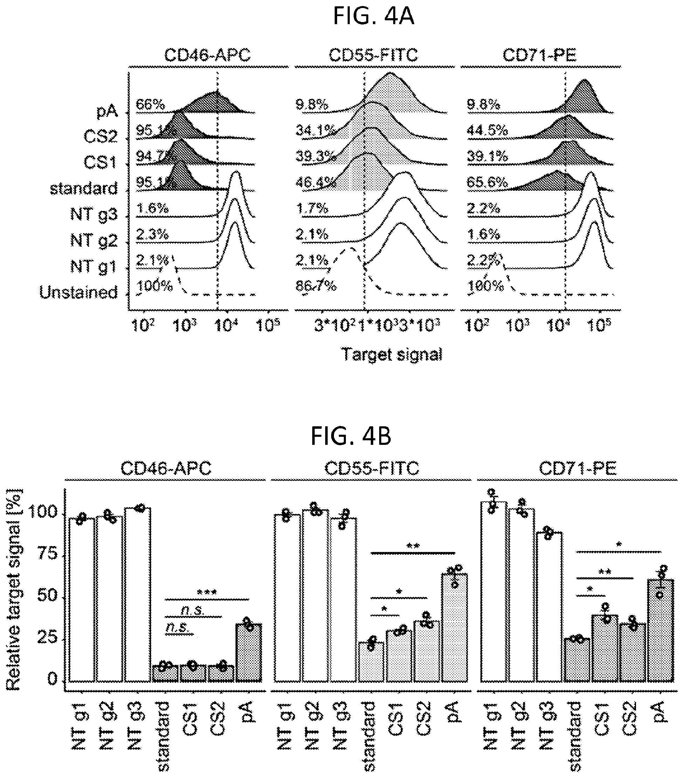

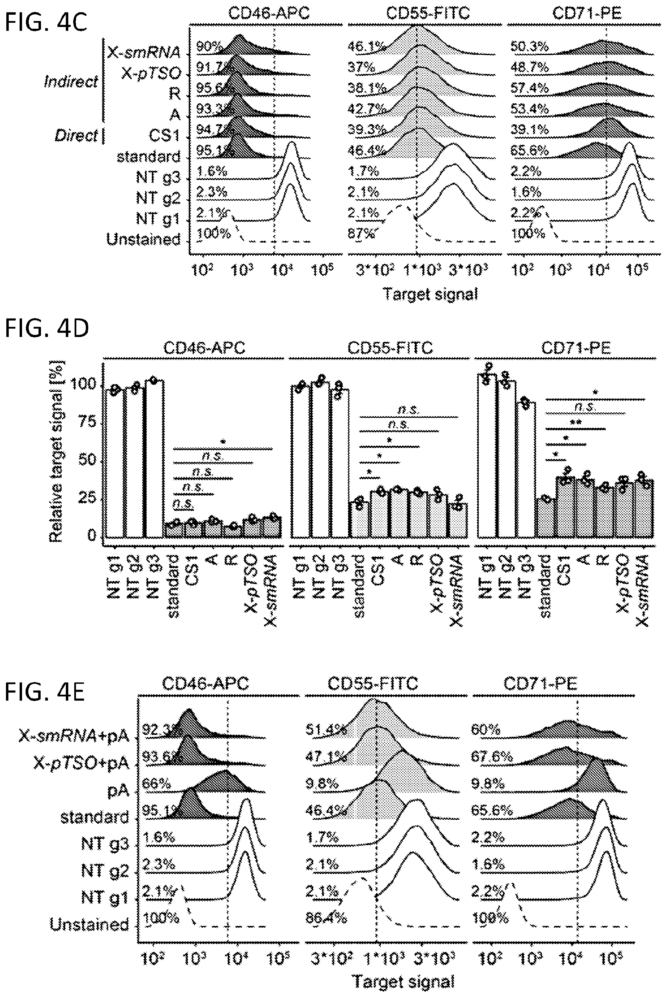

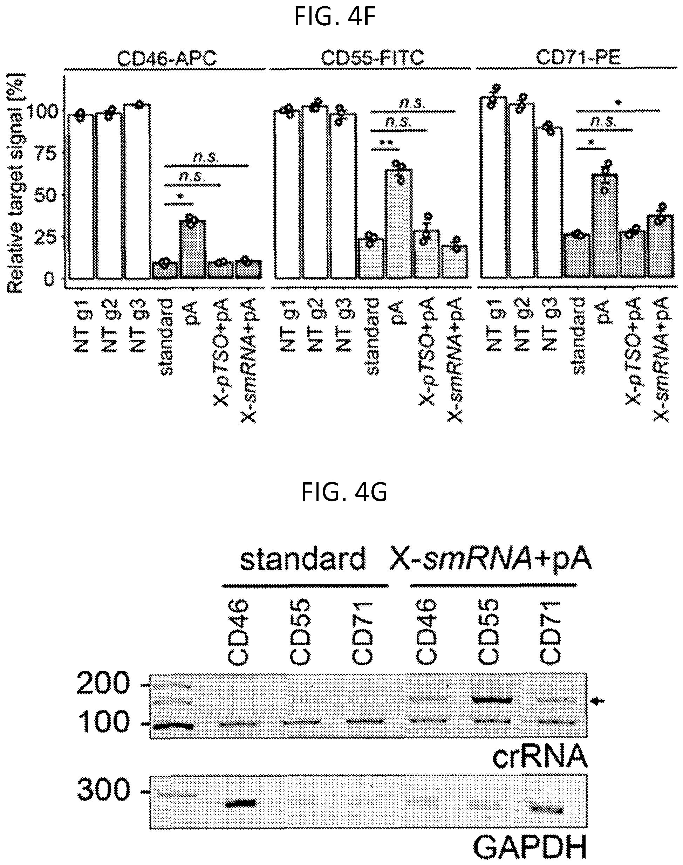

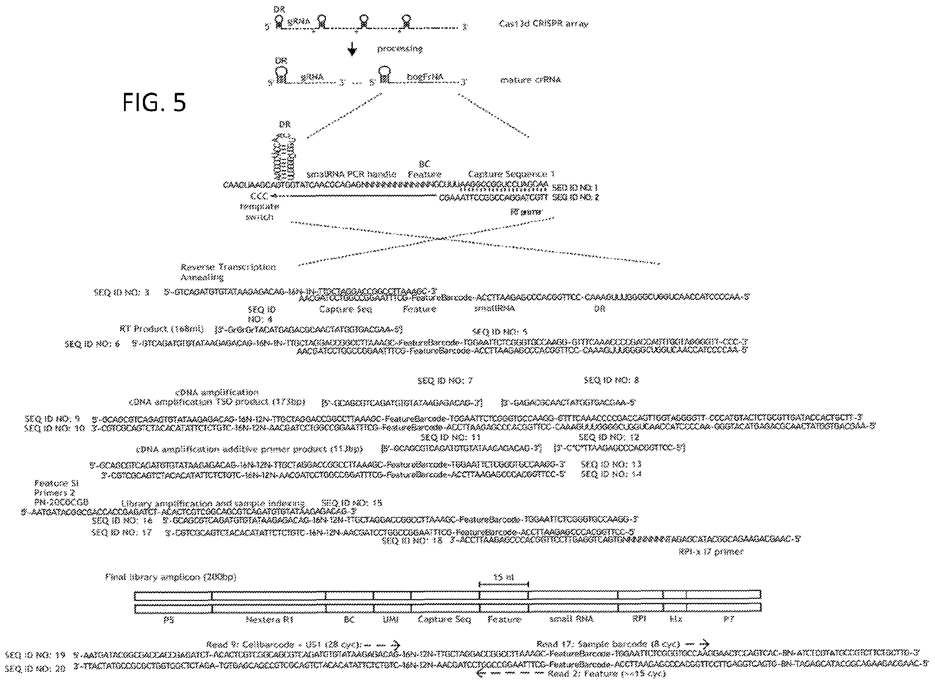

FIG. 4A - FIG. 4G show direct guide RNA capture by addition of a 3' common sequence to the Cas13d spacer RNA. (FIG. 4A) Density plots showing the CD46-APC, CD55-FITC and CD71-PE flow cytometry signal upon Cas13d-mediated knockdown with either regular gRNAs or a direct capture gRNA with one of three reverse transcription handles (pA(30) = polyA-tail of length 30, CS1 = 10x Genomics Capture Sequence 1, CS2 = 10x Genomics Capture Sequence 2, NT = non-targeting). Vertical lines mark the threshold for CD-protein negative cells (2nd percentile of NT cell populations), indicating the percent negative cells for one replicate experiment. Importantly, the Cas13 mediated function shows a unimodal response, suggesting limited cell-to-cell differences in target gene knockdown. (FIG. 4B) Summary analysis of three replicate experiments as shown in (FIG. 4A). Y-axis shows the mean fluorescent intensity (MFI) relative to the average of all NT cell populations. Direct capture constructs with CS1 or CS2 enable strong knockdown for CD46, but reduced knockdown for CD55 and CD71. Direct capture with pA-handle shows strongly reduced knockdown efficiency compared to regular gRNAs (standard condition). Two-sided t-test with * p < 0.05, ** p < 0.01, and *** p < 0.001. (FIG. 4C) Density plots showing the CD46-APC, CD55-FITC, and CD71-PE signal upon Cas13d-mediated knockdown with either regular gRNAs, a direct capture gRNA, or indirect capture construct of types A, R, and X as shown in FIG. 1A. CS1 was used in all constructs with RT-handle. Type X was used with either a partial TSO (pTSO) PCR priming site or an Illumina smallRNA PCR-handle sequence. Vertical lines mark the threshold for CD-protein negative cells, indicating the percent negative cells for one replicate experiment. (FIG. 4D) Summary analysis of three replicate experiments as shown in (FIG. 4C). Y-axis shows the mean fluorescent intensity (MFI) relative to the average of all NT cell populations. Indirect capture constructs show strong target gene knockdown similar to regular gRNAs (standard condition) for all three target genes. The slight reduction in targeting efficiency in indirect guide capture may be explained by CRISPR array processing constraints. Two-sided t-test with * p < 0.05, ** p < 0.01, and *** p < 0.001. (FIG. 4E) Density plots showing the CD46-APC, CD55-FITC, and CD71-PE signal upon Cas13d-mediated knockdown with either regular gRNAs, a direct capture gRNA, or indirect capture construct of type X. Here, comparing the effect and placement of a polyA-tail RT-handle. Type X was used with either a pTSO or smallRNA PCR-handle sequence. Vertical lines mark the threshold for CD-protein negative cells, indicating the percent negative cells for one replicate experiment. (FIG. 4F) Summary analysis of three replicate experiments as shown in (FIG. 4E). Y-axis shows the mean fluorescent intensity (MFI) relative to the average of all NT cell populations. Indirect capture constructs show strong target gene knockdown like regular gRNAs (standard condition) for all three target genes. Target knockdown with direct capture through a polyA-tail sequence is limited. Two-sided t-test with * p < 0.05, ** p < 0.01, and *** p < 0.001. (FIG. 4G) PCR amplicons of reverse-transcribed crRNAs from lentivirally infected cells used in FIG. 4E. Indirect capture of Type-X crRNAs with smallRNA PCR-handle and polyA-tail (arrow) allowed for reverse transcription and amplification. These results show that indirect gRNA capture can be facilitated with polyA-tail capture as an alternative to CS1-based capture. X-type arrays are independent of template switching success. FIG. 5 shows bcgRNA capture scheme adapted from 10x Genomics Feature Barcoding technology.

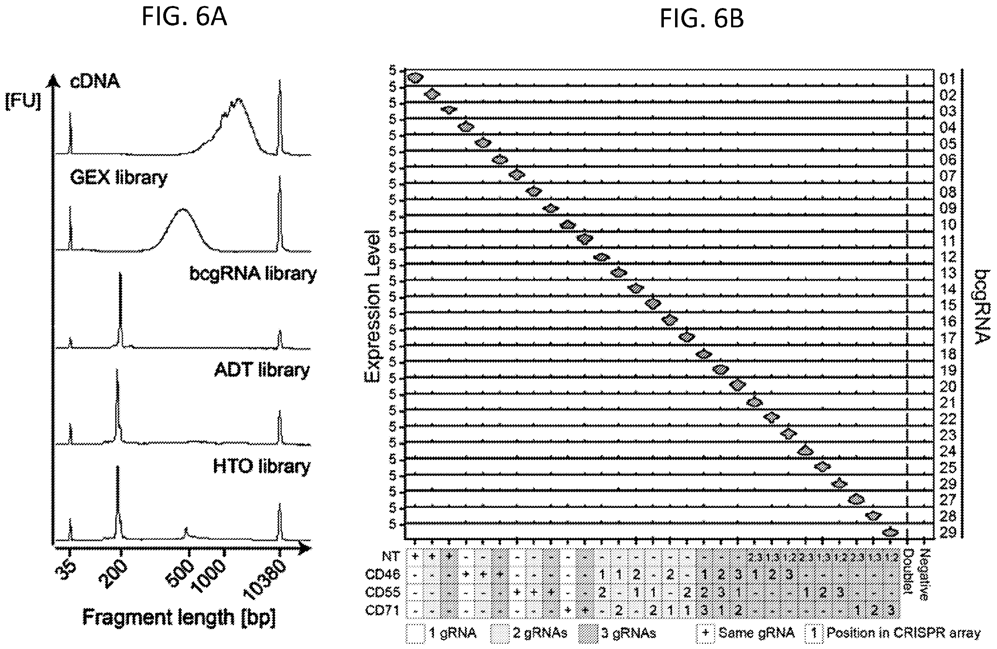

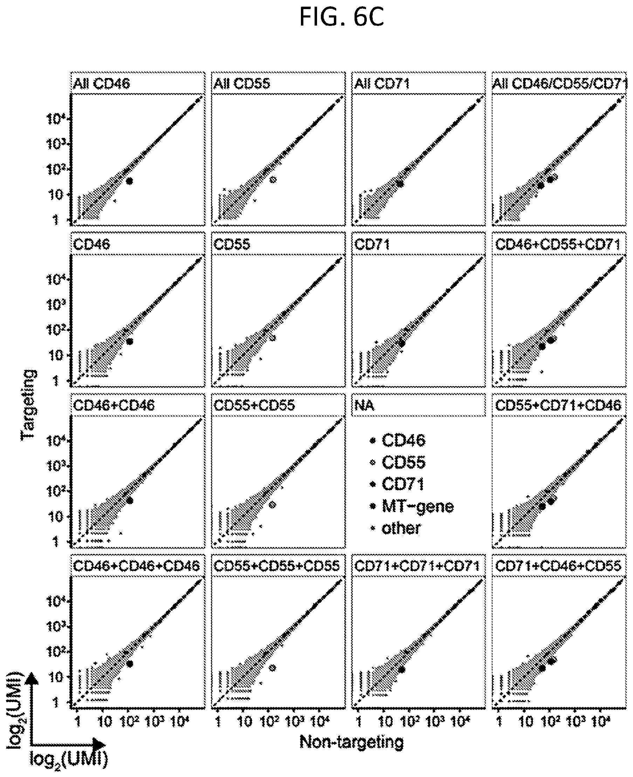

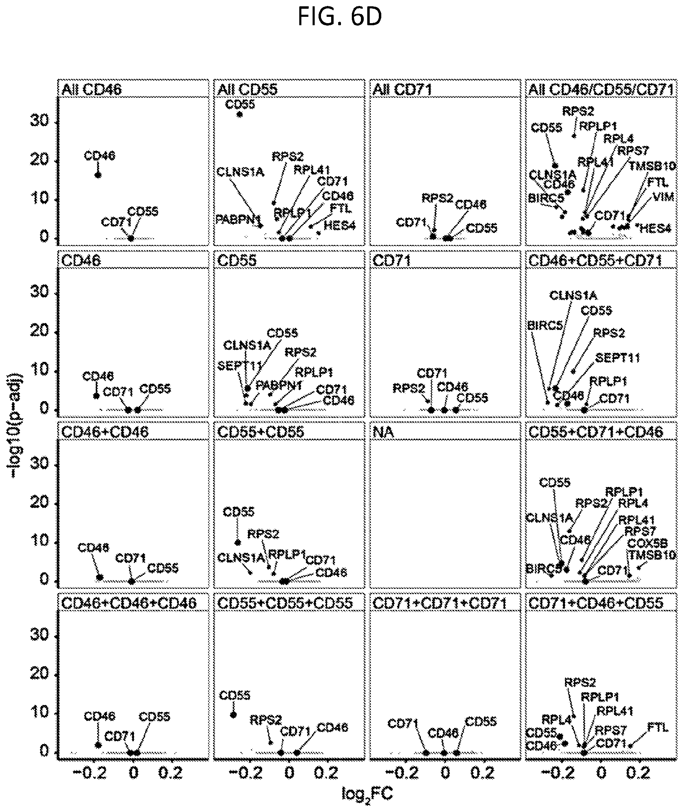

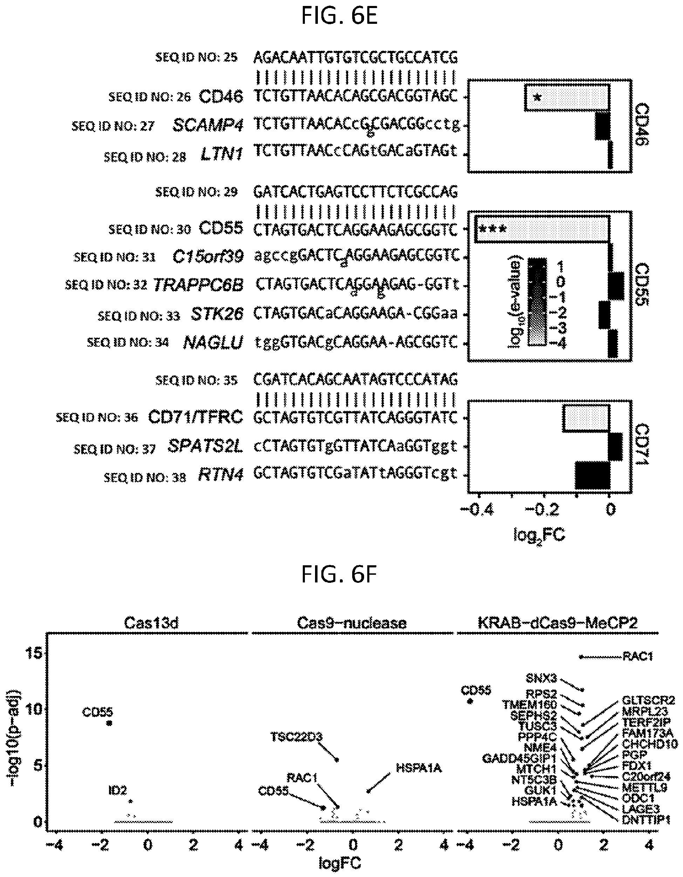

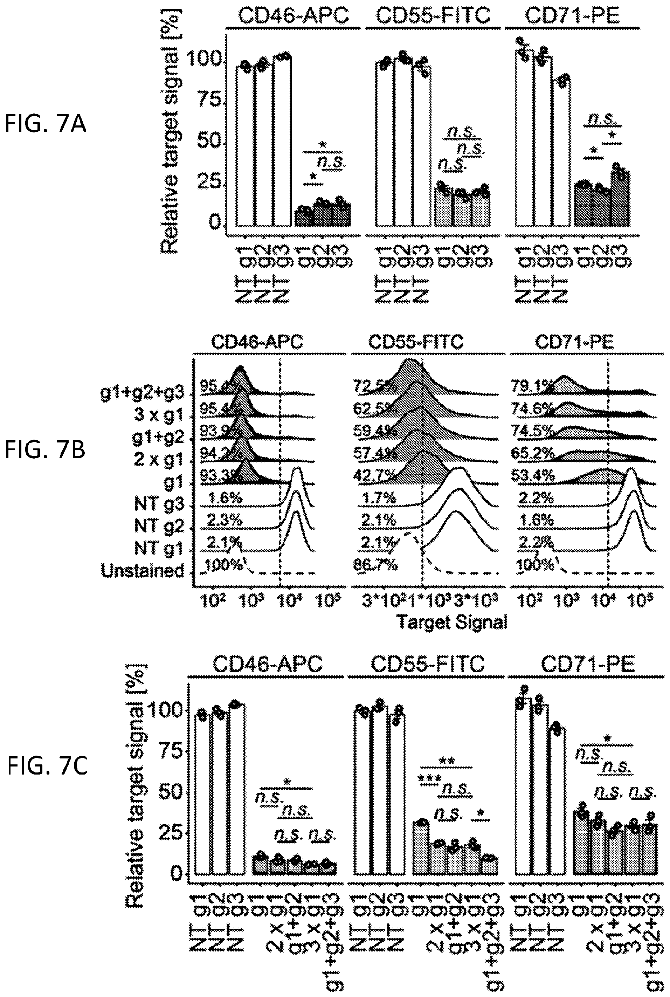

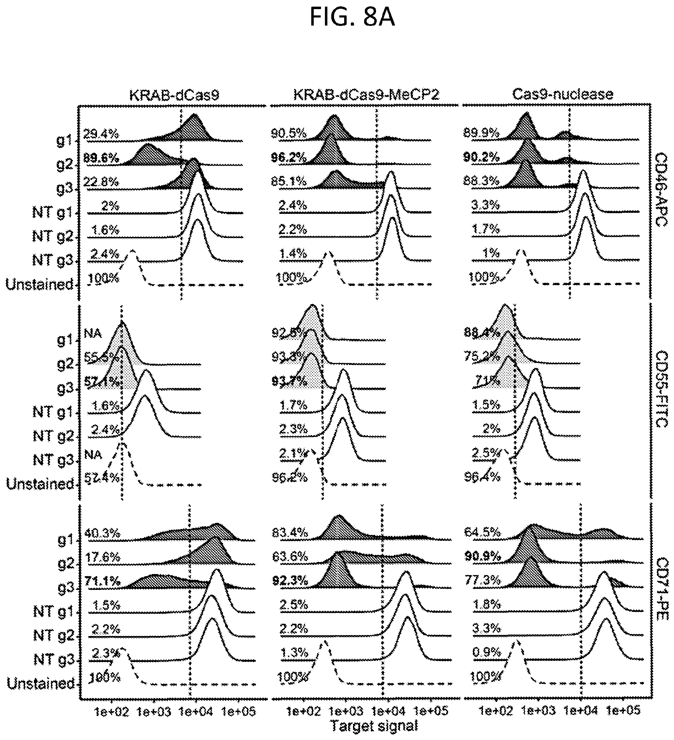

FIG. 6A - FIG. 6F show CaRPool-seq enables efficient bcgRNA capture and specific target RNA knockdown. (FIG. 6A) Representative BioAnalyzer traces of cDNA and four jointly assayed modalities (GEX = gene expression, bcgRNA = barcode guide RNA, ADT = antibody derived tags, HTO = hashtag oligonucleotides). (FIG. 6B) Stacked violin plot showing normalized bcgRNA UMI counts for cells grouped by assigned CRISPR array [total cells n = 9,355, cells with single bcgRNA n = 6,986, (74.7%)]. (FIG. 6C) Scatterplots showing normalized pseudobulk RNA UMI count profiles of cells grouped by indicated CRISPR arrays (y-axis) and control NT-cells (x-axis). Respective target genes (CD46, CD55, CD71) are highlighted. CD71+CD71 was not included in the experiment. (FIG. 6D) Volcano plots showing differential gene expression results cells grouped by indicated CRISPR arrays and control NT cells. Cells grouping is the same as in FIG. 6C. The x-axis indicates log-transformed fold changes. The y-axis depicts -log10-transformed adjusted p-values (Wilcoxon test). (FIG. 6E) Sites, and relative expression levels of gRNA-dependent predicted off-target transcripts from gRNAs targeting CD46, CD55 and CD71. E-values derived from Blastn. (Wilconxon test * p.adj. < 0.05, ** p.adj. < 0.01, *** p.adj. < 0.001). (FIG. 6F) Bulk RNA-seq result for Cas13d, Cas9-nuclease, and KRAB-dCas9-MeCP2 based targeting of CD55 using three independent CD55-targeting and NT (s)gRNAs, respectively. Volcano plots show differential gene expression results of CD55 targeting conditions relative to corresponding NT conditions grouped by indicated CRISPR effector protein. The x-axis indicates log-transformed fold changes. The y-axis depicts -log10-transformed adjusted p-values (DESeq2). The three approaches show a varying number of differentially expressed genes in addition to CD55 reduction (n=1 Cas13d, n=3 Cas9, n=30 KRAB-dCas9-MeCP2). Cas13d gRNA and Cas9 sgRNA efficiency is shown in FIG. 7A and FIG. 8A.

FIG. 7A - FIG. 7C show CaRPool-seq detects target RNA and protein knockdown for single guide RNAs. (FIG. 7A) Bar plots depicting CD46-APC, CD55-FITC, and CD71-PE signal upon Cas13d-mediated knockdown with three alternative gRNAs per target gene relative to the mean of three NT controls measured by flow cytometry. Y-axis shows the mean fluorescent intensity (MFI) relative to the average of all NT cell populations. Two-sided t-test with * p < 0.05, ** p < 0.01, and *** p < 0.001. Guide RNA g1 was used in CaRPool-seq experiments. Guide RNAs g2 and g3 are used in FIG. 7B and FIG. 7C. (FIG. 7B) Density plots showing the CD46-APC, CD55-FITC, and CD71-PE signal upon Cas13d-mediated knockdown with either 1, 2, or 3 copies of the same gRNA (g1) per CRISPR array or 2 and 3 alternative gRNAs (g2, g3). Vertical lines mark the threshold (2nd percentile of combined NT conditions) for CD-protein negative cells, indicating the percent negative cells for one replicate experiment. Shown is one representative replicate. (FIG. 7C) Summary analysis of three replicate experiments as shown in FIG. 7B. Y-axis shows the mean fluorescent intensity (MFI) relative to the average of all NT cell populations. The analysis suggests that target gene knockdown differences between the number of gRNAs per array are more pronounced than differences between gRNA identities with the same total count, given that gRNA efficiencies are comparable as shown in FIG. 7B. CRISPR arrays encoding multiple gRNAs against the same target may be used to further enhance target knockdown. Two-sided t-test with * p < 0.05, ** p < 0.01, and *** p < 0.001.

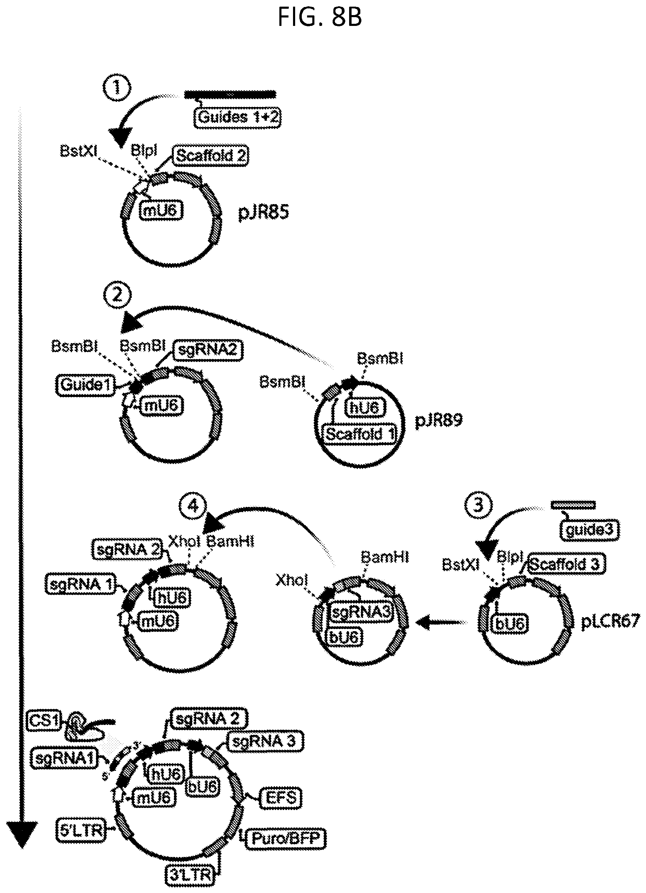

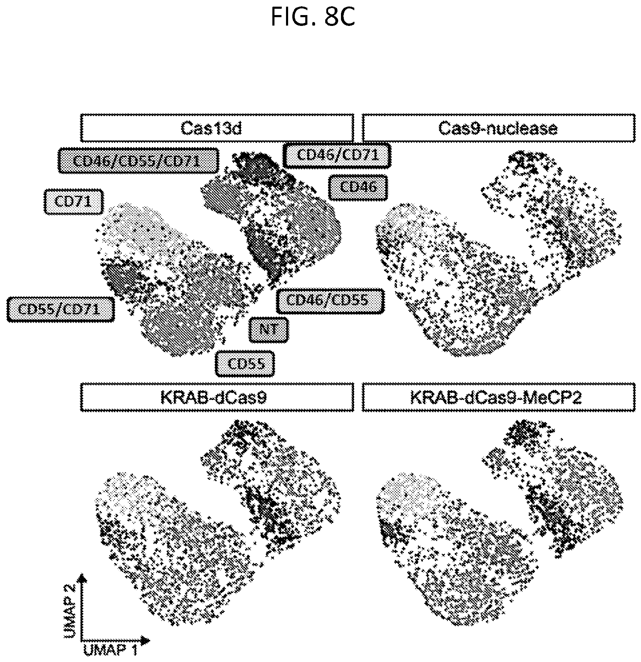

FIG. 8A - FIG. 8C shows CaRPool-seq enables efficient combinatorial target RNA perturbation. (FIG. 8A) Density plots showing the CD46-APC, CD55-FITC, and CD71-PE flow cytometry signal upon Cas9-nuclease mediated knockout (KO) and CRISPRi-mediated (KRAB-dCas9, KRAB-dCas9-MeCP2) knockdown with three alternative sgRNAs from established genome-wide KO (Doench, J. G. et al. Nat. Biotechnol. 34, 184-191 (2016)) and CRISPRi (Sanson, K. R. et al. Nat. Commun. 9, 1-15 (2018)) libraries. Vertical lines mark the threshold (2nd percentile of combined NT conditions) for CD-protein negative cells, indicating the percent negative cells for one replicate experiment. Single guide RNAs with the highest percentage of negative cells were selected for direct capture Perturb-seq experiments (NA = sgRNA not assayed). (FIG. 8B) Cloning strategy for triple sgRNA plasmid vectors. Dual sgRNA constructs were cloned as described before (Replogle, J. M. et al. Nat. Biotechnol. 38, 954-961 (2020)). The third sgRNA was cloned behind a bovine U6 promoter using an alternative sgRNA scaffold tested before (Replogle, J. M. et al. Nat. Biotechnol. 38, 954-961 (2020)). (FIG. 8C) Protein level ADT-based clustering of single-cell expression profiles of merged CaRPool-CITE-seq (n = 6,986 cells) and Perturb-seq experiments using Cas9-nuclease (n = 2,836), KRAB-dCas9 (n = 2,911) or KRAB-dCas9-MeCP2 (n = 3,038) effector proteins as in FIG. 3E. Cells are labelled by the assigned target gene combination based on detected bcgRNA or sgRNAs and split by Perturb-seq.

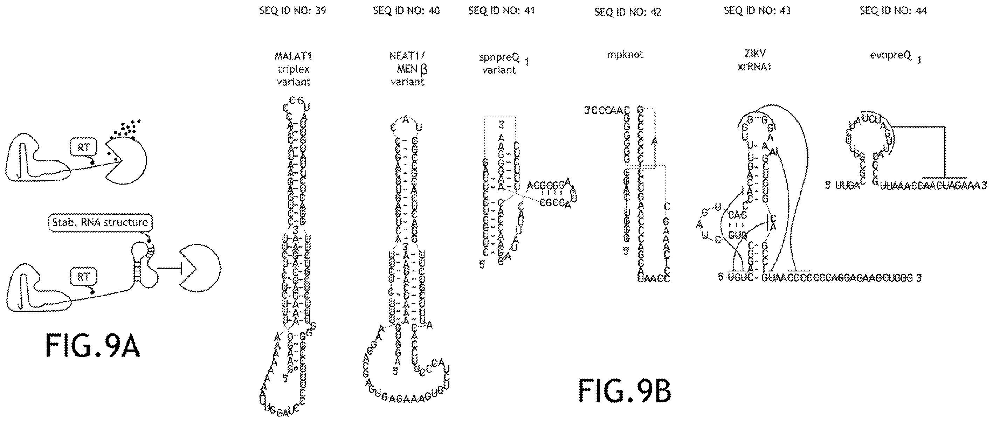

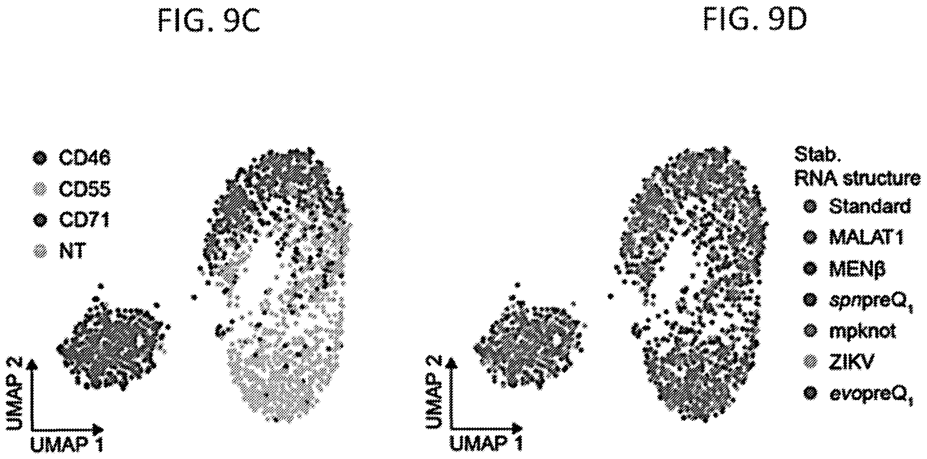

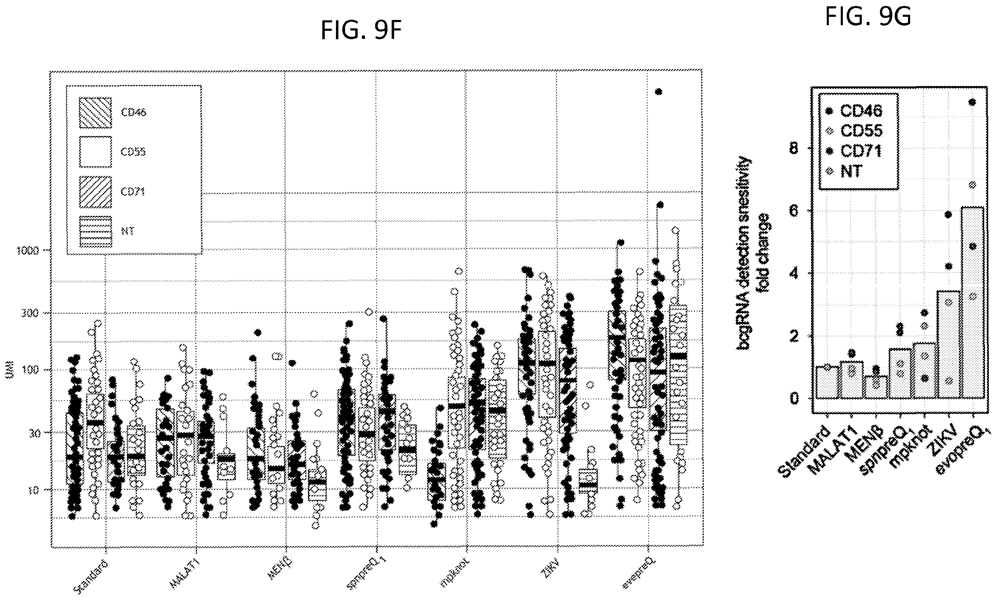

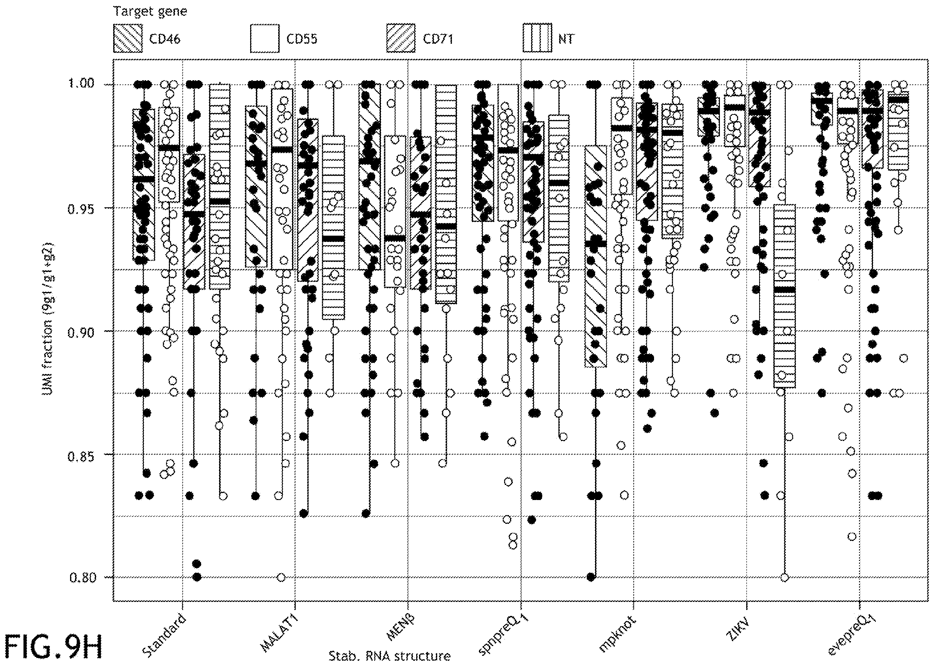

FIG. 9A - FIG. 9H show stable RNA structures can improve bcgRNA detection in CaRPool-seq experiments. (FIG. 9A). Model of 3' exonucleolytic decay of bcgRNAs when embedded in CRISPR Cas13d ribonucleoprotein (RNP) complex. The Cas13 protein embeds the first ~22 nucleotides of the bcgRNA while the 3'end protrudes outside the RNP complex (Mendez-Mancilla, A. et al. Cell Chem. Biol. 1-7 (2021)doi:10.1016/j.chembiol.2021.07.011). Sequences that can form stable RNA structures are placed directly behind the reverse transcription handle (RT) and may antagonize exonucleolytic decay. (FIG. 9B) Nucleotide sequences that can form stable RNA structures when placed 3' to a bcgRNA. Sequences found in MALAT1 and NEAT1 (MENβ) (Brown, J.A. et al. Proc. Natl. Acad. Sci. U. S. A. 109, 19202-19207 (2012)) required nucleotide exchanges (shown in red) to remove potential terminator sequences (>= 4U) and allow the sequences to be fully transcribed by RNA polymerase III. Other sequences tested have been described before (spnpreQ$_1$, ZIKV xrRNA1, mpknot and evopreQ$_1$) (Kang, M. et al. Proc. Natl. Acad. Sci. U. S. A. 111, (2014); Akiyama, B. M. et al. Science. 354, 1148-1152 (2016); Anzalone, A.V. et al. Nat. Methods 13, 453-458 (2016)). (FIG. 9C) UMAP visualization of single-cell protein expression profiles of CaRPool-seq experiment (n = 1,770 cells). The experiment included four different gRNAs [Non-targeting control, CD46, CD55, and CD71) in combination with one out of seven different stabilizing elements [no stabilizing element (standard) or one of the six shown in FIG. 9B]. Cells are shaded based on the single perturbations they received. (FIG. 9D) UMAP visualization of single-cell protein expression profiles of CaRPool-seq experiment (n = 1,770 cells). Cells are shaded based on the stabilizing element they received. (FIG. 10E) Violin plots depicting protein expression of target genes (ADT UMI counts for CD46, CD55, CD71), grouped by CRISPR arrays [combination of target gene (y-axis) and stabilizing RNA element (x-axis); n=28]. Three dashed lines indicate 50%, 25%, and 12.5% UMI count relative to the mean of all non-targeting cells by target ADT. The numbers above each violin plot indicate the median reduction across single cells for cells with matching gRNA and target. (FIG. 9F) UMI counts for the assigned bcgRNA for each cell separated by target gene and RNA element. (FIG. 9G) Fold enrichment of bcgRNA UMI counts relative to UMI counts in the standard bcgRNA capture condition separated by the target gene. The $evo$preQ$_1$ element yielded on average 6-fold higher bcgRNA detection sensitivity. (FIG. 9H) UMI Fraction comparing the assigned bcgRNA to the sum of assigned and second most abundant bcgRNA that may be detected for the same cell [UMI g1 / (UMI g1 + UMI g2)]. If no second bcgRNA has been detected, we set g2 UMI counts to 1.

## DETAILED DESCRIPTION OF THE INVENTION

Pooled CRISPR screens coupled with single-cell RNA-sequencing have enabled systematic interrogation of gene function and regulatory networks. Described herein is Cas13 RNA Perturb-seq (CaRPool-seq) (also referred to herein as CARP-seq) which leverages the RNA-targeting CRISPR/Cas13d system and enables efficient combinatorial perturbations alongside multimodal single-cell profiling. CaRPool-seq encodes multiple perturbations on a cleavable array which is associated with a detectable barcode sequence, allowing for the simultaneous targeting of multiple genes. We compared CaRPool-seq to existing Cas9-based methods and demonstrated its unique strength to efficiently profile combinatorially perturbed cells. It would be recognized by one of skill in the art that the present invention can be used with a variety of cell types and to target any transcript that is amenable to targeting using a CRISPR Cas system. CARP-seq can be utilized, for example, alongside cell hashing and cell surface protein quantification (CITE-seq).

Unless defined otherwise in this specification, technical and scientific terms used herein have the same meaning as commonly understood by one of ordinary skill in the art to which this invention belongs and by reference to published texts, which provide one skilled in the art with a general guide to many of the terms used in the present application.

As used throughout this specification and the claims, the terms "comprising", "containing", "including", and its variants are inclusive of other components, elements, integers, steps and the like. Conversely, the term "consisting" and its variants are exclusive of other components, elements, integers, steps and the like.

It is to be noted that the term "a" or "an", refers to one or more, for example, "RNA target", is understood to represent one or more RNA target(s). As such, the terms "a" (or "an"), "one or more," and "at least one" is used interchangeably herein.

As used herein, the term "about" means a variability of plus or minus 10% from the reference given, unless otherwise specified.

### Compositions

Provided herein are CRISPR arrays that include one or more crRNA(s) and a barcode guide RNA (bcgRNA), as well as nucleic acids, expression cassettes, and vectors comprising sequences encoding for the same. As provided herein, a "CRISPR array" refers to an arrangement of crRNA(s) and a barcode guide RNA (bcgRNA) capable of introducing one or more perturbations in a cell transcriptome. The presence of the bcgRNA facilitates detection of the CRISPR array during downstream analysis, including single cell readouts. In some embodiments, the CRISPR array comprises, from 5' to 3' one, two, three, or more crRNAs, and a bcgRNA comprising a DR sequence, a barcode sequence, and a reverse transcription handle. In a further embodiment, the bcgRNA also includes a PCR handle. In certain embodiments, the CRISPR array comprises, from 5' to 3' one, two, three, or more crRNAs, and a bcgRNA comprising a DR sequence, a barcode sequence, and a reverse transcription handle. In yet another embodiment, the bcgRNA is upstream (5') of the one or more crRNA sequences in a CRISPR array.

### crRNA

As used herein, "crRNA" is an abbreviation of clustered regularly interspaced short palindromic repeats (CRISPR) RNA, which is a nucleic acid molecule composed of a direct repeat (DR) stem loop sequence and a guide sequence. The terms "guide RNA" or "gRNA" or "guide sequence" or "spacer sequence" are used interchangeably herein and refer to a nucleic acid sequence which can hybridize to a sequence (hybridization region or target region) of a target nucleic acid (or target sequence), e.g., a target RNA. In one embodiment, the guide RNA is about 20 nucleotides (nt) to about 33 nt. In a further embodiment, the guide RNA is about 20 nt, about 21 nt, about 22 nt, about 23 nt, about 24 nt, about 25, nt, about 26, nt, about 27 nt, about 28 nt, about 29 nt, about 30 nt, about 31 nt, about 32 nt, or about 33 nt. In one embodiment, the guide RNA is about 23 nt. In another embodiment, the guide RNA is about 27 nt. The gRNAs may comprise non-naturally occurring nucleotides.

As used herein, a "target sequence" refers to a sequence to which a guide (spacer) sequence is designed to have reverse complementarity, where hybridization between a target sequence and a spacer sequence promotes the formation of a CRISPR complex. A target sequence may comprise RNA polynucleotides. The term "target RNA" refers to an RNA polynucleotide being or comprising the target sequence, including coding and non-coding transcripts. In other words, the target RNA may be an RNA polynucleotide or a part of a RNA polynucleotide to which a part of the crRNA, i.e. the spacer sequence, is designed to have complementarity and to which the effector function mediated by the complex comprising CRISPR effector protein and a guide RNA (gRNA) is to be directed. In certain embodiments, a target sequence is located in the nucleus or cytoplasm of a cell. In one embodiment, the target RNA comprises at least 20 nt (or at least 23 nt, or at least 87 nt, or at least 100 nt) RNA residues or a modification thereof. In a further embodiment, the target RNA comprises at least 20 nt contiguous RNA residues or a modification thereof. The region of a target RNA which is capable of hybridizing to a guide of a crRNA is referred to herein as a potential hybridization region. Such target RNA, a hybridization region therein, a crRNA which the hybridization region of the target RNA may hybridize to, and a guide of the crRNA are corresponding to each other.

The nucleic acid sequence of the crRNA can be any polynucleotide sequence having sufficient complementarity with a target polynucleotide sequence (e.g., target RNA sequence) to hybridize with the target sequence and direct sequence-specific binding of a CRISPR complex to the target sequence. In some embodiments, the degree of complementarity between a spacer sequence of the modified crRNA and its corresponding target sequence, when optimally aligned using a suitable alignment algorithm, is about or more than about 50%, 60%, 75%, 80%, 85%, 90%, 95%, 97.5%, 99%, or more. The term "complementarity" refers to the ability of a nucleic acid to form hydrogen bond(s) with another nucleic acid sequence by either traditional Watson-Crick or other non-traditional types. A percent complementarity indicates the percentage of residues in a nucleic acid molecule which can form hydrogen bonds (e.g., Watson-Crick base pairing) with a second nucleic acid sequence. As used herein the term "complementary" may refer to sequences having perfect complementarity, i.e., all the contiguous residues of a nucleic acid sequence will hydrogen bond with the same number of contiguous residues in a second nucleic acid sequence, or substantially complementary sequences, i.e., those having less than 100% complementarity, provided that the two nucleic acids hybridize under stringent conditions. The term "stringent conditions" for hybridization refers to conditions under which a nucleic acid having complementarity to a target sequence predominantly hybridizes with the target sequence, and substantially does not hybridize to non-target sequences. Stringent conditions are generally sequence-dependent, and vary depending on a number of factors. In general, the longer the sequence, the higher the temperature at which the sequence specifically hybridizes to its target sequence. Non-limiting examples of stringent conditions are described in detail in Tijssen (1993), Laboratory Techniques In Biochemistry And Molecular Biology-Hybridization With Nucleic Acid Probes Part 1, Second Chapter "Overview of principles of hybridization and the strategy of nucleic acid probe assay", Elsevier, N.Y. Optimal alignment may be determined with the use of any suitable algorithm for aligning sequences, non-limiting example of which include ClustalW and Clustal X. In some embodiments, a spacer sequence is about 20, 21, 22, 23, 24, 25, 26, 27, 28, 29, 30, 31, 32, 33, 34, 35, 40, 45, 50, 75, or more nucleotides in length. In some instances, a spacer sequence is about 20 nucleotides in length. In other instances, a spacer sequence is 23 nucleotides in length. In other instances, a spacer sequence is about 25 nucleotides in length. The ability of a spacer sequence to direct sequence-specific binding of a CRISPR complex to a target sequence may be assessed by any suitable assay. For example, the components of a CRISPR system sufficient to form a CRISPR complex, including the spacer sequence to be tested, may be provided to a host cell having the corresponding target sequence, such as by transfection with vectors encoding the components of the CRISPR sequence, followed by an assessment of preferential cleavage within the target sequence. Similarly, cleavage of a target polynucleotide sequence may be evaluated in a test tube by providing the target sequence, components of a CRISPR complex, including the spacer sequence to be tested and a control spacer sequence different from the test spacer sequence, and comparing binding or rate of cleavage at the target sequence between the test and control spacer sequence reactions. The nucleotide sequence of a suitable crRNA can be selected using any of the web-based software known in the art, including the Cas13 guide designer provided by the Sanjana lab, available online at cas13design.nygenome.org. See also, Guo et al., Transcriptome-wide Cas13 guide RNA design for model organisms and viral RNA pathogens, bioRxiv 2020.08.20.259762 and Wessels, HH., Méndez-Mancilla, A., Guo, X. et al. Massively parallel Cas13 screens reveal principles for guide RNA design. Nat Biotechnol 38, 722-727 (2020).

The crRNA and bcgRNA described herein include direct repeat (DR) sequences. The DR sequence is a short hairpin region via which the Cas protein complexes with the guide RNA. Like all CRISPR-Cas effectors described to date, Cas13 enzymes each recognize a direct repeat (DR) sequence containing a stem loop structure within their cognate crRNA. In certain embodiments, the crRNA is capable of complexing with a Cas13d protein and providing targeting specificity and binding ability for Class 2, Type VI protein, such as Cas13d or a variant thereof. In some embodiments, the DR sequence is a Cas13d sequence. However, between Cas13 subtypes, the DR sequence motifs, RNA fold, and DR position relative to the spacer sequence are each distinct. For Cas13a and Cas13d, the DR is located on the 5' end while the Cas13b DR is 3' of the spacer sequence. Thus, in one embodiment, the DR is 5' to the spacer sequence. In another embodiment, the DR is 3' to the spacer sequence. See, e.g., Cheng et al, Structural Basis for the RNA-Guided Ribonuclease Activity of CRISPR-Cas13d, Cell. 2018 Sep 20;175(1):212-223.e17. In some embodiments, the DR is 5' to the spacer sequence in the crRNAs.

*bcgRNA*

The CRISPR arrays provided herein require a barcode guide RNA (bcgRNA) which allows for detection and/or identification of the specific array in use. The bcgRNA includes a barcode sequence and a reverse-transcription handle. In certain embodiments, the bcgRNA also includes a PCR handle. The barcode, or barcode sequence, is a unique DNA sequence that corresponds to the specific array in which it is included, such that the barcode encodes the collective identity of the perturbations included in the array. The barcode typically comprises four or more nucleotides. In some embodiments, the barcode comprises 4, 5, 6, 7, 8, 9, 10, 11, 2, 13, 14, or 15 nucleotides. In some embodiments, the barcode comprises 8 to 15 nucleotides. In some embodiments, a library of CRISPR arrays is provided which includes multiple different arrays, each unique array containing a different barcode. In some embodiments, the CRISPR arrays provided include a direct repeat (DR) sequence 5' to the bcgRNA. The DR sequence is specific for the Cas enzyme being used.
The term "reverse-transcription (RT) handle" refers to a sequence of the bcgRNA that is a primer binding site to facilitate reverse-transcription. Thus, in a suitable reverse-transcription reaction, extension of the primer forms a polynucleotide that includes a sequence complementary to the template bcgRNA. In certain embodiments, the RT handle has a length of at least 4 nucleotides, 5 nucleotides, 10 nucleotides, 15 nucleotides, 20 nucleotides, 25 nucleotides, 30 nucleotides, 35 nucleotides, 40 nucleotides, 45 nucleotides, 50 nucleotides, 60 nucleotides, 70 nucleotides, 80 nucleotides, 90 nucleotides, 100 nucleotides, or a length within a range of any two of the foregoing lengths. In certain embodiments, the RT handle includes a polyA sequence (or tail), where the oligo(dT) primers would suitable. In certain embodiments, the RT handle is capable of hybridizing with a polynucleotide on a substrate, e.g., an isolation bead. In one embodiment, the RT handle has a length of 7, 12, 17, or 22 nucleotides. In another embodiment, the RT handle includes a 10x Genomics Capture Sequence, or variant thereof. In one embodiment, the RT handle includes a 10x Genomics Capture Sequence CS1. In another embodiment, the RT handle includes a 10x Genomics Capture Sequence CS2.
In certain embodiments, reverse transcription of the bcgRNA synthesizes a complement to the PCR handle, which is located between the DR 5' to the bcgRNA and the barcode sequence. See, e.g., FIG. 1A. The term "PCR handle" refers to a primer binding site capable of binding to a primer to facilitate PCR. In certain embodiments, the PCR handle is primer binding site to initiate extension in a PCR of a complementary strand that includes the barcode sequence. The PCR handle has a length of at least 4 nucleotides, 5 nucleotides, 10 nucleotides, 15 nucleotides, 20 nucleotides, 25 nucleotides, 30 nucleotides, 35 nucleotides, 40 nucleotides, 45 nucleotides, 50 nucleotides, 60 nucleotides, 70 nucleotides, 80 nucleotides, 90 nucleotides, 100 nucleotides, or a length within a range of any two of the foregoing lengths. In one embodiment, the PCR handle is an Illumina PCR handle.

*CRISPR array*

The CRISPR array comprises at least one crRNA and a bcgRNA. In some embodiments, the array includes one, two, three, or more crRNAs. These crRNAs may be designed to produce the same, or different, perturbation(s) in a cell transcriptome when introduced in conjunction with a Cas enzyme. The crRNAs are, in some embodiments, the same crRNA, for example, to assess the effect of crRNA dosage. In other embodiments, the crRNAs are different. In some embodiments, the crRNAs target the same gene transcript (for example, at different regions/sequences of the target gene transcript). In other embodiments, the crRNAs target different gene transcripts.
In certain embodiments, multiple CRISPR arrays are provided, which include various combinations of crRNAs to be tested. See, e.g., FIG. 2A. For example, in some embodiments, two, three, or more crRNAs are included which target different gene transcripts, for testing combinatorial targeting. In other embodiments, dose dependency may be tested, such as by including one, two, three, or more copies of a single crRNA in different CRISPR arrays and assessing the effects. In still other embodiments, positional dependency of crRNAs can be tested, by changing the location of various crRNAs in the CRISPR arrays. FIG. 2A demonstrates certain embodiments in which three crRNAs (or gRNAs) are utilized. However, other embodiments are contemplated wherein one, two, three, or more crRNAs are utilized. Other combinations are contemplated based on the components described herein.
The CRISPR arrays described herein, when provided with a Cas enzyme/system under the proper conditions, are processed by the enzyme into one, two, three, or more mature crRNAs and the bcgRNA. See, e.g., Konermann, S. et al. Transcriptome Engineering with RNA-Targeting Type VI-D CRISPR Effectors. Cell 173, 665-676 (2018).
In some embodiments, the CRISPR arrays described herein are useful with any RNA-targeting CRISPR enzyme, such as members of the Cas13 family. The diverse Cas13 family contains at least four known subtypes, including Cas13a (formerly C2c2), Cas13b, Cas13c, and Cas13d. The Cas13 family is the only family of class 2 Cas enzymes known to exclusively target single-stranded RNA. Cas13 enzymes and systems are known in the art, see, e.g.,

US Patent No. 10,362,616, Abudayyeh, et al, C2c2 is a single-component programmable RNA-guided RNA-targeting CRISPR effector. Science 353, aaf5573 (2016); S. Shmakov, et al, Discovery and functional characterization of diverse class 2 CRISPR-Cas systems. Mol. Cell 60, 385-397 (2015); S. Shmakov, et al, Diversity and evolution of class 2 CRISPR-Cas systems. Nat. Rev. Microbiol. 15, 169-182 (2017). A. A. Smargon, et al, Cas13b is a type VI-B CRISPR-associated RNA-guided RNase differentially regulated by accessory proteins Csx27 and Csx28. Mol. Cell 65, 618-630.e7 (2017); J. S. Gootenberg, et al, Nucleic acid detection with CRISPR-Cas13a/C2c2. Science 356, 438-442 (2017); O. O. Abudayyeh, et al, RNA targeting with CRISPR-Cas13. Nature 550, 280-284 (2017). Each of these documents is incorporated herein.

A Cas13 protein uses a short crRNA that interacts with the Cas13 molecule through a stem loop and facilitates target binding and cleavage through a series of conformational changes in the Cas13 molecule. In certain embodiments, the Cas13 protein is Cas13a, Cas13b, Cas13c, or Cas13d. In one embodiment, the Cas13 comprises one or more mutations the HEPN domain(s).

The Cas13 protein may be from any organism. In certain embodiments, the Cas13 effector protein is from an organism of a genus selected from: Leptotrichia, Listeria, Corynebacter, Sutterella, Legionella, Treponema, Filifactor, Eubacterium, Streptococcus, Lactobacillus, Mycoplasma, Bacteroides, Flaviivola, Flavobacterium, Sphaerochaeta, Azospirillum, Gluconacetobacter, Neisseria, Roseburia, Parvibaculum, Staphylococcus, Nitratifractor, Mycoplasma, Campylobacter, and Lachnospira. In one embodiment, the Cas13 is derived from Leptotrichia shahii, Leptotrichia wadei, Listeria seeligeri, Clostridium aminophilum, Carnobacterium gallinarum, Paludibacter propionicigenes, Listeria weihenstephanensis, or Ruminococcus flavefaciens.

The Cas13d protein is a Class 2, Type VI CRISPR effector guided by a crRNA. Two higher eukaryotes and prokaryotes nucleotide-binding (HEPN) domains have been found in the Cas13d, flanking a helical domain. See, for example, WO 2019/010384 AI, US 2019/0169595A1, Zhang C, et al. (2018). Structural Basis for the RNA-Guided Ribonuclease Activity of CRISPR-Cas13d. Cell 175, 212-223.e217, golden.com/wiki/CRISPR-Cas13d, and zlab.bio/-cas13. While the term Class 2, Type VI is a broader genus, of which Cas13d is exemplary, throughout the Specification, one of skill in the art would appreciate that the use of the terms "Cas13d" or "Cas13d and a variant thereof" also encompass other Class 2, Type VI proteins, and the terms can be interchangeable. Cas13d and a variant thereof includes, e.g., a wild type or naturally occurring Cas13d protein, an ortholog of a Cas13d, a functional variant thereof, or another modified variant as disclosed.

Orthologs are genes in different species that evolved from a common ancestral gene by speciation. Normally, orthologs retain the same function in the course of evolution. In some embodiments, the Cas13d is selected from a *RftCas13d* from *Ruminococcus flavefaciens* strain XPD3002, an AdmCas13d from *Anaerobic digester metagenome 15706, EsCas13d* from *Eubacterium siraeum DSM15702, P1E0Cas13d* from *Gut metagenome assembly P1F0-k21, UrCas13d* from *Uncultured Ruminoccocus sp., RffCas13d* from *Ruminoccocus flavefaciens FD1*, and RaCas13d from *Ruminoccocus albus.* In one embodiment, the Cas13d protein is a *RfxCas13d* or a variant thereof. The amino acid sequences of the Cas13d orthologs are publicly available. In one embodiment, the Cas13d has an amino acid sequence as provided by a Protein Data Bank (PDB) accession number 6OAW_B or 6OAW_A or 6E9F_A or 6E9E_A or 6IV9_A, or an amino acid sequence as provided by the UniProtKB identifier B0MS50 (B0MS50_9FIRM) or A0A1C5SD84 (A0A1C5SD84_9FIRM).

In one embodiment, a variant of Cas13d may be a functional variant of the Cas13d protein which is a protein or a polypeptide which shares the same biological function with Cas13d. A functional variant of the Cas13d protein might be a Cas13d protein with 1, 2, 3, 4, 5, 6, 7, 8, 9, 10, 11, 12, 13, 14, 15, 16, 17, 18, 19, about 20, about 25, about 30, about 35, about 40, about 45, about 50, about 60, about 70, about 80, about 90, about 100, about 110, about 120, about 130, about 140, about 150, about 160, about 170, about 180, about 200, about 220, about 240, about 260, about 280, about 300, about 330, about 360, about 390 or more conserved amino acid substitution(s). Identifying an amino acid for a possible conserved substitution, determining a substituted amino acid, as well as the methods and techniques involved in incorporating the amino acid substitution into a protein are well-known to one of skill in the art. See, sift.jcvi.org/ and (Ng & Henikoff, Predicting the Effects of Amino Acid Substitutions on Protein Function, 2006; Ng & Henikoff, Predicting the effects of coding non-synonymous variants on protein function using the SIFT algorithm, 2009; Ng PC, 2003; Ng & Henikoff, Accounting for Human Polymorphisms Predicted to Affect Protein Function, 2002; Sim, et al., 2012; Sim, et al., 2012).

crRNA delivery in human cells can be challenging and the transient effects can be limited in half-life. Thus, modified crRNAs which provide increased targeting efficiency and/or half-life in human cells can be used. In other cases, the modified crRNAs provide improved activity and/or specificity compared to their unmodified sequence equivalents. In certain embodiment, the nucleic acid provided includes one or more crRNAs that include a chemical modification in one or more nucleotides. For instance, a spacer sequence that is about 23 nucleotides in length may have 1 or more, e.g., 1, 2, 3, 4, 5, 6, 7, 8, 9, or 10 more modified nucleotides, or a direct repeat sequence that is about 30 nucleotides in length may have 1 or more, e.g., 1, 2, 3, 4, 5, 6, 7, 8, 9, or 10 more modified nucleotides. The term "modified nucleotide" refers to a nucleotide that contains one or more chemical modifications (e.g., substitutions), in or on the nitrogenous base of the nucleoside (e.g., cytosine (C), thymine (T) or uracil (U), adenine (A) or guanine (G)), in or on the sugar moiety of the nucleoside (e.g., ribose, deoxyribose, modified ribose, modified deoxyribose, six-membered sugar analog, or open-chain sugar analog), or the phosphate. In some cases, the spacer sequence includes at least 2, 3, 4, 5, 6, 7, 8, 9, 10, or more modified nucleotides. The modified nucleotides can be located at any nucleic acid position of the crRNA sequence. The chemical modification can be in the direct repeat, the spacer sequence, or both. In one embodiment, the modified nucleotides can be at or near the first (5') and/or last (3') nucleotide of the spacer sequence, and/or at any position in between. In a particular embodiment, the modified nucleotides are at the 3' end of the spacer sequence. In some embodiments, the phosphate backbone of the modified crRNA is altered. The modified crRNA can include one or more phosphorothioate (S), phosphoramidate (e.g., N3'-P5'-phosphoramidate (NP)), 2'-O-methoxy-ethyl (2'MOE), 2'-O-methyl-ethyl (2'ME), and/or methylphosphonate linkages. In some embodiments, the one or more modified nucleotides in the modified crRNA comprise phosphorothioate (S) linkages.

The crRNA and bcgRNA used herein can be synthesized by any method known to one of ordinary skill in the art. In some embodiments, the crRNA and/or bcgRNA is/are chemically synthesized. Methods are described in, e.g., Dellinger et al., J. American Chemical Society 133, 11540-11556 (2011); Threlfall et al., Organic & Biomolecular Chemistry 10, 746-754 (2012); and Dellinger et al., J. American Chemical Society 125, 940-950 (2003). Chemical modifications useful herein have been described for use in other Cas systems. See, e.g., US2018/0119140.

In certain embodiments, the CRISPR array comprises a stabilizing RNA element. We have shown that the addition of a stabilizing RNA element at the bcgRNA 3'end may antagonize nucleolytic decay and improve bcgRNA detection sensitivity. Stably structured RNA elements are known in the art (see, e.g., Mendez-Mancilla, A. et al. Cell Chem. Biol. 1-7 (2021) doi:10.1016/j.chembiol.2021.07.011). In certain embodiments, the stabilizing RNA element is a MALAT1-triplex structure (see Brown, J. A. et al. Proc. Natl. Acad. Sci. U. S. A. 109, 19202-19207 (2012). In certain embodiments, the stabilizing RNA element is a NEAT1 (MENβ) structure (see Brown, J. A. et al. Proc. Natl. Acad. Sci. U. S. A. 109, 19202-19207 (2012). In certain embodiments, the stabilizing RNA element is a spnpreQ$_1$ structure (see Kang, M. et al. Proc. Natl. Acad. Sci. U. S. A. 111, (2014). In certain embodiments, the stabilizing RNA element is an mpknot element (see Anzalone, A. V. et al. Nat. Methods 13, 453-458 (2016). In certain embodiments, the stabilizing RNA element is a ZIKA virus-derived xrRNA1 dumbbell (see Akiyama, B. M. et al. Science. 354, 1148-1152 (2016). In certain embodiments, the stabilizing RNA element is a evopreQ1 pseudoknot (see, e.g., Nelson, J. W. et al. Nat. Biotechnol. (2021) doi:10.1038/s41587-021-01039-7). In certain embodiments, the stabilizing RNA element is an evopreQ1 pseudoknot and improves detection sensitivity at least 3-fold, 4-fold, 5-fold, or 6-fold compared to a CRISPR array without a stabilizing RNA element. In certain embodiments, the stabilizing RNA element comprises the sequence set forth in SEQ ID NO: 39, 40, 41, 42, 43, or 44.

As used herein, the term "host cell" may refer to any target cell having a target RNA or suspected of having a target RNA. Thus, a "host cell," refers to a prokaryotic or eukaryotic cell that contains the CRISPR array described herein, that has been introduced into the cell by any means, e.g., electroporation, nucleofection, calcium phosphate precipitation, microinjection, transformation, viral infection, transfection, liposome delivery, membrane fusion techniques, high velocity DNA-coated pellets, viral infection and protoplast fusion. In certain embodiments herein, the term "host cell" refers to a cultured cell of any mammalian species for in vitro assessment of the compositions described herein. The term "host cell" may also refer to the packaging cell line that contains a production plasmid to generate a viral or non-viral vector describe herein.

In one embodiment, the host cell is a primary cell. The term "primary cell" refers to a cell isolated directly from a multicellular organism. Primary cells typically have undergone very few population doublings and are therefore more representative of the main functional component of the tissue from which they are derived in comparison to continuous (tumor or artificially immortalized) cell lines. In some cases, primary cells are cells that have been isolated and then used immediately. In other cases, primary cells cannot divide indefinitely and thus cannot be cultured for long periods of time in vitro. In other embodiments, the cell is a cultured cell, e.g., a mammalian cell or bacterial cell. In certain embodiments, the host cell is an immortalized cell line. In certain instances, the primary cell is a stem cell or an immune cell. Non-limiting examples of stem cells include hematopoietic stem and progenitor cells (HSPCs) such as CD34+ HSPCs, mesenchymal stem cells, neural stem cells, organ stem cells, and combinations thereof. Non-limiting examples of immune cells include T cells (e.g., CD3+ T cells, CD4+ T cells, CD8+ T cells, tumor infiltrating cells (TILs), memory T cells, memory stem T cells, effector T cells), natural killer cells, monocytes, peripheral blood mononuclear cells (PBMCs), peripheral blood lymphocytes (PBLs), and combinations thereof. In other embodiments, the primary cell or a progeny thereof (e.g., a cell derived from the primary cell) is returned (e.g., administered via any acceptable delivery system and delivery route) to the multicellular organism (e.g., human) after introducing the CRISPR array and the Cas polypeptide into the primary cell.

A "nucleic acid" or a "nucleotide", as described herein, can be RNA, DNA, or a modification thereof, and can be selected, for example, from a group including: nucleic acid encoding a protein of interest, oligonucleotides, nucleic acid analogues, for example peptide-nucleic acid (PNA), pseudocomplementary PNA (pc-PNA), locked nucleic acid (LNA) etc. In certain embodiments, the terms "nucleotide" "nucleic acid" "nucleotide residue" and "nucleic acid residue" are used interchangeably, referring to a nucleotide in a nucleic acid polymer. In a further embodiment, consecutive nucleotide residues refer to nucleotide residues in a contiguous region of a nucleic acid polymer.

Ribonucleic acid (RNA) is a polymeric molecule essential in various biological roles in coding, decoding, regulation and expression of genes. As used herein, RNA may refer to a CRISPR guide RNA, a messenger RNA (mRNA), a mitochondrial RNA, short hairpin RNAi (shRNAi), small interfering RNA (siRNA), a mature mRNA, a primary transcript mRNA (pre-mRNA), a ribosomal RNA (rRNA), a 5.8S rRNA, a 5S rRNA, a transfer RNA (tRNA), a transfer-messenger RNA (tmRNA), an enhancer RNA (eRNA), a small interfering RNA (siRNA), a microRNA (miRNA), a small nucleolar RNA (snoRNA), a Piwi-interacting RNA (piRNA), a tRNA-derived small RNA (tsRNA), a small rDNA-derived RNA (srRNA), a non-coding RNA (ncRNA), long (intergenic) non-coding RNA (lincRNA/lncRNA), a single-stranded RNA (ssRNA), a circular RNA (circRNA), a vault RNA (vRNA/vtRNA), a SmY RNA, a double-stranded RNA (dsRNA), a small Cajal body-specific RNA (scaRNA), an antisense RNA (aRNA/asRNA), a ribonuclease RNA (e.g. RNase P), a non-coding regulatory RNA (e.g. 7SK RNA), RNA-viruses or single stranded DNA. In one embodiment, the target RNA is an endogenous RNA, e.g., an mRNA. Additionally, or alternatively, the target RNA comprises/is a CDS. In another embodiment, the target RNA comprises/is a UTR (including a 5' UTR or a 3' UTR). In yet another embodiment, the target RNA comprises/is an intron. In certain embodiments, the target RNA is a non-coding transcript.

As used herein, deoxyribonucleic acid (DNA) is a polymeric molecule formed by deoxyribonucleic acid, including, but not limited to, genomic DNA, double-strand DNA, single-strand DNA, DNA packaged with a histone protein, complementary DNA (cDNA which is reverse-transcribed from a RNA), mitochondrial DNA, and chromosomal DNA.

Nucleic acid sequences described herein can be cloned using routine molecular biology techniques, or generated de novo by DNA synthesis, which can be performed using routine procedures by service companies having business in the field of DNA synthesis and/or molecular cloning (e.g. GeneArt, GenScript, Life Technologies, Eurofins). The nucleic acid sequences encoding aspects of a CRISPR-Cas editing system described herein are assembled and placed into any suitable genetic element, e.g., naked DNA, phage, transposon, cosmid, episome, etc., which transfers the sequences carried thereon to a host cell, e.g., for generating non-viral delivery systems (e.g., RNA-based systems, naked DNA, or the like), or for generating viral vectors in a packaging host cell, and/or for delivery to a host cells in a subject. In one embodiment, the genetic element is a vector. In one embodiment, the genetic element is a plasmid. The methods used to make such engineered constructs are known to those with skill in nucleic acid manipulation and include genetic engineering, recombinant engineering, and synthetic techniques. See, e.g., Green and Sambrook, Molecular Cloning: A Laboratory Manual, Cold Spring Harbor Press, Cold Spring Harbor, NY (2012).

As used herein, an "expression cassette" refers to a nucleic acid molecule which encodes one or more elements of a gene editing system, e.g. CRISPR array as described herein and/or a Cas enzyme. An expression cassette also contains a promoter and may contain additional regulatory elements that control expression of one or more elements of a gene editing system in a host cell. In one embodiment, the expression cassette may be packaged into the capsid of a viral vector (e.g., a viral particle). In one embodiment, such an expression cassette for generating a viral vector as described herein is flanked by packaging signals of the viral genome and other expression control sequences such as those described herein. In the embodiments relating to a nucleic acid molecule or a vector or uses thereof, a nucleic acid molecule encoding a CRISPR array may be in operative association with, for example, an RNA pol II promoter or RNA pol III promoter. A RNA pol III promoter is a promoter that is sufficient to direct accurate initiation of transcription by the RNA polymerase III machinery, wherein the RNA polymerase III (RNAP III and Pol III) is a RNA polymerase transcribing DNA to synthesize ribosomal 5S ribosomal RNA (rRNA), transfer RNA (tRNA), crRNA, and other small RNAs. A variety of Polymerase III promoters which can be used are publicly or commercially available, for example the U6 promoter, the promoter fragments derived from H1 RNA genes or U6 snRNA genes of human or mouse origin or from any other species. In addition, pol III promoters can be modified/engineered to incorporate other desirable properties such as the ability to be induced by small chemical molecules, either ubiquitously or in a tissue-specific manner. For example, in one embodiment the promoter may be activated by tetracycline. In another embodiment, the promoter may be activated by IPTG (lacI system). See, US5902880A and US7195916B2. In another embodiment, a Pol III promoter from various species might be utilized, such as human, mouse or rat.

The term "regulatory element" or "regulatory sequence" refers to expression control sequences which are contiguous with the nucleic acid sequence of interest (for example, a Cas13d coding sequence or a sequence for expressing a CRISPR array) and expression control sequences that act in trans or at a distance to control the nucleic acid sequence of interest. As described herein, regulatory elements comprise but are not limited to: promoter; enhancer; transcription factor; transcription terminator; efficient RNA processing signals such as splicing and polyadenylation signals (polyA); sequences that stabilize cytoplasmic mRNA, for example Woodchuck Hepatitis Virus (WHP) Posttranscriptional Regulatory Element (WPRE); sequences that enhance translation efficiency (i.e., Kozak consensus sequence); sequences that enhance protein stability; and when desired, sequences that enhance secretion of the encoded product. Also, see Goeddel; Gene Expression Technology: Methods in Enzymology 185, Academic Press, San Diego, CA (1990). Regulatory sequences include those which direct constitutive expression of a nucleic acid sequence in many types of target cell and those which direct expression of the nucleic acid sequence only in certain target cells (e.g., tissue-specific regulatory sequences). Furthermore, the CRISPR array or Cas13d can be delivered by way of a vector comprising a regulatory sequence to direct synthesis of the CRISPR array or Cas13d at specific intervals, or over a specific time period. It will be appreciated by those skilled in the art that the design of the vector can depend on such factors as the choice of the target cell, the level of expression desired, and the like.

As used herein, "operably linked" sequences or sequences "in operative association" include both expression control sequences that are contiguous with the nucleic acid sequence of interest (for example, a Cas13d coding sequence or a sequence for expressing a CRISPR array) and expression control sequences that act in trans or at a distance to control the nucleic acid sequence of interest.

The term "expression" is used herein in its broadest meaning and comprises the production of RNA, of protein, or of both RNA and protein. Expression may be transient or may be stable.

Expression cassettes can be delivered to a host cell via any suitable delivery system. Suitable non-viral delivery systems are known in the art (see, e.g., Ramamoorth and Narvekar. J Clin Diagn Res. 2015 Jan; 9(1):GE01-GE06) and can be readily selected by one of skill in the art and may include, e.g., naked DNA, naked RNA, dendrimers, PLGA, polymethacrylate, an inorganic particle, a lipid particle (e.g., a lipid nanoparticle or LNP), or a chitosan-based formulation.

In certain embodiments, one or more elements of gene editing system are encoded by a nucleic acid sequence that is delivered to a host cell by a vector or a viral vector, of which many are known and available in the art. In one embodiment, provided is a vector comprising an expression cassette as described herein. In one embodiment, the vector is a non-viral vector. In another embodiment, the vector is a viral vector. A "viral vector" refers to a synthetic or artificial viral particle in which an expression cassette containing a nucleic acid sequence of interest is packaged in a viral capsid or envelope. Examples of viral vectors include but are not limited to lentivirus, adenoviruses (Ads), retroviruses (γ-retroviruses and lentiviruses), poxviruses, adeno-associated viruses (AAV), baculoviruses, herpes simplex viruses. In one embodiment, the viral vector is replication defective. A "replication-defective virus" refers to a viral vector, wherein any viral genomic sequences also packaged within the viral capsid or envelope are replication-deficient, *i.e.,* they cannot generate progeny virions but retain the ability to infect cells.

In one embodiment, the vector is a non-viral plasmid that comprises an expression cassette described herein, e.g., naked DNA, naked plasmid DNA, RNA, and mRNA; coupled with various compositions and nano particles, including, e.g., micelles, liposomes, cationic lipid - nucleic acid compositions, poly-glycan compositions and other polymers, lipid and/or cholesterol-based - nucleic acid conjugates, and other constructs such as are described herein. See, e.g., X. Su et al, Mol. Pharmaceutics, 2011, 8 (3), pp 774-787; web publication: March 21, 2011; WO2013/182683, WO 2010/053572 and WO 2012/170930.

Plasmids, other cloning and expression vectors, properties thereof, and constructing/manipulating methods thereof that can be used in accordance with the present invention are readily apparent to those of skill in the art. In one embodiment, the elements of a gene editing system as described herein or the expression cassette as described herein are engineered into a suitable genetic element (a vector) useful for generating viral vectors and/or for delivery to a host cell, e.g., naked DNA, phage, transposon, cosmid, episome, etc., which transfers the sequences carried thereon. The selected vector may be delivered by any suitable method, including transfection, electroporation, liposome delivery, membrane fusion techniques, high velocity DNA-coated pellets, viral infection and protoplast fusion. The methods used to make such constructs are known to those with skill in nucleic acid manipulation and include genetic engineering, recombinant engineering, and synthetic techniques. See, e.g., Sambrook et al, Molecular Cloning: A Laboratory Manual, Cold Spring Harbor Press, Cold Spring Harbor, NY.

As used herein, the term "perturbation" refers to the effects on one more transcripts, genes, or gene products (including protein) as a result of a mutation or modification of a target sequence. Mutation or modifications include, e.g. small nucleotide insertions or deletions (indels) or a larger deletion, insertion, or inversion. In certain embodiments, the introduction a mutation or modification is referred to as "editing" or "gene editing".

As used herein, "gene editing system" refers to technologies or molecular machinery for modifying genetic material, typically with specificity for a particular gene or nucleic acid sequence (i.e., a target sequence). In certain embodiments, the gene editing system is a Clustered Regulatory Interspaced Short Palindromic Repeats (CRISPR) system introducing perturbations in one or more RNA target sequences.

In certain embodiments, a suitable CRISPR gene editing system includes, at a minimum, a Cas enzyme and a CRISPR array as provided herein. In certain embodiments, the gene editing system comprises a Cas13d as the editing enzyme and a CRISPR array provided herein. In certain embodiments, a vector is provided that includes a nucleic acid having an expression cassette comprising a nucleic acid sequence encoding a CRISPR array and a nucleic acid sequence encoding a Cas13d enzyme. Thus, in certain embodiments, a dual-vector system (as described for example in WO 2016/176191) is provided, wherein the gene editing system includes an expression cassette comprising a Cas13 gene under control of regulatory sequences which direct its expression and a second expression cassette comprising a CRISPR array as provided herein.

It is also noted that any embodiment listed with respect to a CRISPR array, a nucleic acid molecule, a vector, a composition, any other component, a method, or a use, may be combined with any other embodiments with respect to a CRISPR array, a nucleic acid molecule, a vector, a composition, any other component, a method, or a use.

*Methods*

One or more of the CRISPR arrays, nucleic acid molecules, RNPs, vectors, cells, and libraries described herein are useful in a variety of methods including without limitation, treating a disease associated with an abnormal RNA; screening functional RNA(s); knocking-down, detecting, or editing a target RNA; or detecting or editing splicing, alternative isoforms, intron retention or differential UTR usage, or binding but not degrading the target. In one aspect, a method of performing gene perturbation profiling is provided.

In one aspect, a method of performing gene perturbation profiling is provided. The method includes providing a host cell comprising a CRISPR array as described herein and a Cas enzyme, wherein the CRISPR-Cas enzyme introduces one or more perturbations in the cell transcriptome. The method further includes isolating RNA from the cell and performing reverse-transcription comprising contacting an RNA sample with a primer specific for the reverse-transcription handle and identifying the barcode sequence. The method further includes detecting expression of one or more transcripts or gene products. In some embodiments, the method further includes the use of one or more of flow cytometric analysis, cell-hashing, single-cell sequencing analysis, single cell RNA sequencing (scRNA-seq), Perturb-seq, CROP-seq, CRISP-seq, ECCITE-seq., and cellular indexing of transcriptomes and epitopes (CITE-seq). In some embodiments, the method includes amplifying the barcode sequence using a primer specific for the PCR handle.

In another embodiment, the method of performing gene perturbation profiling includes providing a host cell comprising a CRISPR array as described herein and a Cas enzyme, wherein the CRISPR-Cas enzyme introduces one or more perturbations in the cell transcriptome, and the host cell is labeled with one or fluorophore-conjugated antibodies, and sorted using flow cytometry. The method further includes isolating RNA from the isolate cell and performing reverse-transcription comprising contacting an RNA sample with a primer specific for the reverse-transcription handle and identifying the barcode sequence. The method further includes detecting expression of one or more transcripts or gene products. In some embodiments, the method further includes the use of one or more of flow cytometric analysis, cell-hashing, single-cell sequencing analysis, single cell RNA sequencing (scRNA-seq), Perturb-seq, CROP-seq, CRISP-seq, ECCITE-seq., and cellular indexing of transcriptomes and epitopes (CITE-seq). In some embodiments, the method includes amplifying the barcode sequence using a primer specific for the PCR handle.

To use the CRISPR arrays described herein, it may be desirable to provide them in conjunction with a nucleic acid that encodes a Cas13d protein. The nucleic acid encoding the Cas13d may be cloned into an intermediate vector for transformation into prokaryotic or eukaryotic cells for replication and/or expression. Intermediate vectors are typically prokaryote vectors, e.g., plasmids, or shuttle vectors, or insect vectors, for storage or manipulation of the nucleic acid encoding the Cas13 protein variant for production of the same. The nucleic acid encoding the Cas13d protein can also be cloned into an expression vector, for administration to a plant cell, animal cell, preferably a mammalian cell or a human cell, fungal cell, bacterial cell, or protozoan cell.

The CRISPR arrays as described herein and Cas13 polypeptide, mRNA encoding a Cas13d polypeptide, and/or recombinant expression vector comprising a nucleotide sequence encoding a Cas13d polypeptide variant or fragment thereof are introduced into the cell using any suitable method such as by electroporation. In certain instances, the CRISPR array is complexed with a Cas nuclease (e.g., Cas13d polypeptide) or a variant or fragment thereof to form a ribonucleoprotein (RNP)-based delivery system for introduction into a cell (e.g., an in vitro cell for transcriptome perturbation analysis). In other instances, the CRISPR array is introduced into a cell (e.g., an in vitro cell for transcriptome perturbation analysis) with an mRNA encoding a Cas nuclease (e.g., Cas13d polypeptide) or a variant or fragment thereof. In yet other instances, the CRISPR array is introduced into a cell (e.g., an in vitro cell for transcriptome perturbation analysis) with a recombinant expression vector comprising a nucleotide sequence encoding a Cas nuclease (e.g., Cas13 polypeptide) or a variant or fragment thereof.

In some embodiments, the RNA or DNA sequencing occurs by methods that include, without limitation, whole transcriptome analysis, whole genome analysis, barcoded sequencing of whole or targeted regions of the genome, and combinations thereof. In some embodiments, the method comprises detection of cell surface proteins using, e.g. flow cytometry. In certain embodiments, the methods, comprise detection or identification of a CRISPR array as provide herein in combination with profiling additional molecular modalities using methods described in the art, including for example single-cell sequencing analysis (e.g. 10X Genomics Multiome platform), single-cell RNA-sequencing (scRNA-seq) (See, e.g., Haque et al. A practical guide to single-cell RNA-sequencing for biomedical research and clinical applications, Genome Medicine, 9, Article number: 75 (2017); Hwang et al. Single-cell RNA sequencing technologies and bioinformatics pipelines. Exp Mol Med. 2018 Aug 7;50(8):96), cell-hashing (See, e.g., Stoeckius et al. Cell Hashing with barcoded antibodies enables multiplexing and doublet detection for single cell genomics. Genome Biol. 2018; 19: 224), Perturb-Seq. (See, e.g., Dixit et al. Perturb-seq: Dissecting molecular circuits with scalable single cell RNA profiling of pooled genetic screens. Cell. 2016 Dec 15; 167(7): 1853-1866.e17), CROP-seq (See, e.g., Datlinger et al. Pooled CRISPR screening with single-cell transcriptome readout Nat Methods. 2017 Mar;14(3):297-301), CRISP-seq (See, e.g., Jaitin et al. Dissecting Immune Circuits by Linking CRISPR-Pooled Screens with Single-Cell RNA-Seq Cell. 2016 Dec 15;167(7):1883-1896.e15), Expanded CRISPR-compatible CITE-seq (ECCITE-seq) (See, e.g., Mimitou et al. Multiplexed detection of proteins, transcriptomes, clonotypes and CRISPR perturbations in single cells. Nat Methods. 2019 May;16(5):409-41), and cellular indexing of transcriptomes and epitopes-seq (CITE-seq) (See, e.g., Stoeckius et al. Simultaneous epitope and transcriptome measurement in single cells. Nat Methods. 2017 Sep;14(9):865-868).

As used herein, "reverse transcription" refers to the process of copying the nucleotide sequence of an RNA molecule into a DNA molecule. Reverse transcription can be done by contacting an RNA template with an RNA-dependent DNA polymerase, also known as a reverse transcriptase. A reverse transcriptase is a DNA polymerase that transcribes single-stranded RNA into single-stranded DNA. Depending on the polymerase used, the reverse transcriptase can also have RNase H activity for subsequent degradation of the RNA template.

As used herein, "complementary DNA" or "cDNA" can refer to a synthetic DNA reverse transcribed from RNA through the action of a reverse transcriptase. The cDNA may be single-stranded or double-stranded and can include strands that have either or both of a sequence that is substantially identical to a part of the RNA sequence or a complement to a part of the RNA sequence.

Some embodiments include the use of primers. As used herein, a "primer" can refer to a short polynucleotide, generally with a free 3'-OH group, that binds to a target or template polynucleotide present in a sample by hybridizing with the target or template, and thereafter promoting extension of the primer to form a polynucleotide complementary to the target or template. Primers can include polynucleotides ranging from 5 to 1000 or more nucleotides. In some embodiments, the primer has a length of at least 4 nucleotides, 5 nucleotides, 10 nucleotides, 15 nucleotides, 20 nucleotides, 25 nucleotides, 30 nucleotides, 35 nucleotides, 40 nucleotides, 45 nucleotides, 50 nucleotides, 60 nucleotides, 70 nucleotides, 80 nucleotides, 90 nucleotides, 100 nucleotides, or a length within a range of any two of the foregoing lengths.

To obtain expression, a sequence encoding a Cas13 protein is typically subcloned into an expression vector that contains a promoter to direct transcription. Suitable bacterial and eukaryotic promoters are well known in the art and described, e.g., in Sambrook et al., Molecular Cloning, A Laboratory Manual (3d ed. 2001); Kriegler, Gene Transfer and Expression: A Laboratory Manual (1990); and Current Protocols in Molecular Biology (Ausubel et al., eds., 2010). Bacterial expression systems for expressing the engineered protein are available in, e.g., E. coli, Bacillus sp., and Salmonella (Palva et al., 1983, Gene 22:229-235). Kits for such expression systems are commercially available. Eukaryotic expression systems for mammalian cells, yeast, and insect cells are well known in the art and are also commercially available.

In one embodiment, the target RNA is mRNA and the modulation or perturbation comprises inhibition, cutting, editing or modulation of protein expression of the target RNA. Target RNAs include, without limitation, mRNA, viral RNAs, noncoding RNAs, miRNA, piRNAs, circRNAs, synthetic RNA and other RNAs.

To functionally test the presence of the correct transcript modulation, the target RNA can be analyzed by standard methods known to those in the art. For example, denaturing gel electrophoresis can be performed to determine whether the target RNA has been fragmented. Other techniques, including FACS can be performed, using antibodies directed to the proteins corresponding to the target RNAs. Other methods useful to test the presence of the correct transcript modulation include, without limitation, qPCR and RNA-seq.

In one aspect, a functional screening method is provided. The method comprises contacting one or more CRISPR array(s), and/or nucleic acid molecule(s), and/or vector(s), and/or a library as disclosed with a target cell of a cell culture, a tissue, or a subject. In one embodiment, the method comprises amplifying the nucleic acid molecule or the vector in the target cell, and optionally quantifying the nucleic acid molecule or the vector.

In one embodiment, a Cas13d protein is expressed by a nucleic acid molecule or a vector in the target cell. Thus, in the target cell, the CRISPR array forms a complex with a Cas13d or a variation thereof, and directs the complex to a target RNA. In a further embodiment, the nucleic acid molecule or vector is the same nucleic acid molecule or vector which comprises or expresses the CRISPR array(s). In another embodiment, the nucleic acid molecule or vector expresses the Cas13d protein but not the CRISPR array and thus, is referred to as "Cas13d molecule" or "Cas13d vector" as used herein. In one embodiment, the ratio of the Cas13d molecule (or Cas13d vector) to a CRISPR array (or nucleic acid molecule and/or vectors providing the crRNA) is about 100 to 1 to about 1 to 100, including each ratio therebetween. In one embodiment, the ratio is about 10 to 1, about 5 to 1, about 4 to 1, about 3 to 1, about 2 to 1, about 1 to 1, about 1 to 2, about 1 to 3, about 1 to 4, about 1 to 5, or about 1 to 10. In a further embodiment, the ratio is a molar ratio.

In one embodiment, the encoded Cas13d protein is a *Rfx*Cas13d from *Ruminococcus flavefaciens* strain XPD3002. Other Cas13d may also be utilized, for example, *Adm*Cas13d from *Anaerobic digester metagenome 15706, Es*Cas13d from *Eubacterium siraeum DSM15702, P1E0*Cas13d from *Gut metagenome assembly P1E0-k21*, UrCas13d from *Uncultured Ruminoccocus sp., Rff*Cas 13 d from *Ruminoccocus flavefaciens FD1*, and *Ra*Cas13d from *Ruminoccocus albus.* In a further embodiment, the Cas13d or a variant thereof further comprises a nuclear localization signal (NLS) or a cytosolic signal or a nuclear-export signal (NES). In yet a further embodiment, the Cas13d or a variant thereof is capable of nicking a target RNA. In one embodiment, the Cas13d or a variant thereof has been engineered and does not have a nuclease activity. In one embodiment, the Cas13d is conjugated to a reporter molecule.

In one embodiment, the method reduces level of one or more of target RNA(s) in a target cell. In a further embodiment, the method functionally knocks down or knocks out one or more gene(s) expressing the target RNA(s). In yet a further embodiment, the method knocks down or knocks out one or more gene(s) in a plurality of targets cells in parallel.

In certain embodiments, a selective pressure or a stimulus is applied to the target cells prior to, during or after the contacting step, which is referred to as a perturbation step. Such selective pressure or a stimulus includes, for example, a chemical agent or a biological agent or actively physically disturbing the target cell(s).

In certain embodiments, the method further comprises assessing cell viability, cell proliferation, cell apoptosis, cell death, cell phenotype, existence or concentration of a molecule (for example, the target RNA(s)), protein or cell marker expression, or response to a stimulus of a target cell, or a function which may be achieved by the cell culture, tissue, or subject comprising the target cell(s).

The method comprises contacting a biological sample with a CRISPR array (or a nucleic acid or a vector expressing the crRNA) as disclosed. In one embodiment, the CRISPR array is conjugated with a reporter molecule. In another embodiment, the method further comprises contacting the biological sample with a Cas13d or a variant thereof, prior to, concurrently with, or after the contacting step with the crRNA(s). In a further embodiment, the Cas13d or a variant thereof is expressed by a nucleic acid molecule or a vector as described herein (which may be the same nucleic acid molecule or vector providing a CRISPR array or a different one) in a target cell of the biological sample.

EXAMPLES

The following examples disclose both general and specific embodiments of the disclosed compositions and methods described herein, which should be construed to encompass any and all variations that become evident as a result of the teaching provided herein.

Example 1: Materials and Methods

*Cell culture and monoclonal cell line generation*

HEK293FT cells were acquired from ThermoFisher (R70007), NIH/3T3, and THP1 cells were obtained from ATCC (CRL-1658, TIB-202). HEK293FT and NIH/3T3 cells were maintained at 37°C with 5% $CO_2$ in DMEM with high glucose and stabilized L-glutamine (Caisson DML23) supplemented with 10% fetal bovine serum (Serum Plus II Sigma-Aldrich 14009C) and no antibiotics. THP1 cells were grown in RPMI supplemented with 10% FBS at 37°C with 5% $CO_2$ and no antibiotics. Doxycycline-inducible *Rfx*Cas13d-NLS HEK293FT, THP1, and NIH/3T3 cells (Addgene #138149), as well as doxycycline-inducible nuclease-inactive *Rfx*dCas13d-NLS HEK293FT, have been generated as described before (Wessels, H. H. et al. Nat. Biotechnol. 38, 722-727 (2020)). We sorted individual suspension THP1 cells using a flow cytometer (SONY SH800) to select single clonal lines. Each THP1-Cas13d clone was evaluated to provide homogenously strong CD46 knockdown using lentiviral integration of a single gRNA, puromycin selection, and flow cytometry.

Monoclonal Cas9-effector protein-expressing HEK293FT cell lines were generated using dilution plating as described above (Wessels, H. H. et al. Nat. Biotechnol. 38, 722-727 (2020)). For each Cas9-effector cell line, we evaluated multiple clones in their ability to provide homogenous and complete CD55 knockout or knockdown using lentiviral integration of a single sgRNA expressing cassette, puromycin selection, and flow cytometry. The cloning of KRAB-dCas9 and KRAB-dCas9-MeCP2 constructs have been described before (Morris, J. A. et al. bioRxiv 2021.04.07.438882 (2021)). For Cas9-nuclease, we previously cloned lentiCas9-Blast (Addgene #52962) (Sanjana, N. E. et al. Nat. Methods 11, 783-784 (2014)). All monoclonal CRISPR Cas effector expressing cells were maintained using 5μg/mL Blasticidin S (ThermoFisher A1113903).

*Cloning of individual Cas13 and Cas9 guide RNAs*

Cas13 guide RNA cloning was done as described previously (Sanjana, N. E. et al. Nat. Methods 11, 783-784 (2014)). Specifically, we cloned gRNA or barcode oligos into pLentiRNAGuide_001 (Addgene #138150). Guide RNA constructs with reverse transcription handles on the 3' end of the spacer sequence were synthesized and cloned together. Guide RNA constructs using CRISPR arrays used in data presented in FIG. 2A -FIG. 2D were cloned stepwise by introducing a guide along with a direct repeat and reconstituted BsmBI restriction sites to allow for serial cloning and extension of CRISPR arrays.

For direct capture Perturb-seq, we cloned all sgRNA-expressing constructs stepwise. First, we cloned dual sgRNA oligos into pJR85 and pJR89 as described before (Replogle, J. M. et al. Nat. Biotechnol. 38, 954-961 (2020)). In addition, we cloned single sgRNAs into custom sgRNA expressing plasmids. We used either a human U6 (hU6) promoter driving the sgRNA scaffold described in the 10x Genomics manual CG000184 Rev C (internal CS1) (pLCR36) or using the sgRNA cassette from pJR73 (Replogle, J. M. et al. Nat. Biotechnol. 38, 954-961 (2020) driven by a bovine U6 (bU6) promoter (pLCR67). The bU6 and sgRNA cassette from pLCR67 was then subcloned into the dual sgRNA plasmid to generate triple sgRNA expressing plasmids (see also FIG. 8B). For direct capture Perturb-seq, we used pLCR36 single sgRNA constructs for pools expecting one sgRNA, pJR85/pJR89 constructs for pools expecting two sgRNAs, and pJR85/pJR89/pLCR67 constructs for pools expecting three sgRNAs. All constructs were confirmed by Sanger sequencing. Plasmids (pLCR36 and pLCR67) will be made available on Addgene by the time of publication.

*Pooled Cas13d library design and cloning*

We design two libraries for pooled cloning, one to identify genes that lead to THP1 cell differentiation, and one for combinatorial targeting with CaRPool-seq. First, we designed a *Rfx*Cas13d gRNAs library for single gRNA expression targeting 439 individual genes. We selected 240 target genes that led to CD11b and CD14 upregulation in a previous Cas9-based pooled screen, in addition to 199 control genes involved in TLR4 signaling. For each, we selected the transcript with the highest isoform expression (CCLE - broadinstitute.org/ccle/datasets) and scored possible gRNA sequences using our previously described Cas13design algorithm (Wessels, H. H. et al. Nat. Biotechnol. 38, 722-727 (2020)). For each gene, we selected 10 gRNA from efficacy quartile Q4 (or Q3 if not sufficient Q4 gRNAs were found), while trying to evenly spread the gRNAs along the coding region. We only selected gRNAs without secondary target sites with 0-2 mismatches to the gRNA sequence (Guo, X. et al. Cell Genomics 1, 100001 (2021)). In total, we designed 4390 gRNAs and added 410 nontargeting control gRNAs (>3 mismatches to any hg19-annotated transcript). The gRNA library was cloned as described before (Konermann, S. et al. Cell 173, 665-676 (2018)). Pooled oligonucleotides were synthesized (Twist), amplified using 8x PCR reactions with 8 amplification cycles using a direct repeat specific forward primer. The amplicon was Gibson cloned into pLentiRNAGuide_001 and pLentiRNAGuide_002 (Addgene 138150, 138151). Complete library representation with minimal bias (90th percentile/10th percentile crRNA read ratio: 1.8 for both libraries) was verified by Illumina sequencing (MiSeq).

To design the CaRPool-seq library, we manually inspected all gRNA enrichments from the pooled screen library described above. For the 28 selected target genes, we picked the two most enriched (or depleted for CD14 and ATXN7L3) gRNAs, while avoiding overlapping gRNAs. For each gene, we paired the two gRNA with a non-targeting gRNA (n=28 single perturbations, n=58 arrays). For 17 genes, we designed all pairwise combinations (n = 132 gene pairs, n = 264 arrays). And for 9 genes we designed a subset of possible gene pairs within the same complex (n = 22 gene pairs, n = 44 arrays). We added 13 arrays nontargeting control arrays. In total, we design 385 arrays with 186 single or double perturbation constructs, each represented by two independent technical replicate gRNA combinations. We designed random 15mer sequences with hamming distance >4 to one another. We balanced the relative CRISPR array abundance by the effect on cell proliferation of the targeted genes and increased the number of array copies in the pool to minimize dropout in the CaRPool-seq experiment. The oligos for synthesis were designed in the following way:

PCR-handle::BsmBI-site::DR::gRNA1::DR::gRNA2::LguI-bridge::
barcode::BsmBI-site::PCR-handle

Pooled oligonucleotides were synthesized (Twist) and diluted to 1 ng/ul before amplification. The outer PCR-handles allowed for PCR amplification of the oligo pool (we used Pfu-Ultra- II following the manufacturer's recommendation using 1 $\mu$l of enzyme and 20ng of oligo pool in a 50 $\mu$l reaction 95C/2m, 5x[95C/20s, 58C/20s, 72C/15s], 72C/3min ). The amplicon was purified using a 2x SPRI cleanup followed by BsmBI-digestion, and SPRI cleanup. All of the purified product was ligated into BsmBI-digested pLCR65 using T7-DNA ligase and cloned as described before (Wessels, H. H. et al. Nat. Biotechnol. 38, 722-727 (2020)) with > 1000 colonies per construct. The resulting plasmid pool was digested with LguI to enable ligation of the third DR and smallRNA-handle to complete the bcgRNA and CRISPR array. pLCR65 was generated by removing all LguI sites from the pLentiRNAGuide_002 plasmid, introducing the CS1 capture sequence 5' to the terminator sequence, and replacing the puromycin gene with GFP-P2A-puromycin. The LguI insert containing the third DR and smallRNA handle was cloned into pLentiRNAGuide_001, digested with LguI, and gel-purified (2% eGel). Complete library representation with minimal bias (90th percentile/10th percentile crRNA read ratio: 2.6-4.8 for the relative abundance tiers and 5.0 overall) and correct gene pair to bcgRNA linkage (>94%) was verified by Illumina sequencing (MiSeq). During library cloning, we noticed two critical details: Alternative polymerase KAPA and Q5 can lead to a stronger bias in relative array abundance. Additionally, reducing the number of PCR cycles with increased oligo pool input amounts can decrease bcgRNA reassortment. Further, while we chose a two-step cloning strategy, we believe that a single step cloning strategy may yield similar results. pLCR65 will be deposited to Addgene by the time of publication.

*Virus production and viral transductions*

For virus production of individual gRNA and sgRNA expressing plasmids, we seeded 1x10^6 HEK293FT cells per well in 6-well format 12-18 hours before transfection. Per transfection reaction we used 7.5ul of 1 mg/mL polyethyleneimine (PEI) and 2.325 $\mu$g of total plasmid DNA (825 ng psPAX2: Addgene #12260; 550 ng pMD2.G: Addgene #12259; 1000 ng of gRNA/sgRNA expressing plasmid). Six to eight hours posttransfection, the medium was exchanged for 2 ml of DMEM + 10% FBS containing 1% bovine serum albumin (BSA). Viral supernatants were collected after additional 48 hours, spun down to remove cellular debris for 5 min at 4°C and 1000xg, and stored at -80C until use.

For pooled virus production the plasmid pools were transfected as described above in a 10 cm dish format (1x10$^7$ HEK293FT cells, 70$\mu$l PEI, 6.4 $\mu$g psPAX2, 4.4 $\mu$g pMD2.G and 9.2 $\mu$g of the plasmid pool). The pooled virus was cleared by spinning down cell debris (3min, 1000xg) and passed through a 0.45 $\mu$m filter prior to storage at -80°C. For the CaRPool-seq experiments presented in FIG. 1A - FIG. 1E and FIG. 2A - FIG. 2D, and the direct capture Perturb-seq experiments presented in FIG. 3A - FIG. 3E, we pooled individual plasmids at equal amounts. In this way, we generated separate sgRNA pools for 1, 2, or 3 sgRNAs to a total of 6 pools (3 for Cas9-nuclease and 3 for KRAB-dCas9(-MeCP2)).

For experiments using single gRNA or CRISPR arrays, we transduced 1x10$^6$ HEK293FT or THP1 cells with an MOI of 0.2-0.5. The cells were selected with 1$\mu$g/ml puromycin (ThermoFisher A1113803) starting at 24 hours post-transduction for at least 48 hours for HEK293FT cells and 5 days for THP1. For CaRPoolseq experiments (FIG. 1A -FIG. 1E and FIG. 2A - FIG. 2D) and direct capture Perturb-seq experiments (FIG. 3A - FIG. 3E), we transduced 3x10$^6$ cells HEK293FT or NIH/3T3 cells. For screens conducted in THP1 cells we transduced at least 12x10$^6$ cells per condition. HEK293FT and NIH/3T3 cells were selected for at least two days, and THP1 cells for at least 5 days. For single-cell screens we selected conditions with < 10% survival 48 hours post puromycin selection or fraction of GFP-positive cells below 10% (MOI < 0.1), assuring high coverage (>1000x representation) and a single integration probability >95%. Pooled screens were conducted with MOIs between 0.13 and 0.45 always maintaining coverage of >1000x. Cas13d expression was induced using 1$\mu$g/ml doxycycline (Sigma D9891). Cells were maintained with doxycycline, blasticidin, and puromycin until the single-cell experiment. During this period, the cells were passaged every 2-3 days into fresh media supplemented with doxycycline, blasticidin, and puromycin.

*Flow cytometry*

Puromycin-selected cells were harvested 2-7 days after selection start (HEK cells) or Cas13d induction (THP1 cells). dCas9 sgRNAs were evaluated 7 days post-transduction. Cells were stained for the respective cell surface protein for 30 min at 4°C and measured by fluorescence-activated cell sorting (Sony SH800) (BioLegend: CD46 clone TRA-2-10 #352405 - 3$\mu$l per 1x10$^6$ cells; CD55 clone JS11 #311311 - 5$\mu$l per 1x10$^6$ cells; CD71 clone CYIG4 #334105 - 4$\mu$l per 1x10$^6$ cells, CD11b clone ICRF44 #301322 - 2$\mu$l per 1x10$^6$ cells). For flow cytometry analysis (FlowJo v10), cells were gated by forward and side scatters and signal intensity to remove potential multiplets. If present, cells were additionally gated with live-dead staining (LIVE/DEAD Fixable Violet Dead Cell Stain Kit, Thermo Fisher L34963). For each sample, we analyzed at least 5,000 cells. If cell numbers varied, we always subsampled (randomly) all samples to the same number of cells within an experiment.

*Bulk guide RNA detection*

Puromycin-selected cells were harvested 48 hours or more after selection start. RNA was extracted from ~1x10$^6$ cells (Zymo Direct-zol RNA microPrep). Total RNA was reverse transcribed using a capture sequence-specific reverse transcription primer along with an oligo(dT)V30 primer (400ng RNA, 4$\mu$l 5x RT Buffer, 1$\mu$l SuperasIN, 1$\mu$l dNTPs (10mM each), 1$\mu$l Maxima H Minus RT enzyme, 1.5$\mu$l 10$\mu$M Template Switch Oligo, 1$\mu$l 10$\mu$M oligo(dT)V30 primer, 1$\mu$l 10$\mu$M gRNA capture sequence primer, 20$\mu$l reaction volume; 53°C/90min, 70°C/15min). The Cas13 crRNA and *GAPDH* mRNA was amplified from 1:2 diluted cDNA using partial TSO and capture sequence primers or gene-specific primers (3$\mu$l cDNA, 10$\mu$l KAPA 2x master mix, 1$\mu$l primer (10 $\mu$M each), 5$\mu$l H2O; PCR conditions: 98°C/45s, 18x[98°C/20s, 60°C/10s, 72°C/10s], 72°C/5min). Oligonucleotides are provided in Supplementary Table 2.

*Cas13d gRNA off-target prediction*

To identify potential Cas13d gRNA off-targets (alternative binding sites) we first aligned gRNAs to the human transcriptome (Grch38 cdna.all and ncRNA from emsembl release 97) using blastn (megablast) with the following parameters (-strand minus - max_target_seqs 10000 -evalue 10000 -word_size 5 - perc_identity 0.7). Secondly, candidates were further filtered to match with at least 17 bases, as shorter matches do not lead to target knockdown, and show a blastn e.value of < 100. In FIG. 6E, we demonstrate that despite the potential for reduced off-target binding, we do not observe transcriptomic perturbation for these genes.

*Bulk RNA-seq*

To more carefully test for the potential of Cas 13 to introduce off-target effects, we performed a bulk RNAseq experiment, where we expect to have additional power to detect differential expression for lowly expressed transcripts. For the bulk RNA-seq experiment, we performed CD55 knockdown (Cas13d cells, KRAB-dCas9-MeCP2 cells) or knockout (Cas9-nuclease cells) using three individual targeting (s)gRMAs and three individual non-targeting (s)gRMAs. Monoclonal HEK293FT cell lines were transduced with a guide expressing lentivirus (MOI 0.2-0.5) in three independent transductions. Puromycin selection was started 24 hours post-transduction (1μg/mL), and Cas13d expression was induced (1μg/ml Doxycycline). Seven days post-transduction, we confirmed efficient CD55 targeting using flow cytometry, and total RNA was extracted (Zymo Direct-zol RNA microPrep). We performed a modified version of the Smart-seq2 protocol using 100ng purified total RNA input (protocols.io/view/barcoded-plate-basedsingle-cell-rna-seq-nkgdctw). Bulk RNA-seq samples were processed with Drop-seq tools v1.0 28 using a hg19 reference. On average we obtained 352611 UMI (+/-72067 UMI) per sample. Differential gene expression was assessed with Seurat's DESeq2 (Love, M. I. et al. Genome Biol. 15, 550 (2014)) implementation in FindMarkers.

*Pooled CRISPR screens in THP1 cells*

Experimental procedures for performing multiplexed Cas13d screens in bulk were performed as described before (Wessels, H. H. et al. Nat. Biotechnol. 38, 722-727 (2020)), with minor modifications. THP1 cells were transduced and puromycin-selected as described above. Cas13d expression was induced after cells were fully selected (1μg/mL Doxycycline). Growth medium with fresh puromycin, blasticidin, and doxycycline was replenished every 2-4 days, and cells were split as needed always maintaining a guide representation of >1000x.

For the single guide RNA pooled screen, we collected a 1000x representation at 7 and 14 days post Cas13d induction and before sorting. After two weeks (14-16 days) we stained 15 million cells (~3000x representation), using FcX-blocking buffer (BioLegend #422302; 10 min at room temperature) and followed by either CD11b (BioLegend clone ICRF44 #301322 - 4μl per 1x106 cells) or CD14 (BioLegend clone HCD14 #325608- 4μl per $1x10^6$ cells) staining (30 min at 4°C), and finally resuspending cells on PBS with DAPI (Sigma #D9542 - 0.4μg ml-1) to detect any apoptotic or dead cell. We sorted the cells (Sony SH800) based on their signal intensities (CD11b or CD14: lowest 10-15%, and highest 10-15%). Cells were PBS-washed and frozen at -80°C until sequencing library preparation. In total, we prepared four independent transductions (two MOIs and two alternative direct repeats), performed CD14 sorts for all four transduction replicates, and CD11b sorts for three transduction replicates collecting $1x10^6$ to $1.5x10^6$ cells per bin.

For the combinatorial targeting pooled screen, we prepared three transduction replicates (MOI 0.13-0.2). Eight days post Cas13d induction, we collected an input representation (>1000x coverage) and stained 20-30mio cells with FcX-blocking, CD11b, and DAPI as described above. We sorted the cells based on their CD11b signal intensity (lowest 15%, and highest 15%). Cells were PBS-washed and frozen at -80°C until sequencing library preparation. Library preparations for the single gRNA pooled screen were done as described before (Wessels, H. H. et al. Nat. Biotechnol. 38, 722-727 (2020)). For the combinatorial targeting pooled screen, we adopted a PCR strategy similar to the CaRPool-seq bcgRNA readout. Pooled screen readout PCR1 remained unchanged. In PCR2, we amplified the 15bp barcode sequence using a soluble Nextera-Read1-CS1 feature capture primer including an optional 28 randomized bases mirroring UMI and cell barcode, and RPIx Read2 i7 index primer. The amplicon was completed in PCR3 using Feature SI primer 2 (10x Genomics) and P7 primer.

*Pooled CRISPR screen analysis*

Reads were demultiplexed based on Illumina i7 barcodes present in PCR2 reverse primers using bcl2fastq and, if applicable, by their custom in-read i5 barcode using a custom python script. For the single gRNA pooled screen, read1 sequencing reads were trimmed to the expected gRNA length by searching for known anchor sequences relative to the guide sequence using a custom python script. For the combinatorial pooled screen, we extracted the first 15 bases in read2. For the single gRNA pooled screen, we collapsed (FASTXToolkit) processed reads to count perfect duplicates followed by string-match intersection with the reference to retain only perfectly matching and unique alignments (average mapping rate 82.3%; median gRNA count 167). For the combinatorial pooled screen, pre-processed reads were either aligned to the barcode reference using bowtie (Langmead, B. et al. Genome Biol. 10, (2009)) (v.1.1.2) with parameters -v 1 -m 1 --best -strata (average mapping rate 97%; median barcode read count 635; 1 barcode was not detected in input samples). For each dataset, raw counts were normalized using a median of ratios method as in DESeq2 29 and batch-corrected using combat implemented in the SVA R package (Leek, J. T. et al. Bioinformatics 28, 882-883 (2012)). Guide RNA and barcode enrichments were calculated building the count ratios between a sorting bin or timepoint and the indicated reference sample followed by log₂-transformation (log₂FC). For every single gRNA or bcgRNA, we considered the mean log₂FC across replicates. For the single gRNA pooled screen, used the four best performing gRNAs per target gene to calculate the mean log₂FC, where we determined best as either highest or lowest dependent on the sign of the mean enrichment across all ten gRNAs. As we have previously described (Wessels, H. H. et al. Nat. Biotechnol. 38, 722-727 (2020)), we noticed that log₂FC enrichments were generally more pronounced in samples using the enhanced DR. Consistency between replicates and selected gRNAs was estimated using robust rank aggregation (RRA) (Kolde, R. et al. Bioinformatics 28, 573-580 (2012)). For the combinatorial pooled screen, we calculated the mean of both replicate arrays per gene pair. We noticed GFI1 g2 did not lead to strong effects in the pooled screen and in the CaRPool-seq experiment. The technical replicate arrays including GFI1 g2 were removed in all analyses.

*Direct capture Perturb-seq*

Monoclonal CRISPR-Cas effector protein-expressing cell lines (Cas9-nuclease, KRAB-dCas9, KRABdCas9-MeCP2) were infected with one of six sgRNA pools (KO-1, KO-2, KO-3, or CRISPRi-1, CRISPRi-2, CRISPRi-3), providing 1-3 sgRNAs in a single vector and a total of nine cell line pool combinations. Cell survival after selection ranged between 1.7 and 5.5% (MOI < 0.1) assuring a high single integration probability. Viral titers were confirmed by measuring the fraction of BFP-positive cells for pools that have received vectors carrying 2+ sgRNAs using flow cytometry. Cells were passaged every 2-3 days continuously receiving puromycin and blasticidin at each split maintaining high sgRNA representation (>1000x coverage). We confirmed that >98% of cells were BFP-positive prior to the 10x experiment. We performed 10x V3.0 (Chromium Single Cell 3' Gene Expression v3 with Feature Barcoding technology for CRISPR screening, #1000074, #1000075, #1000079) twelve days post-transduction. Cells were stained with a pool of five TotalSeq-A antibodies (0.75ug per antibody per $2x10^6$ cells) following the CITE-seq protocol (Stoeckius, M. et al. Nat. Methods 14, 865-868 (2017)). In addition, we used Cell Hashing (Stoeckius, M. et al. Genome Biol. 19, 1-12 (2018)) to track the nine cell line pool combinations. Before the run, cell viability was determined ($\geq$96%). We ran one 10x lane, leveraging our hashed experimental design to overload with 38,600 cells. mRNA, sgRNA feature, hashtags (Hashtag-derived oligos, HTOs), protein (Antibody-derived oligos, ADTs) libraries were constructed by following 10x Genomics Cell-hashing and CITE-seq protocols. All libraries were sequenced together on one NextSeq 75 cycle high-output run.

*Direct capture Perturb-seq analysis*

Sequencing reads coming from the mRNA library were mapped to the *hg38* (ensembl v97) genome reference using *Cellranger* (v3.1). Guide RNA reads were mapped simultaneously to a sgRNA feature reference. Prior to feature mapping, we performed 5' adapter trimming using cutadapt to account for varying lengths of poly-G tracks five prime to the feature and trimmed the resulting sgRNA reads to a length of 18 bases. To generate count matrices for HTO and ADT libraries, the *CITE-seq-count* package (v1.4.2) was used (github.com/Hoohm/CITE-seq-Count). Count matrices were then used as input into the Seurat R package (v4.0) (Hao, Y. et al. Cell 184, 3573-3587.e29 (2021)) to perform downstream analyses. We detected 16842 cells. HTO and sgRNA counts were normalized using the centered log-ratio transformation approach, with a margin = 2 (to normalize across cells instead of across features). To identity assign experimental conditions to cells and remove cell doublets, we used the *HTODemux* function in Seurat, with default parameters.

We customized *HTODemux* to return identities of second and third sgRNA assignments without changing the underlying modeling approach. We flagged cells with an incorrect number of expected sgRNAs numbers based on the HTO pool assignment. Furthermore, we flagged cells with an unexpected combination of sgRNAs not present in the sgRNA pool used to transduce the cells.

For the analysis shown in FIG. 3A - FIG. 3E, we only retained cells with the correct sgRNA numbers and identities. ADT counts were log-normalized, before running ScaleData with do.scale = FALSE and vars.to.regress = "PerturbSeq.approach". PCA was performed on the log-normalized and regressed ADT counts using all five features, followed by UMAP dimensional reduction using four dimensions. To compare target knockdown across Perturb-seq approaches for NT-cell and cells that received all three (s)gRNAs (CD46, CD55, CD71), we normalized cellular ADT counts using median of ratios across ADT features that were not targeted (CD29, CD56) to derive a scaling factor per cell, and divided the normalized ADT counts by the mean ADT counts in NT cells for each Perturb-seq approach.

*Cas13 RNA Perturb-seq (CaRPool-seq) library preparation*

We used Cas13 CRISPR array configurations of type X (extra PCR handle) as shown in FIG. 1A and FIG. 5. Specifically, the bcgRNA was placed in the last array position and entailed a spacer sequence composed of a five-prime Illumina smallRNA PCR handle, a 15 base pair long barcode, and a three-prime capture sequence 1 (CS 1) compatible with 10x Feature Barcoding technology. This composition allowed the specific amplification of a bcgRNA amplicon with a unique combination of forward and reverse primers. Moreover, usage of the Illumina 5' PCR handle allows for efficient sequencing of the bcgRNA amplicon with the first base of Read 2 being the first barcode base. While this configuration has many advantages, other PCR primer sequences or capture sequences may be possible.

CaRPool-seq experiments were conducted using the 10x Genomics 3' kit (Chromium Single Cell 3' Gene Expression v3 with Feature Barcoding technology for CRISPR screening, #1000074, #1000075, #1000079). Library construction for bcgRNA derived oligos is outlined in FIG. 5 and largely followed 10x Genomics user guide CG000184 Rev C for Feature Barcoding with some modifications. Specifically, we eluted the GEM-RT in 33ul and added 2$\mu$l containing 0.4$\mu$M ADT additive primer (for bcgRNAs and ADTs) and 0.2$\mu$M HTO additive primer prior to cDNA amplification. The cDNA was purified using 0.6x SPRI cleanup for mRNA fraction. The supernatant containing ADT, HTO, and bcgRNA cDNA was purified was purified by adding another 1.4x SPRI (0.6+1.4 = 2x SPRI) followed by an additional 2x SPRI clean up. The purified short fragments were split evenly into three pools (e.g. 3 x 20µl). One pool each was used for HTO and ADT library construction as described below. Half of the remaining pool (10µl) was used to construct the bcgRNA library using two PCR recipes. PCR1 adds Illumina P5 and P7 handles to the bcgRNA amplicon (100µl PCR1: 50µl 2x KAPA Hifi PCR Mastermix, up to 45µl bcgRNA PCR template, 2.5µl Feature SI Primers 2 10µM, 2.5µl TruSeq Small RNA RPIx primer (containing i7 index) 10µM; 95°C 3min, 12x[95°C 20sec, 60°C 8sec, 72°C 8sec], 72°C 1min). PCR2 amplifies with P5 and P7 primers (100µl PCR2: 50µl 2x KAPA Hifi PCR Mastermix, up to 45µl purified PCR1 product, 2.5µl P5 primer 10µM, 2.5µl P7 primer 10µM; 95°C 3min, 4x[95°C 20sec, 60°C 8sec, 72°C 8sec], 72°C 1min). The final bcgRNA amplicon has a length of 203bp and can be sequenced on a standard Illumina sequencing platform with standard Illumina sequencing primers ($\geq$28 cycles Read1 and $\geq$15 cycles Read 2) (FIG. 5 and FIG. 6A).

*CaRPool-seq experiments*

We transduced and treated Cas13d-NLS expressing HEK293FT, NIH/3T3, or THP1 cells as described above. In the species mixing, we used a pool of three bcgRNAs per species together with non-targeting gRNAs. The HEK293FT CaRPool-CITE-seq experiment included 29 CRISPR arrays barcoding a diverse set of array configurations around four gRNAs that allowed us to assess gRNA positioning within the CRISPR array, effects of the relative gRNA amount per cell, and combinatorial targeting of multiple RNA transcripts. CaRPool-seq species mixing and CaRPool-CITE-seq were conducted simultaneously in one lane of 10x Genomics 3' v3 kit. CaRPool-seq was performed on THP1 cells five days post Cas13d induction (1µg/mL Doxycycline) using four lanes of a10x Genomics 3' v3 kit. THP1 CaRPool-seq library design and cloning were described above. Prior to the runs, cell viability was determined [3] 95% for each experiment.

The HEK293FT CaRPool-seq experiment was stained with a pool of five TotalSeq-A antibodies (0.75ug per antibody per $2x10^6$ cells) as following the CITE-seq protocol (Stoeckius, M. et al. Nat. Methods 14, 865-868 (2017)). Similarly, THP1 cells were first treated with FcX-blocking buffer (BioLegend #422302; 10 min at room temperature), before staining cells with a pool of 22 TotalSeq-A antibodies. To keep track of the experiment identity and identify multiplets, samples were hashed (subsequent to CITE-seq antibody staining) following the Cell Hashing protocol (Stoeckius, M. et al. Genome Biol. 19, 1-12 (2018)). mRNA, hashtags (Hashtagderived oligos, HTOs), protein (Antibody-derived oligos, ADTs) libraries were constructed by following 10x Genomics Cell-hashing and CITE-seq protocols.

Species mixing and HEK293FT CaRPool-seq experiment libraries were sequenced together on one NextSeq 75 cycle high-output run. THP1 CaRPool-seq libraries were sequenced on NovaSeq6000 using the XP S4 2x100 v1.5 workflow. Sequencing reads coming from the mRNA library were mapped to a joined genome reference of *hg38* (ensemble v97) and *mm10* using the *Cellranger* Software (v3.0.1), or to *hg38* using *Cellranger* v6.0.0 for the THP1 experiment. Barcode guide RNA library reads were mapped simultaneously to a barcode reference using *Cellranger.* To generate count matrices for HTO and ADT libraries, the *CITE-seq-count* package (v1.4.2) was used (github.com/Hoohm/CITE-seq-Count). Count matrices were then used as input into the *Seurat* R package (v4.0) 33 to perform all downstream analyses.

*CaRPool-seq data analysis*

Cells from species-mixing and HEK293FT CaRPool-seq experiments were processed together. Cells with <2,500 UMI were removed. HTO and bcgRNA counts were normalized using the centered log-ratio transformation approach, with a margin = 2 (to normalize across cells instead of across features). To identity cell doublets and assign experimental conditions to cells, we used the *HTODemux* function. Only human cells were hashed, with mouse NIH/3T3 cells being the only cell population without a hashtag. We removed all hashing doublets within the CaRpool-CITE-seq experiment (HTO-01 to HTO-08) and to human cells in the species mixing experiment (HTO-10). In addition, we removed all cells labeled with a single HTO-01 to HTO-08 if the fraction of mouse reads was >10%, and cells without any HTO if not at least 10% mouse reads were present. Like this, we removed all doublets between CaRPool-seq species mixing and CaRPool-CITE-seq experiments while retaining potential collisions/doublets between mouse and human cells as part of the CaRPool-seq species mixing branch. At this point, the experiment was split into two separate objects. For the CaRPool-seq species mixing experiment, we determined species identity by quantifying the fraction of human reads for RNA and for the species-specific bcgRNAs (human: > 0.9, mouse: < 0.1, collision: 0.9 to 0.1). For the HEK293FT CaRPool-CITE-seq experiment RNA counts were log-normalized using the standard Seurat workflow after removing all mouse features and RNA counts. Barcode guide RNA identity was determined using *MultiSeqDemux( autoThresh = T )*. Cells without bcgRNA assigned and cells with multiple bcgRNA assignments were removed.

For the THP1 experiment we detected 52,496 single cells (nFeature_RNA > 1000, nFeature_RNA < 8000, percent.mt < 20) after HTO demultiplexing using *HTOdemux* as described above. Model-based bcgRNA assignments (*HTODemux* or *MultiSeqDemux*) did not yield satisfying results supported by the observed phenotypic changes, likely due to model limitations imposed by the high number of bcgRNA features. Instead, we assigned bcgRNAs to single cells by applying the following rules:

We compared UMI counts for the bcgRNA with the highest UMI count (g1) to, if present, the second detected bcgRNA (g2). bcgRNA counts for g2 may derive from spurious counts arising from library preparation, or from integration of more than one viral element (bcgRNA multiplet). We considered cells with g1 < 5 as Negative. We assigned g1 if: 1) g1 = {5-9} and g2 = {0-1}, or 2) g1 > 9 and g1/(g1+g2) > 0.8 and g2 < 11. All other cells were considered bcgRNA multiplets. We assigned 31,308 with a single bcgRNA. Comparing differential gene expression results for technical replicates embedded in the CaRPoolseq library, we noticed GFI1 g2 did not lead to upregulation of CD11b ADT and did not lead to upregulation of the expected gene expression signature. We removed all cells with GFI1 g2 (n=601). Changes in cell surface protein ADT levels for gene pair or individual CRISPR array were calculated using Wilcoxon's rank-sum test in FindMarkers relative to NT control cells. Changes were determined by repeating the differential expression analysis ten times with <= 30 randomly samples cells per cell group to account for differing numbers of cells.

*Extrapolation of sgRNA detection in direct capture Perturb-seq experiments*

We used published sgRNA assignment rates for single and dual sgRNA targeting using direct sgRNA capture via Feature Barcoding technology (Replogle, J. M. et al. Nat. Biotechnol. 38, 954-961 (2020)). We determined the mean sgRNA assignment rate to be 80.9% by averaging the assignment rate for exactly one sgRNA (80% in single guide experiments) and taking the square root of the assignment rate of exactly two sgRNAs per cell (67% in dual guide experiments). In our simulation, we assume that a single viral particle will be taken up by a cell during a low-MOI infection. A single integration event may deliver up to three sgRNAs that are independently expressed, similar to the two sgRNA experiments described previously (Replogle, J. M. et al. Nat. Biotechnol. 38, 954-961 (2020)). We assume that sgRNA-detection for each sgRNA is an independent event. These can be modeled by multiplying detection and editing probabilities $p$ by the number of sgRNA feature $n$ ($pn$). The resulting curve shows the fraction of cells that have received exactly $n$ sgRNAs (FIG. 3B).

*Modeling of genetic interactions (GI) in single-cell data*

To decompose transcriptomic profiles of double perturbation, we used a linear regression model as previously introduced (Norman, T. M. et al. Science. 365, 786-793 (2019)) and implemented it in R. First, we z-scaled the log-normalized gene expression counts for all cells with respect to the mean and standard deviation of the control group (non-targeting cells). In this way, we have subtracted the baseline expression profiles from each cell and can directly compare the deviation from each perturbation to NT conditions. Next, we grouped cells by gene pair and calculated pseudo-bulk z-scaled profiles [single perturbations (a, b), and double perturbation (ab)] by calculating the mean across cells for each feature. The average NT-cell profile returns a vector of all zeros. We generated average profiles for 1,530 genes with an average UMI count > 0.5. We included gene pairs when all cell groups were represented by at least 25 cells.

As previously introduced (Norman, T. M. et al. Science. 365, 786-793 (2019), we model the average z-scale profiles using:

$$\delta ab = c1\delta a + c2\delta b + \epsilon$$

With $\delta a$ is the pseudobulk z-scaled profile for cells assigned to single perturbation a, $\delta b$ is the pseudobulk z-scaled profile for cells assigned to single perturbation b, and $\delta ab$ is the pseudobulk z-scaled profile for cells assigned to double perturbation ab. $c_1$ and $c_2$ are constants fitted to the data indicating the relative weight of $\delta a$ and $\delta b$ profiles. The vector $\varepsilon$ collects the residuals to the model fit. In our plots, a is the first gene in the gene pair, and b is the second gene. $c_1$ corresponds to a, and $c_2$ to b.

We implemented the previously-introduced model-fitting procedure (Norman, T. M. et al. Science. 365, 786-793 (2019)), using the *rlm* function from the MASS package, and extracted the mean coefficients ($c_1$ and $c_2$) and residual error $\varepsilon$. We collected six measures to evaluate the fit as described before (Norman, T. M. et al. Science. 365, 786-793 (2019)) (*dcor* function from energy package):

| | |
|---|---|
| Model fit: | dcor( $c_1$a + $c_2$a, ab ) |
| Dominance: | \|log10($c_1$ / $c_2$)\| |
| Magnitude: | ($c_1$2 + $c_2$2)$^{1/2}$ |
| Similarity of single to double profiles: | dcor( [a,b], ab ) |
| Similarity of single profiles: | dcor( a,b ) |
| Equality of contribution: | min( dcor( a,ab ), dcor( b,ab )) / max( dcor( a,ab ), dcor( b,ab )) |

Each feature and its interpretation are described in detail here (Norman, T. M. et al. Science. 365, 786-793 (2019)). Features were scaled (margin = 2) prior to hierarchal clustering (dist = euclidean, methods = ward) to generate a dendrogram.

Example 2: Efficient combinatorial targeting of RNA transcripts in single cells with Cas13 RNA Perturb-seq

Type VI CRISPR Cas proteins, such as the VI-D family member *Rft*Cas13d, are programmable RNAguided and RNA-targeting nucleases that enable targeted RNA knockdown. Notably, *Rft*Cas13d is also capable of processing a CRISPR array into multiple mature CRISPR RNAs (crRNAs) (Konermann, S. et al. Cell 173, 665-676 (2018))., presenting an attractive option for combinatorial perturbations at the RNA level. Recently, we confirmed that RfxCas13d can lead to striking target-RNA knockdown, and learned a set of optimal targeting rules from thousands of gRNAs tiling different transcripts (Wessels, H. H. et al. Nat. Biotechnol. 38, 722-727 (2020)). We therefore sought to combine pooled CRISPR-Cas13 screens with single-cell readouts to perform combinatorial and multimodal pooled genetic screens.

Our method for Cas13 RNA Perturb-Seq (CaRPool-seq) is enabled via an optimized molecular strategy to deliver individual or multiple gRNA perturbations in each cell and detect their identity during a single-cell sequencing experiment. Type VI A, C, and D Cas13 crRNAs consist of a short 5' direct repeat (DR) and a variable spacer (also called guide RNA; gRNA) at the 3'end, and therefore lack a common priming site for reverse transcription. We developed an approach for direct gRNA detection by adding a 10x Genomics compatible 'capture' sequence on the 3' end of the 23nt target spacer (FIG. 1A). We also explored an 'indirect' capture strategy, where a dedicated crRNA of the CRISPR array contains an array specific barcode (barcode gRNA; bcgRNA), and tested different positional configurations of the bcgRNA and gRNA (FIG. 1A). We evaluated the performance of each method by targeting cell surface proteins and measuring knockdown via flow cytometry (FIG. 1B and FIG. 4A - FIG. 4G), and by quantifying crRNA detection via RT-PCR (FIG. 1C). While all methods successfully induced robust knockdown (FIG. 1B), we found that indirect guide capture with an optimized configuration resulted in the strongest crRNA transcript detection ability (Configuration X; FIG. 1C).

These results demonstrate that *Rfx*Cas13d crRNAs can be modified by adding a common RT handle either directly to the gRNA or as a separate bcgRNA as part of a CRISPR array, allowing for reverse transcription and amplification. Notably, our strategy for indirect detection is well-suited for delivering multiple gRNAs into a single cell alongside a detectable bcgRNA that encodes the collective identity of these perturbations. In addition, utilizing a unique set of reverse transcription handle and Illumina PCR priming sequence in our modified crRNA (FIG. 5) ensures that these perturbations can be detected not only alongside scRNA-seq, but also when profiling additional molecular modalities (e.g. CITE-seq for simultaneous transcriptome and surface protein profiling). Finally, we found that introducing a stably structured RNA element ('evopreQ1 pseudoknot') at the bcgRNA 3'end further improved quantitative recovery of bcgRNA 6-fold, likely by antagonizing the nucleolytic decay of the bcgRNA (see Example 3).

As proof of principle, we first tested the ability of CaRPool-seq to detect and assign bcgRNAs in a single-cell species mixing experiment. We separately transduced *Rfx*Cas13d-expressing human HEK293FT and mouse NIH/3T3 cells with a viral pool of three CRISPR arrays containing a non-targeting (NT) gRNA and a species-specific bcgRNA. We profiled a mixture of human and mouse cells with the 10x Genomics Chromium system (v3), aiming to detect both cellular transcriptomes and the bcgRNAs. Of 2387 cells, we found that 78.5% expressed a single bcgRNA (1.1% >1 bcgRNAs; 20.4% no detected bcgRNA). Moreover, we observed extremely high concordance between RNA and bcgRNA labels in singlet cells (99.2%) (FIG. 1D and FIG. 1E). These numbers demonstrate that CaRPool-seq enables pooled perturbation screens that can be efficiently and accurately demultiplexed into a single-cell readout.

Next, we tested the ability of CaRPool-seq to distinguish combinatorial perturbations on multiple molecular modalities at single-cell resolution. We designed gRNAs targeting three cell surface proteins, CD46, CD55, and CD71, as well as NT gRNAs. We created 29 crRNA arrays, each of which contains up to three gRNAs and a bcgRNA, allowing for the perturbation of these genes individually or in combination. We transduced HEK293FT cells with a viral pool of all crRNAs and performed CaRPool-seq with CITE-seq readout (FIG. 2A and FIG. 6A), allowing the assessment of each perturbation on both the cellular transcriptome and antibody-derived tags (ADTs) associated with CD46, CD55, and CD71 surface protein levels.

We obtained 9,355 single-cell profiles and demultiplexed them into groups based on the detected bcgRNA (FIG. 6B; 74.7% expressed a single bcgRNA, 80.8% expressed at least one bcgRNA). We observed, on average, a 76.5% (+/-5.7%) mean reduction in protein levels for each targeted gene after perturbation with Cas13 demonstrating clear evidence of robust molecular perturbation (FIG. 2B - FIG. 2D). Moreover, the strength of knockdown was similar for multi-gRNA crRNA arrays relative to single gRNA perturbations (FIG. 2B and FIG. 7C). When examining transcriptomic pseudobulk profiles for all 26 targeting gRNA groups, we observed decreased mRNA expression for each targeted transcript, even when perturbing transcripts of three genes simultaneously (15 examples in FIG. 6C and FIG. 6D). The average strength of transcriptomic knockdown (mean 15%, sd 5.2%) was consistently reduced compared to the observed protein reduction. This is concordant with the mode of knockdown exhibited by Cas13. Target RNAs are continuously being produced and degraded before the target can be translated into protein. Further, analogous to how Cas9-nuclease targeting often produces RNAs degraded by nonsense mediated decay (Tuladhar, R. et al. Nat. Commun. 10, 1-10 (2019)), it is possible that Cas13 cleavage produces RNA molecules that can be detected by scRNA-seq but cannot be translated into functional protein, suggesting that the level of measured RNA knockdown underestimates the phenotypic effect of Cas13 perturbation. Importantly, we found that Cas13d-mediated gene knockdown was highly specific, with no evidence of off-target effects in any of the three target gene perturbations (FIG. 6E and FIG. 6F).

We next benchmarked the performance of CaRPool-seq against direct capture Perturb-Seq (Replogle, J. M. et al. Nat. Biotechnol. 38, 954-961 (2020)) using three different Cas9 effectors: Cas9-nuclease, a first-generation CRISPR inhibition (CRISPRi) system, KRABdCas9 (Gilbert, L. A. et al. Cell 154, 442 (2013); Morris, J. A. et al. bioRxiv 2021.04.07.438882 (2021)) and a second-generation, dual-effector CRISPRi system, KRAB-dCas9-MeCP2 (Morris, J. A. et al. bioRxiv 2021.04.07.438882 (2021); Yeo, N. C. et al. Nat. Methods 15, 611-616 (2018)). In CaRPool-seq one bcgRNA encodes the combined gRNA identities, while direct capture Perturb-Seq requires independent detection of one sgRNA feature per perturbation (FIG. 3A). We replicated our previously described experimental system, targeting the same three cell surface markers (CD46, CD55, and CD71) alone or in combination. For each target, we evaluated three sgRNAs from established CRISPR-KO (Doench, J. G. et al. Nat. Biotechnol. 34, 184-191 (2016)) and CRISPRi (Sanson, K. R. et al. Nat. Commun. 9, 1-15 (2018)). sgRNA libraries (FIG. 8A) and selected the best sgRNA for Perturb-Seq (FIG. 8B). In addition, we utilized Cell Hashing (Stoeckius, M. et al. Genome Biol. 19, 1-12 (2018)) to label cells targeted with vectors encoding single, double, or triple perturbations. As in CaRPool-seq, we quantified gRNA, RNA, and ADT levels in each cell.

Our benchmarking analysis found that, in contrast to CaRPool-seq, alternative Cas9-based approaches struggled to efficiently identify and detect combinatorial perturbations (FIG. 3B). For example, in the KRAB-dCas9-MeCP2 experiment, we recovered 1,570 cells that received vectors targeting three genes. Among these cells, only 779 (49.6%) were associated with the correct three sgRNA after sequencing. In the remaining cells, we detected too few perturbations (0, 1, or 2 gRNAs, 31.2%), too many (4+ gRNAs 10.0%), or an improper combination of 3 gRNA (9.2%). This observed drop-off is fully consistent with the theoretical expectation of recovery for multiple independently detected gRNAs and highlights the challenge of efficiently profiling multiple perturbations with existing approaches. Since CaRPool-seq associates combinatorial perturbations with a single bcgRNA, the efficiency of detection does not vary between single and multiple perturbations.

We next compared the strength of perturbation across methods. We first considered cells where three perturbations were successfully detected based on either the bcgRNA (CaRPool-seq) or independently detected gRNA (Perturb-Seq). When considering these cells, all methods successfully induced a similarly strong depletion of all three surface proteins (Cas13d: 74.5%, Cas9; 75.5%, KRAB-dCas9; 75.2%, KRABdCas9-MeCP2; 77.3%) (FIG. 3C We next analyzed all cells based on their ADT levels. CaRPool-seq and Perturb-Seq cells clustered together (FIG. 3D), and grouped by gRNA identity (FIG. 3E and FIG. 8C), again demonstrating that the strength of phenotypic protein perturbation was similar across all methods. We conclude that CaRPool-seq and Perturb-Seq can both effectively introduce combinatorial perturbations into single cells. However, CaRPool-seq exhibits clear advantages in the ability to successfully identify and detect these perturbations and therefore represents an attractive approach for performing combinatorial single-cell CRISPR screens.

To demonstrate the throughput and potential of CaRPool-seq to characterize genetic interactions, we performed a multiplexed screen of 158 combinatorial gene pairs. We aimed to characterize potential interactions between previously identified regulators of leukemic differentiation, which can influence the response to chemotherapy and small-molecule drugs (See Wessels, H-H et al. Efficient combinatorial targeting of RNA transcripts in single cells with Cas13 RNA Perturb-seq, bioRxiv 2022.02.02.478894).

We generated a human MLL-AF9 NRASG12D AML cell line (THP-1 cells), with a stably integrated doxycycline-inducible Cas13d cassette, as a model system. We first performed a bulk Cas13d CRISPR screen using a targeted library of 439 genes with 10 gRNA per gene. On day 13-16 post Cas13d-induction, cells were sorted into bins based on their surface expression of CD14 and CD11b, immunophenotypic markers of monocyte differentiation. By comparing gRNA representation between low and high-expressing bins, we selected 26 genes (and for each, the two best performing, non-overlapping gRNAs) that influenced differentiation. Through individual perturbations with a flow-cytometry readout, we validated that each of these genes regulated CD11b expression. These genes were largely associated with DNA-binding and chromatin remodeling functions, and include a subset of previously identified regulators of AML differentiation.

We next applied CaRPool-seq to test the effects of combinatorially perturbing these regulators. We infected cells with a pooled library of 385 crRNA arrays. This library encoded 28 single perturbations (26 regulators and two negative control genes) and 158 paired perturbations. It also encompassed technical replicates for each perturbation using independent gRNAs, as well as NT controls. We profiled the transcriptome, cell surface protein levels, and gRNA expression for 31,308 demultiplexed single cells.

We first compared the level of surface protein expression for each perturbation to NT controls. As expected, we found that each single-gene perturbation affected CD11b expression, with observed $\log_2$-fold changes that were in strong agreement (R=0.86) with the level of gRNA enrichment from bulk CRISPR screens. Observed $\log_2$-fold changes for all perturbations were also reproducible (R=0.82) across technical replicate perturbations. We next compared the observed effect of the 158 dual gene perturbations to the effects resulting from the two single perturbations. We observed a strong correlation and found that the dual perturbation was typically stronger than the average of individual knockdowns, but weaker than the product. We also observed both synergistic and dampening effects. For example, individual knockdown of the histone demethylase KDM1A ($log_2$FC 2.41) and the histone deacetylase HDAC3 ($log_2$FC 0.53) lead to strong and weak CD11b-upregulation, but dual perturbation leads to a synergistic effect ($log_2$FC 2.85). In contrast, while individual knockdown of EP300 also leads to CD11b upregulation ($log_2$FC 1.57), dual perturbation with KDM1A ($log_2$FC 2.05) was weaker than the individual KDM1A knockdown. We validated these findings using data from our Cas13d CD11b pooled screen.

We next explored the transcriptional profiles of both single and double perturbations in our CaRPool-seq dataset. As expected, up-regulated gene modules in singly perturbed cells were typically associated with genetic programs associated with the differentiation and function of myeloid cells. We applied a recent pioneering framework (Norman, T. M. et al. Science. 365, 786-793 (2019)) that fits a regression model to decompose the observed perturbation responses in doubly perturbed cells as a linear combination of single gene perturbation responses. The fit and coefficients of this model describe multiple types of genetic interactions, including epistasis, genetic suppression, and synergistic relationships. Fitting these models to each of our pairwise perturbations revealed a diversity of genetic interactions, which we broadly clustered into 4 groups. For 33 gene pairs in Cluster 1, we saw that each individual gene's profile contributed equally to the dual perturbation response, and the linear model exhibited a strong fit. As a positive, control, many of the pairs in this cluster represented perturbations of two proteins in the same complex (i.e. MED14/MED24; SUPT16H/SUPT6H). This cluster also represented pairs of proteins residing in separate complexes (MED24/SMARCD1 of mediator and SWI/SNF complexes) which share similar perturbation signatures. Dual perturbation of KDM1A and the transcriptional repressor GSE1 also fell in this cluster, consistent with previous work that suggests a cooperative interaction via colocalization at repressed promoters to inhibit myeloid differentiation.

In cluster 4, we identified genetic interactions where one gene's effect appeared to dominate over the other. We generally observed that transcriptional responses varied widely when pairing KDM1A knockdown with different chromatin regulators. For example, we found that the EP300-signature appeared more strongly than the KDM1A-signature when combinatorial perturbing both genes. Dually perturbed cells exhibited higher expression of progenitor genes (i.e. the progenitor marker AZU1), and reduced expression of differentiated marker genes (myeloid marker S100A4) compared to individual KDM1A perturbation. Contrastingly, the KDM1A response signature dominated when paired with perturbation of the polycomb repressive complex member RING1. Dual perturbation of HDAC3 enhanced the KDM1A transcriptional response signature, consistent with our previously described immunophenotypic results for these cells. These findings also support and provide a molecular explanation for recent observations that combination therapies of KDM1A antagonist and HDAC inhibitors exhibit an enhanced response. The heterogeneity across interaction responses was not unique to KDM1A, but describes many genetic regulators in our study, and highlights CaRPool-seq's ability to robustly characterize complex genetic interactions at scale.

In summary, we have developed CaRPool-seq, a flexible method for performing CRISPR Cas13 RNA-targeting screens with a single-cell sequencing-based readout. We introduced an optimized strategy to deliver multiple gRNA as part of a single CRISPR array, which is subsequently cleaved into individual crRNAs. We demonstrate that this strategy is well-suited for performing combinatorial perturbations, whose identity is encoded in a single barcode that can be reliably detected alongside multiple molecule modalities including scRNA-seq and CITE-seq.

Through benchmarking, we show that CaRPool-seq is more efficient and accurate when assigning multiple perturbations in single cells when compared to Cas9-based technologies. Even with individual perturbations, user will still benefit from CaRPool-seq. In particular, as an RNA-targeting enzyme, Cas13d can be uniquely applied to target specific RNA isoforms, or even circular, enhancer, or antisense RNA molecules. RNA-directed approaches may also be optimal when targeting a single member of a local gene cluster, where alternative KRAB-mediated repressive strategies may 'spread', and introduce off-target effects. CaRPool-seq can profile additional cellular modalities such as cell surface protein levels and, in the future, can be extended to additional molecular modalities including intracellular protein levels and chromatin accessibility.

Combinatorial screens have the potential to shed substantial new light on the structure of genetic regulatory networks, but also to identify combinatorial perturbations that achieve desirable cellular phenotypes. Our CaRPool-seq analysis of AML differentiation regulators benefited from recently developed computational frameworks to identify genetic interactions from multiplexed perturbation screens, and these types of data will be valuable resources for systematic reconstruction of complex pathways and cell circuits. Moreover, our identification of combinatorial perturbations that enhanced AML differentiation phenotypes was consistent with previous identification of efficacious multi-drug therapies, suggesting that future experiments may help to nominate candidates for combined drug treatments. CaRPoolseq represents a powerful addition to the growing toolbox of methods for multiplexed single-cell perturbations.

Example 3: The addition of a stable structured RNA element at the 3' end of the bcgRNA

It was recently shown that exogenously administered crRNAs are protected against rapid cellular RNA decay mechanisms in the presence of Cas13d (Mendez-Mancilla, A. et al. Cell Chem. Biol. 1-7 (2021) doi:10.1016/j.chembiol.2021.07.011), likely because crRNAs are rendered inaccessible to RNAse degradation when embedded in a Cas13d RNP complex. We recognized that a bcgRNA is longer than a standard targeting gRNA. Consequently, the reverse transcription handle likely protrudes outside the Cas13d RNP complex (Zhang, C. et al. Cell. 175, 212-223 (2018)) and thus might be subject to 3' nucleolytic decay by endogenous RNA nucleases. We, therefore, hypothesized that the addition of a stable structured RNA element at the bcgRNA 3'end may antagonize nucleolytic decay and improve bcgRNA detection sensitivity (FIG. 9A).

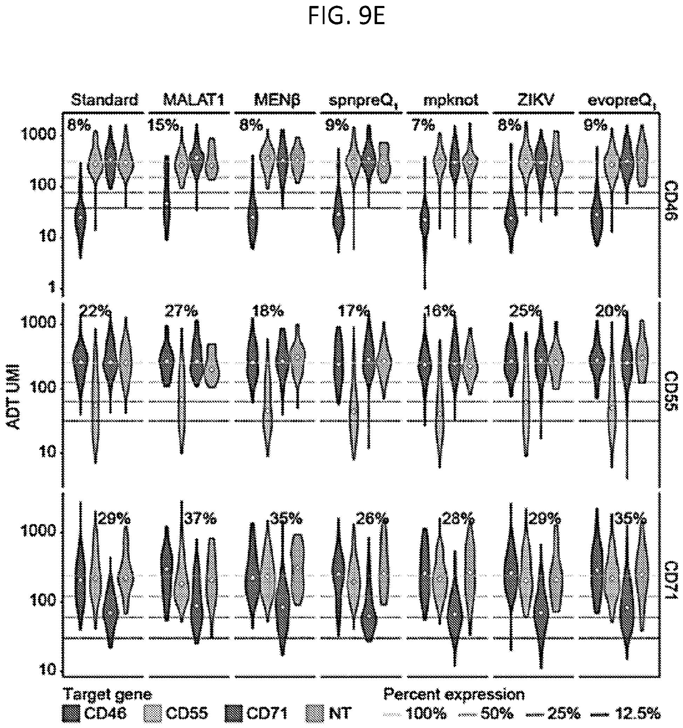

We performed CaRPool-seq with CITE-seq readout targeting the cell surface proteins CD46, CD55, and CD71, and assessed bcgRNA detection comparing the effect of six structured RNAs when placed 3' to the reverse transcription handle (FIG. 9B). We assessed single-cell similarity on protein ADT expression level and found the recovered cells clustering by target gene (FIG. 9C). The identity from the employed stabilizing RNA element did not influence cell clustering (FIG. 9D). Indeed, we found that target knockdown was similar between the stabilizing RNA elements (FIG. 9E). Only the usage of the MALAT1-triplex structure led to a slight reduction in target, likely due to improper folding as a consequence of required nucleotide substitutions. Two structures (ZIKA virus-derived xrRNA1 dumbbell; evopreQ1 pseudoknot) led to a robust increase in bcgRNA detection (FIG. 9F). The evopreQ1 pseudoknot element has been used before to improve prime editing efficiency by about 2-fold (Nelson, J. W. et al. Nat. Biotechnol. (2021) doi: 10.1038/s41587-021-01039-7). For CaRPool-seq, the evopreQ1 element led to a 6-fold higher bcgRNA detection sensitivity compared to our initial design (FIG. 9G). The increased sensitivity also systematically improved the signal-to-noise ratio to distinguish true bcgRNA UMI counts from spurious secondary bcgRNA UMI counts (FIG. 9H).

REFERENCES

1. Dixit, A. et al. Perturb-Seq: Dissecting Molecular Circuits with Scalable Single-Cell RNAProfiling of Pooled Genetic Screens. Cell 167, 1853-1866 (2016).

2. Datlinger, P. et al. Pooled CRISPR screening with single-cell transcriptome readout. Nat. Methods. 14, 297-301 (2017).

3. Jaitin, D. A. et al. Dissecting Immune Circuits by Linking CRISPR-Pooled Screens with Single-Cell RNA-Seq. Cell 167, 1883-1896 (2016).

4. Mimitou, E. P. et al. Multiplexed detection of proteins, transcriptomes, clonotypes and CRISPR perturbations in single cells. Nat. Methods 16, 409-412 (2019).

5. Adamson, B. et al. A Multiplexed Single-Cell CRISPR Screening Platform Enables SystematicDissection of the Unfolded Protein Response. Cell 167, 1867-1882 (2016).

6. Replogle, J. M. et al. Combinatorial single-cell CRISPR screens by direct guide RNA capture andtargeted sequencing. Nat. Biotechnol. 38, 954-961 (2020).

7. Norman, T. M. et al. Exploring genetic interaction manifolds constructed from rich single-cell phenotypes. Science (80-. ). 365, 786-793 (2019).

8. Michlits, G. et al. Multilayered VBC score predicts sgRNAs that efficiently generate loss-of-function alleles. Nature Methods 17, (2020).

9. Papalexi, E. et al. Characterizing the molecular regulation of inhibitory immune checkpoints withmulti-modal single-cell screens. bioRxiv (2020).

10. Konermann, S. et al. Transcriptome Engineering with RNA-Targeting Type VI-D CRISPREffectors. Cell 173, 665-676 (2018).

11. Wessels, H. H. et al. Massively parallel Cas13 screens reveal principles for guide RNA design. Nat. Biotechnol. 38, 722-727 (2020).

12. Stoeckius, M. et al. Simultaneous epitope and transcriptome measurement in single cells. Nat.Methods 14, 865-868 (2017).

13. Anzalone, A. V., Lin, A. J., Zairis, S., Rabadan, R. & Cornish, V. W. Reprogramming eukaryotictranslation with ligand-responsive synthetic RNA switches. Nat. Methods 13, 453-458 (2016).

14. Nelson, J. W. et al. Engineered pegRNAs improve prime editing efficiency. Nat. Biotechnol. (2021). doi:10.1038/s41587-021-01039-7

15. Tuladhar, R. et al. CRISPR-Cas9-based mutagenesis frequently provokes on-target mRNAmisregulation. Nat. Commun. 10, 1-10 (2019).

16. Gilbert, L. A. et al. CRISPR-mediated modular RNA-guided regulation of transcription ineukaryotes. Cell 154, 442 (2013).

17. Morris, J. A. et al. Discovery of target genes and pathways of blood trait loci using pooledCRISPR screens and single cell RNA sequencing. bioRxiv 2021.04.07.438882 (2021).

18. Yeo, N. C. et al. An enhanced CRISPR repressor for targeted mammalian gene regulation. Nat.Methods 15, 611-616 (2018).

19. Doench, J. G. et al. Optimized sgRNA design to maximize activity and minimize off-target effectsof CRISPR-Cas9. Nat. Biotechnol. 34, 184-191 (2016).

20. Sanson, K. R. et al. Optimized libraries for CRISPR-Cas9 genetic screens with multiplemodalities. Nat. Commun. 9, 1-15 (2018).

21. Stoeckius, M. et al. Cell Hashing with barcoded antibodies enables multiplexing and doubletdetection for single cell genomics. Genome Biol. 19, 1-12 (2018).

22. Wang, E. et al. Surface antigen-guided CRISPR screens identify regulators of myeloid leukemiadifferentiation. Cell Stem Cell 28, 718-731 (2021).

23. Nicosia, L. et al. Pharmacological inhibition of LSD1 triggers myeloid differentiation by targetingGSE1 oncogenic functions in AML. Oncogene (2021). doi:10.1038/s41388-021-02123-7

24. Fiskus, W. et al. Highly effective combination of LSD1 (KDM1A) antagonist and pan-histonedeacetylase inhibitor against human AML cells. Leukemia 28, 2155-2164 (2014).

25. Lensch, S. et al. Dynamic spreading of chromatin-mediated gene silencing and reactivation between neighboring genes in single cells. J. Apl. Teknol. Pangan (2021). doi:10.1101/2021.11.04.467237

26. Sanjana, N. E., Shalem, O. & Zhang, F. Improved vectors and genome-wide libraries for CRISPRscreening. Nat. Methods 11, 783-784 (2014).

27. Guo, X. et al. Transcriptome-wide Cas13 guide RNA design for model organisms and viral RNApathogens. Cell Genomics 1, 100001 (2021).

28. Macosko, E. Z. et al. Highly parallel genome-wide expression profiling of individual cells usingnanoliter droplets. Cell 161, 1202-1214 (2015).

29. Love, M. I., Huber, W. & Anders, S. Moderated estimation of fold change and dispersion forRNA-seq data with DESeq2. Genome Biol. 15, 550 (2014).

30. Langmead, B., Trapnell, C., Pop, M. & Salzberg, S. L. Ultrafast and memory-efficient alignmentof short DNA sequences to the human genome. Genome Biol. 10, (2009).

31. Leek, J. T., Johnson, W. E., Parker, H. S., Jaffe, A. E. & Storey, J. D. The sva package forremoving batch effects and other unwanted variation in high-throughput experiments. Bioinformatics 28, 882-883 (2012).

32. Kolde, R., Laur, S., Adler, P. & Vilo, J. Robust rank aggregation for gene list integration andmeta-analysis. Bioinformatics 28, 573-580 (2012).

33. Hao, Y. et al. Integrated analysis of multi modal single-cell data. Cell 184, 3573-3587.e29 (2021).

**Claims**

1. A nucleic acid comprising a CRISPR array comprising one or more crRNA sequences, each crRNA comprising a direct repeat sequence and a gRNA sequence, and a 5' direct repeat sequence linked to a barcode guide RNA (bcgRNA), the bcgRNA comprising from 5' to 3'

    (a) optionally, a PCR handle;
    (b) a barcode sequence; and
    (c) a reverse-transcription handle.

2. The nucleic acid according to claim 1, wherein the CRISPR array comprises one, two, three, or more crRNA sequences, optionally wherein each of the one or more crRNA sequences comprises a gRNA that comprises an at least 20 nucleotide sequence that is complementary to a target RNA sequence.

3. The nucleic acid according to claim 1 or 2, wherein each of the crRNA present in the CRISPR array has a different gRNA sequence.

4. The nucleic acid according to claim 1 or 2, wherein two or more of the crRNA present in the CRISPR array comprise the same gRNA sequence.

5. The nucleic acid according to any one of claims 1 to 3, wherein each of the crRNA sequences present in the CRISPR array is specific for a different region of a target transcript.

**6.** The nucleic acid according to any one of claims 1 to 5, wherein the CRISPR array comprises crRNA sequences having guide RNA (gRNA) sequences that target multiple transcripts.

**7.** The nucleic acid according to any one of claims 1 to 6, wherein the bcgRNA is downstream (3') of the one or more crRNA sequences of the CRISPR array.

**8.** The nucleic acid according to any one of claims 1 to 6, wherein the bcgRNA is upstream (5') of the one or more crRNA sequences of the CRISPR array.

**9.** The nucleic acid according to any one of claims 1 to 8, wherein

(a) the direct repeat is capable of binding a CRISPR-Cas13 enzyme, optionally Cas13d;
(b) the reverse-transcription handle comprises a polyA sequence, a CS1, or a CS2;
(c) the barcode comprises 8 to 15 nucleotides; and/or
(d) the CRISPR array further comprises a stabilizing RNA element at its 3' end, optionally wherein the stabilizing RNA element is a MALAT1, NEAT1 (MENB), spnpreQ$_1$, ZIKV xrRNA1, mpknot, or evopreQ$_1$ element or the stabilizing RNA element comprises SEQ ID NO: 39, 40, 41, 42, 43, or 44.

**10.** An expression cassette comprising a sequence encoding the nucleic acid according to any one of claims 1 to 9.

**11.** A vector comprising the expression cassette according to claim 10, wherein the vector is a non-viral vector, optionally a plasmid, or a viral vector, optionally a retrovirus vector, a lentivirus vector, an adenovirus vector, or an adeno-associated virus vector.

**12.** The vector according to claim 11, wherein the plasmid comprises a sequence encoding a CRISPR-Cas enzyme, optionally a Cas13 enzyme, a Cas12 enzyme, or a type VI CRISPR-Cas enzyme.

**13.** A host cell comprising the nucleic acid according to any one of claims 1 to 9, or the expression cassette according to claim 10, and a CRISPR-Cas enzyme, optionally a Cas13 enzyme or a Cas12 enzyme.

**14.** A method of introducing one or more gene perturbations in a single cell transcriptome, the method comprising culturing the host cell according to claim 13.

**15.** A method of performing gene perturbation profiling, the method comprising:

(a) obtaining the host cell according to claim 13, optionally labeling the cell with a fluorophore-conjugated antibody, and sorting the cell using flow cytometry;
(b) isolating RNA from the cell;
(c) performing reverse-transcription comprising contacting the RNA with a primer specific for the reverse-transcription handle;
(d) identifying the barcode sequence, optionally by amplifying the barcode sequence using a primer specific for the PCR handle; and
(e) detecting expression of one or more transcripts or gene products, optionally by using one or more of flow cytometric analysis, cell-hashing, single-cell sequencing analysis, single cell RNA sequencing (scRNA-seq), Perturb-seq, CROP-seq, CRISP-seq, ECCITE-seq, cellular indexing of transcriptomes and epitopes (CITE-seq),

wherein the CRISPR-Cas enzyme introduces one or more perturbations in the cell transcriptome.

**Patentansprüche**

**1.** Nukleinsäure, umfassend ein CRISPR-Array, das eine oder mehrere crRNA-Sequenzen, wobei jede crRNA eine Direct-Repeat-Sequenz und eine gRNA-Sequenz umfasst, und eine 5'-Direct-Repeat-Sequenz, die mit einer Barcodeguide-RNA (bcgRNA) verknüpft ist, umfasst, wobei die bcgRNA von 5' nach 3' Folgendes umfasst:

(a) gegebenenfalls eine PCR-Handle;
(b) eine Barcodesequenz; und
(c) eine Reverse-Transkription-Handle.

2. Nukleinsäure nach Anspruch 1, wobei das CRISPR-Array eine, zwei, drei oder mehr crRNA-Sequenzen umfasst, gegebenenfalls wobei die eine oder die mehreren crRNA-Sequenzen jeweils eine gRNA umfassen, die eine Sequenz von mindestens 20 Nukleotiden umfasst, die komplementär zu einer Ziel-RNA-Sequenz ist.

3. Nukleinsäure nach Anspruch 1 oder 2, wobei die im CRISPR-Array vorhandenen crRNA jeweils eine unterschiedliche gRNA-Sequenz aufweisen.

4. Nukleinsäure nach Anspruch 1 oder 2, wobei zwei oder mehr der im CRISPR-Array vorhandenen crRNA die gleiche gRNA-Sequenz umfassen.

5. Nukleinsäure nach einem der Ansprüche 1 bis 3, wobei jede der im CRISPR-Array vorhandenen crRNA-Sequenzen für eine andere Region eines Zieltranskripts spezifisch ist.

6. Nukleinsäure nach einem der Ansprüche 1 bis 5, wobei das CRISPR-Array crRNA-Sequenzen mit Guide-RNA(gRNA)-Sequenzen umfasst, die auf mehrere Transkripte zielen.

7. Nukleinsäure nach einem der Ansprüche 1 bis 6, wobei sich die bcgRNA stromabwärts (3') von der einen oder den mehreren crRNA-Sequenzen des CRISPR-Array befindet.

8. Nukleinsäure nach einem der Ansprüche 1 bis 6, wobei sich die bcgRNA stromaufwärts (5') von der einen oder den mehreren crRNA-Sequenzen des CRISPR-Array befindet.

9. Nukleinsäure nach einem der Ansprüche 1 bis 8, wobei

(a) die Direct Repeat in der Lage ist, ein CRISPR-Cas13-Enzym, gegebenenfalls Cas13d, zu binden;
(b) die Reverse-Transkription-Handle eine PolyA-Sequenz, eine CS1 oder eine CS2 umfasst;
(c) der Barcode 8 bis 15 Nukleotide umfasst; und/oder
(d) das CRISPR-Array ferner ein stabilisierendes RNA-Element an seinem 3'-Ende umfasst, gegebenenfalls wobei es sich bei dem stabilisierenden RNA-Element um ein MALAT1-, NEAT1- (MENβ-), spnpreQ$_1$-, ZIKV-xrRNA1-, mpknot- oder evopreQ$_1$-Element handelt oder das stabilisierende RNA-Element SEQ ID NO: 39, 40, 41, 42, 43 oder 44 umfasst.

10. Expressionskassette, umfassend eine Sequenz, die die Nukleinsäure nach einem der Ansprüche 1 bis 9 codiert.

11. Vektor, umfassend die Expressionskassette nach Anspruch 10, wobei es sich bei dem Vektor um einen Nicht-Virus-Vektor, gegebenenfalls ein Plasmid, oder einen Virus-Vektor, gegebenenfalls einen Retrovirus-Vektor, einen Lentivirus-Vektor, einen Adenovirus-Vektor oder einen Adeno-assoziiertes-Virus-Vektor, handelt.

12. Vektor nach Anspruch 11, wobei das Plasmid eine Sequenz umfasst, die ein CRISPR-Cas-Enzym, gegebenenfalls ein Cas13-Enzym, ein Cas12-Enzym oder ein Typ-VI-CRISPR-Cas-Enzym, codiert.

13. Wirtszelle, umfassend die Nukleinsäure nach einem der Ansprüche 1 bis 9 oder die Expressionskassette nach Anspruch 10 und ein CRISPR-Cas-Enzym, gegebenenfalls ein Cas13-Enzym oder ein Cas12-Enzym.

14. Verfahren zur Einführung einer oder mehrerer Genperturbationen in ein Einzelzelltranskriptom, wobei das Verfahren das Kultivieren der Wirtszelle nach Anspruch 13 umfasst.

15. Verfahren zur Erstellung von Genperturbationsprofilen, wobei das Verfahren Folgendes umfasst:

(a) Erhalten der Wirtszelle nach Anspruch 13, gegebenenfalls Markieren der Zelle mit einem Fluorophorkonjugierten Antikörper und Sortieren der Zelle unter Verwendung von Durchflusszytometrie;
(b) Isolieren von RNA aus der Zelle;
(c) Durchführen von Reverse-Transkription, was Inkontaktbringen der RNA mit einem für die Reverse-Transkription-Handle spezifischen Primer umfasst;
(d) Identifizieren der Barcodesequenz, gegebenenfalls durch Amplifizieren der Barcodesequenz unter Verwendung eines für die PCR-Handle spezifischen Primers; und
(e) Nachweisen der Expression eines oder mehrerer Transkripte oder Genprodukte, gegebenenfalls unter Verwendung einer oder mehrerer von Durchflusszytometrie-Analyse, Zell-Hashing, Einzelzellsequenzierung-Analyse, Einzelzell-RNA-Sequenzierung (scRNA-seq), Perturb-seq, CROP-seq, CRISP-seq, ECCITE-seq, CITE(Cellular Indexing of Transcriptomes and Epitopes)-seq,

wobei das CRISPR-Cas-Enzym eine oder mehrere Perturbationen in das Zelltranskriptom einführt.

## Revendications

1. Acide nucléique comprenant un réseau CRISPR comprenant une ou plusieurs séquences d'ARNc, chaque ARNc comprenant une séquence répétée directe et une séquence d'ARNg, et une séquence répétée directe en 5' liée à un ARN guide de code à barres (ARNbcg), l'ARNbcg comprenant de 5' à 3'

    (a) éventuellement, une poignée PCR ;
    (b) une séquence de code à barres ; et
    (c) un poignée de transcription inverse.

2. Acide nucléique selon la revendication 1, dans lequel le réseau CRISPR comprend une, deux, trois ou plusieurs séquences d'ARNc, facultativement où chacune des une ou plusieurs séquences d'ARNc comprend un ARNg qui comprend une séquence d'au moins 20 nucléotides qui est complémentaire d'une séquence d'ARN cible.

3. Acide nucléique selon la revendication 1 ou 2, dans lequel chacun des ARNc présents dans le réseau CRISPR a une séquence d'ARNg différente.

4. Acide nucléique selon la revendication 1 ou 2, dans lequel deux ou plus des ARNc présents dans le réseau CRISPR comprennent la même séquence d'ARNg.

5. Acide nucléique selon l'une quelconque des revendications 1 à 3, dans lequel chacune des séquences d'ARNc présentes dans le réseau CRISPR est spécifique d'une région différente d'un transcrit cible.

6. Acide nucléique selon l'une quelconque des revendications 1 à 5, dans lequel le réseau CRISPR comprend des séquences d'ARNc ayant des séquences d'ARN guide (ARNg) qui ciblent des transcrits multiples.

7. Acide nucléique selon l'une quelconque des revendications 1 à 6, dans lequel l'ARNbcg est en aval (3') de la ou des séquences d'ARNc du réseau CRISPR.

8. Acide nucléique selon l'une quelconque des revendications 1 à 6, dans lequel l'ARNbcg est en amont (5') de la ou des séquences d'ARNc du réseau CRISPR.

9. Acide nucléique selon l'une quelconque des revendications 1 à 8, dans lequel

    (a) la répétition directe est capable de se lier à une enzyme CRISPR-Cas13, éventuellement Cas13d ;
    (b) la poignée de transcription inverse comprend une séquence polyA, un CS1, ou un CS2 ;
    (c) le code à barres comprend 8 à 15 nucléotides ; et/ou
    (d) le réseau CRISPR comprend en outre un élément ARN stabilisant à son extrémité 3', facultativement où l'élément ARN stabilisant est un élément MALAT1, NEAT1 (MENβ), $spnpreQ_1$, ZIKV xrRNA1, mpknot, ou $evopreQ_1$ ou l'élément ARN stabilisant comprend SEQ ID NO: 39, 40, 41, 42, 43 ou 44.

10. Cassette d'expression comprenant une séquence codant pour l'acide nucléique selon l'une quelconque des revendications 1 à 9.

11. Vecteur comprenant la cassette d'expression selon la revendication 10, dans lequel le vecteur est un vecteur non viral, facultativement un plasmide, ou un vecteur viral, facultativement un vecteur rétroviral, un vecteur lentiviral, un vecteur adénoviral, ou un vecteur viral adéno-associé.

12. Vecteur selon la revendication 11, dans lequel le plasmide comprend une séquence codant pour une enzyme CRISPR-Cas, facultativement une enzyme Cas13, une enzyme Cas12, ou une enzyme CRISPR-Cas de type VI.

13. Cellule hôte comprenant l'acide nucléique selon l'une quelconque des revendications 1 à 9, ou la cassette d'expression selon la revendication 10, et une enzyme CRISPR-Cas, facultativement une enzyme Cas13 ou une enzyme Cas12.

14. Procédé d'introduction d'une ou plusieurs perturbations géniques dans un transcriptome cellulaire unique, le procédé comprenant la culture de la cellule hôte selon la revendication 13.

15. Procédé de réalisation d'un profilage de perturbation génique, le procédé comprenant :

(a) l'obtention de la cellule hôte selon la revendication 13, facultativement le marquage de la cellule par un anticorps conjugué à un fluorophore, et le tri de la cellule en utilisant une cytométrie en flux ;
(b) l'isolement d'ARN à partir de la cellule ;
(c) la mise en œuvre d'une transcription inverse comprenant la mise en contact de l'ARN avec une amorce spécifique pour la poignée de transcription inverse ;
(d) l'identification de la séquence de code à barres, éventuellement par amplification de la séquence de code à barres en utilisant une amorce spécifique pour la poignée PCR ; et
(e) la détection de l'expression d'un ou plusieurs produits de transcription ou produits géniques, facultativement par l'utilisation d'un ou plusieurs parmi une analyse par cytométrie en flux, un hachage de cellules, une analyse par séquençage monocellulaire, un séquençage d'ARN monocellulaire (scRNA-seq), Perturb-seq, CROP-seq, CRISP-seq, ECCITE-seq, indexation cellulaire de transcriptomes et d'épitopes (CITE-seq), où l'enzyme CRISPR-Cas introduit une ou plusieurs perturbations dans le transcriptome cellulaire.

FIG. 1A

FIG. 1B

FIG. 1C

crRNA

GAPDH

FIG. 1D

Transcriptome assignment
○ Multiplet (26; 1.1%)
● Human (1374; 57.6%)
● Mouse (987; 41.3%)

FIG. 1E

bcgRNA assignment:
Multiplet (26; 1.4%)
Human (1128; 62.2%)
Mouse (692; 36.4%)
+ 487 Guide-negative

FIG. 2A

Combinatorial targeting

Dose dependencies

Positional dependencies

gRNA1  gRNA2  gRNA3  bcgRNA

bcgRNA

SEQ ID NO: 21
CGCAGAGNNNNNNNNNGCUUUAAGGCCGGUCCUAGCAA
CGAAATTCCGGCCAGGATCGTT
SEQ ID NO: 22

Feature Barcoding

CITE-Seq and Cell hashing

SEQ ID NO: 23
BAAAAAAAAAAAA
VTTTTTTTTTTTT
SEQ ID NO: 24

oligo(dT)-capture

polyA RNA

SEQ ID NO: 23
BAAAAAAAAAAAA
VTTTTTTTTTTTT
SEQ ID NO: 24

oligo(dT)-capture

## FIG. 2B

## FIG. 2C

## FIG. 2D

FIG. 3A

FIG. 3B

## FIG. 3C

# FIG. 3D

Cas13d ●
Cas9-nuclease ◦
KRAB-dCas9 ✳
KRAB-dCas9-MeCP2 ●

UMAP 2

UMAP 1

# FIG. 3E

CD46/CD55/CD71

CD46/CD71

CD71

CD46

CD55/CD71

CD46/CD55

NT

CD55

UMAP 2

UMAP 1

FIG. 4A

FIG. 4B

FIG. 4C

FIG. 4D

FIG. 4E

FIG. 4F

FIG. 4G

**FIG. 5**

Cas13d CRISPR array

processing

mature crRNA

smallRNA PCR handle    BC Feature    Capture Sequence 1

CAAGUAAGCAGUGGTATCAACGCAGAGNNNNNNNNNNNCGGGAAAGGCCCGGUCCUUGCAA   SEQ ID NO: 1
CCC ←————————————————————————————— CGAAATTCCCGGCCAGGGATCGTT   SEQ ID NO: 2
template switch                                    RT primer

Reverse Transcription Annealing

SEQ ID NO: 3    5'-GTCAGATGTGTATAAGAGACAG-16N-1N-TTGCTAGGACCGGCCTTAAAGC-3'
                AACGATCCTGGCCGGAATTCG-FeatureBarcode-ACCTTAAGAGCCCACGGTTCC-CAAAGUUUGGGGCUGGUCAACCATCCCCAA-5'

SEQ ID NO: 4    Capture Seq    Feature    smallRNA    DR

RT Product (168mi)    [3'-GrGrGrTACATCAGAGCCAACTATGGTGACGAA-5']    SEQ ID NO: 5

SEQ ID NO: 6    5'-GTCAGATGTGTATAAGAGACAG-16N-1N-TTGCTAGGACCGGCCTTAAAGC-FeatureBarcode-TGGAATTCTCGGGTGCCAAGG-GTTTCAAACCCCGACCAGTTGGTAGGGGTT-CCC-3'
                AACGATCCTGGCCGGAATTCG-FeatureBarcode-ACCTTAAGAGCCCACGGTTCC-CAAAGUUUGGGGCUGGUCAACCATCCCCAA-5'

cDNA amplification                                      SEQ ID NO: 7              SEQ ID NO: 8
cDNA amplification TSO product (173bp)    [5'-GCAGCGTCAGATGTGTATAAGAGACAG-3']    [3'-GAGAGGCAACTATCGTGACGAA-5']

SEQ ID NO: 9    5'-GCAGCGTCAGATGTGTATAAGAGACAG-16N-12N-TTGCTAGGACCGGCCTTAAAGC-FeatureBarcode-TGGAATTCTCGGGTGCCAAGG-GTTTCAAACCCCGACCAGTTGGTAGGGGTT-CCCATGTACTCTGCGTTGATACCACTGCTT-3'
SEQ ID NO: 10   3'-CGTCGCAGTCTACACATATTCTCTGTC-16N-12N-AACGATCCTGGCCGGAATTCG-FeatureBarcode-ACCTTAAGAGCCCACGGTTCC-CAAAGUUUGGGGCUGGUCAACCATCCCCAA-GGGTACATGAGACGCAACTATGGTGACGAA-5'

cDNA amplification additive primer products (113bp)    SEQ ID NO: 11                   SEQ ID NO: 12
                                                       [5'-GCAGCGTCAGATGTGTATAAGAGACAG-3']    [3'-CTCTTAAGAGCCCACGGTTCC-5']

5'-GCAGCGTCAGATGTGTATAAGAGACAG-16N-12N-TTGCTAGGACCGGCCTTAAAGC-FeatureBarcode-TGGAATTCTCGGGTGCCAAGG-3'    SEQ ID NO: 13
3'-CGTCGCAGTCTACACATATTCTCTGTC-16N-12N-AACGATCCTGGCCGGAATTCG-FeatureBarcode-ACCTTAAGAGCCCACGGTTCC-5'    SEQ ID NO: 14

Feature Si Primers 2
PN-2000C.G0
5'-AATGATACGGCGACCACCGAGATCT-ACACTCGTCGGCAGCGTCAGATGTGTATAAGAGACAG-3'
SEQ ID NO: 16    5'-GCAGCGTCAGATGTGTATAAGAGACAG-16N-12N-TTGCTAGGACCGGCCTTAAAGC-FeatureBarcode-TGGAATTCTCGGGTGCCAAGG-3'
SEQ ID NO: 17    3'-CGTCGCAGTCTACACATATTCTCTGTC-16N-12N-AACGATCCTGGCCGGAATTCG-FeatureBarcode-ACCTTAAGAGCCCACGGTTCC-5'
Library amplification and sample indexing    SEQ ID NO: 15
SEQ ID NO: 18    3'-ACCTTAAGAGCCCACGGTTCCTTGAGGTCAGTGNNNNNNNNTAGAGCATACGGCAGAAGACGAAC-5'
                                                                     RPI-x i7 primer

Final library amplicon (200bp)                            15 nt

P5    Nextera R1    BC    UMI    Capture Seq    Feature    small RNA    RPI    I5x    P7

Read 9: Cellbarcode + UBI (28 cyc): ————→                    Read 17: Sample barcode (8 cyc) ————→
SEQ ID NO: 19   5'-AATGATACGGCGACCACCGAGATCT-ACACTCGTCGGCAGCGTCAGATGTGTATAAGAGACAG-16N-12N-TTGCTAGGACCGGCCTTAAAGC-FeatureBarcode-TGGAATTCTCGGGTGCCAAGGGAACTCCAGTCAC-16N-ATCTCGTATGCCGTCTTCTGCTTG-3'
SEQ ID NO: 20   3'-TTACTATGCCGCCGCTGGTCGCTCTAGA-TGTGAGCAGCCGTCGCAGTCTACACATATTCTCTGTC-16N-12N-AACGATCCTGGCCGGAATTCG-FeatureBarcode-ACCTTAAGAGCCCACGGTTCCTTGAGGTCAGTG-8N-TAGAGCATACGGCAGAAGACGAAC-5'
                                                          ←———————— Read 2: Feature (~15 cyc)

FIG. 6A

FIG. 6B

# FIG. 6C

# FIG. 6D

## FIG. 6E

| | | |
|---|---|---|
| SEQ ID NO: 25 | | AGACAATTGTGTCGCTGCCATCG |
| | | ││││││││││││││││││││││ |
| SEQ ID NO: 26 | CD46 | TCTGTTAACACAGCGACGGTAGC |
| SEQ ID NO: 27 | *SCAMP4* | TCTGTTAACACcGCGACGGcctg |
| SEQ ID NO: 28 | *LTN1* | TCTGTTAACcCAGtGACaGTAGt |
| | | |
| SEQ ID NO: 29 | | GATCACTGAGTCCTTCTCGCCAG |
| | | ││││││││││││││││││││││ |
| SEQ ID NO: 30 | CD55 | CTAGTGACTCAGGAAGAGCGGTC |
| SEQ ID NO: 31 | *C15orf39* | agccgGACTCaAGGAAGAGCGGTC |
| SEQ ID NO: 32 | *TRAPPC6B* | CTAGTGACTCAGGAaGAG-GGTt |
| SEQ ID NO: 33 | *STK26* | CTAGTGACaCAGGAAGA-CGGaa |
| SEQ ID NO: 34 | *NAGLU* | tggGTGACgCAGGAA-AGCGGTC |
| | | |
| SEQ ID NO: 35 | | CGATCACAGCAATAGTCCCATAG |
| | | ││││││││││││││││││││││ |
| SEQ ID NO: 36 | CD71/TFRC | GCTAGTGTCGTTATCAGGGTATC |
| SEQ ID NO: 37 | *SPATS2L* | cCTAGTGTgGTTATCAaGGTggt |
| SEQ ID NO: 38 | *RTN4* | GCTAGTGTCGaTATtAGGGTcgt |

## FIG. 6F

FIG. 7A

FIG. 7B

FIG. 7C

EP 4 301 868 B1

# FIG. 8A

49

# FIG. 8B

## FIG. 8C

FIG.9A

SEQ ID NO: 39    SEQ ID NO: 40    SEQ ID NO: 41    SEQ ID NO: 42    SEQ ID NO: 43    SEQ ID NO: 44

MALAT1 triplex variant    NEAT1/ MEN β variant    spnpreQ₁ variant    mpknot    ZIKV xrRNA1    evopreQ₁

FIG.9B

# FIG. 9E

# FIG. 9F

# FIG. 9G

EP 4 301 868 B1

FIG.9H

## REFERENCES CITED IN THE DESCRIPTION

### Patent documents cited in the description

- WO 2018005691 A1
- WO 2016182959 A1
- US 10362616 B
- WO 2019010384 A
- US 20190169595 A1
- US 20180119140 A
- US 5902880 A
- US 7195916 B2
- WO 2013182683 A
- WO 2010053572 A
- WO 2012170930 A
- WO 2016176191 A

### Non-patent literature cited in the description

- **DIXIT, A. ; PARNAS, O. ; LI, B. et al.** *Cell*, 2016, vol. 167 (7), 1853-1866
- **REPLOGLE et al.** *Nat. Biotechnol.*, 2020, vol. 38, 954-961
- **DOENCH, J. G. et al.** *Nat. Biotechnol.*, 2016, vol. 34, 184-191
- **SANSON, K. R. et al.** *Nat. Commun.*, 2018, vol. 9, 1-15
- **REPLOGLE, J. M. et al.** *Nat. Biotechnol.*, 2020, vol. 38, 954-961
- **MENDEZ-MANCILLA, A. et al.** *Cell Chem. Biol.*, 2021, 1-7
- **BROWN, J.A. et al.** *Proc. Natl. Acad. Sci. U. S. A.*, 2012, vol. 109, 19202-19207
- **KANG, M. et al.** *Proc. Natl. Acad. Sci. U. S. A.*, 2014, vol. 111
- **AKIYAMA, B. M. et al.** *Science.*, 2016, vol. 354, 1148-1152
- **ANZALONE, A.V. et al.** *Nat. Methods*, 2016, vol. 13, 453-458
- Overview of principles of hybridization and the strategy of nucleic acid probe assay. **TIJSSEN**. Laboratory Techniques In Biochemistry And Molecular Biology-Hybridization With Nucleic Acid Probes Part 1. Elsevier, 1993
- **GUO et al.** Transcriptome-wide Cas13 guide RNA design for model organisms and viral RNA pathogens. *bioRxiv 2020.08.20.259762*
- **WESSELS, HH. ; MÉNDEZ-MANCILLA, A. ; GUO, X. et al.** Massively parallel Cas13 screens reveal principles for guide RNA design. *Nat Biotechnol*, 2020, vol. 38, 722-727
- **CHENG et al.** Structural Basis for the RNA-Guided Ribonuclease Activity of CRISPR-Cas13d. *Cell*, 20 September 2018, vol. 175 (1), 212-223
- **KONERMANN, S. et al.** Transcriptome Engineering with RNA-Targeting Type VI-D CRISPR Effectors. *Cell*, 2018, vol. 173, 665-676
- **ABUDAYYEH et al.** C2c2 is a single-component programmable RNA-guided RNA-targeting CRISPR effector. *Science*, 2016, vol. 353, aaf5573
- **S. SHMAKOV et al.** Discovery and functional characterization of diverse class 2 CRISPR-Cas systems. *Mol. Cell*, 2015, vol. 60, 385-397
- **S. SHMAKOV et al.** Diversity and evolution of class 2 CRISPR-Cas systems. *Nat. Rev. Microbiol.*, 2017, vol. 15, 169-182
- **A. A. SMARGON et al.** Cas13b is a type VI-B CRISPR-associated RNA-guided RNase differentially regulated by accessory proteins Csx27 and Csx28. *Mol. Cell*, 2017, vol. 65, 618-630
- **J. S. GOOTENBERG et al.** Nucleic acid detection with CRISPR-Cas13a/C2c2. *Science*, 2017, vol. 356, 438-442
- **O. O. ABUDAYYEH et al.** RNA targeting with CRISPR-Cas13. *Nature*, 2017, vol. 550, 280-284
- **ZHANG C et al.** Structural Basis for the RNA-Guided Ribonuclease Activity of CRISPR-Cas13d. *Cell*, 2018, vol. 175, 212-223
- **NG ; HENIKOFF.** *Predicting the Effects of Amino Acid Substitutions on Protein Function*, 2006
- **NG ; HENIKOFF.** *Predicting the effects of coding nonsynonymous variants on protein function using the SIFT algorithm*, 2009
- **NG ; HENIKOFF.** *Accounting for Human Polymorphisms Predicted to Affect Protein Function*, 2002
- **DELLINGER et al.** *J. American Chemical Society*, 2011, vol. 133, 11540-11556
- **THRELFALL et al.** *Organic & Biomolecular Chemistry*, 2012, vol. 10, 746-754
- **DELLINGER et al.** *J. American Chemical Society*, 2003, vol. 125, 940-950
- **BROWN, J. A. et al.** *Proc. Natl. Acad. Sci. U. S. A.*, 2012, vol. 109, 19202-19207

- **ANZALONE, A. V. et al.** *Nat. Methods*, 2016, vol. 13, 453-458
- **KIYAMA, B. M. et al.** *Science*, 2016, vol. 354, 1148-1152
- **NELSON, J. W. et al.** *Nat. Biotechnol.*, 2021
- **GREEN ; SAMBROOK**. Molecular Cloning: A Laboratory Manual. Cold Spring Harbor Press, 2012
- **GOEDDEL**. Gene Expression Technology: Methods in Enzymology. Academic Press, 1990, vol. 185
- **RAMAMOORTH ; NARVEKAR**. *J Clin Diagn Res.*, January 2015, vol. 9 (1), GE01-GE06
- **X. SU et al.** *Mol. Pharmaceutics*, 21 March 2011, vol. 8 (3), 774-787
- **SAMBROOK et al.** Molecular Cloning: A Laboratory Manual. Cold Spring Harbor Press
- **HAQUE et al.** A practical guide to single-cell RNA-sequencing for biomedical research and clinical applications. *Genome Medicine*, 2017, vol. 9 (75)
- **HWANG et al.** Single-cell RNA sequencing technologies and bioinformatics pipelines. *Exp Mol Med.*, 07 August 2018, vol. 50 (8), 96
- **STOECKIUS et al.** Cell Hashing with barcoded antibodies enables multiplexing and doublet detection for single cell genomics. *Genome Biol*, 2018, vol. 19, 224
- **DIXIT et al.** Perturb-seq: Dissecting molecular circuits with scalable single cell RNA profiling of pooled genetic screens. *Cell*, 15 December 2016, vol. 167 (7), 1853-1866
- **DATLINGER et al.** Pooled CRISPR screening with single-cell transcriptome readout. *Nat Methods.*, March 2017, vol. 14 (3), 297-301
- **JAITIN et al.** Dissecting Immune Circuits by Linking CRISPR-Pooled Screens with Single-Cell RNA-Seq. *Cell*, 15 December 2016, vol. 167 (7), 1883-1896
- **MIMITOU et al.** Multiplexed detection of proteins, transcriptomes, clonotypes and CRISPR perturbations in single cells. *Nat Methods.*, May 2019, vol. 16 (5), 409-41
- **STOECKIUS et al.** Simultaneous epitope and transcriptome measurement in single cells. *Nat Methods.*, September 2017, vol. 14 (9), 865-868
- **SAMBROOK et al.** Molecular Cloning, A Laboratory Manual. 2001
- **KRIEGLER**. Gene Transfer and Expression: A Laboratory Manual. 1990
- Current Protocols in Molecular Biology. 2010
- **PALVA et al.** *Gene*, 1983, vol. 22, 229-235
- **WESSELS, H. H. et al.** *Nat. Biotechnol.*, 2020, vol. 38, 722-727
- **MORRIS, J. A. et al.** *bioRxiv 2021.04.07.438882*, 2021
- **SANJANA, N. E. et al.** *Nat. Methods*, 2014, vol. 11, 783-784
- **GUO, X. et al.** *Cell Genomics*, 2021, vol. 1, 100001
- **KONERMANN, S. et al.** *Cell*, 2018, vol. 173, 665-676
- **LOVE, M. I. et al.** *Genome Biol*, 2014, vol. 15, 550
- **LANGMEAD, B. et al.** *Genome Biol*, 2009, vol. 10
- **LEEK, J. T. et al.** *Bioinformatics*, 2012, vol. 28, 882-883
- **KOLDE, R. et al.** *Bioinformatics*, 2012, vol. 28, 573-580
- **STOECKIUS, M. et al.** *Nat. Methods*, 2017, vol. 14, 865-868
- **STOECKIUS, M. et al.** *Genome Biol.*, 2018, vol. 19, 1-12
- **HAO, Y. et al.** *Cell*, 2021, vol. 184, 3573-3587
- **NORMAN, T. M. et al.** *Science*, 2019, vol. 365, 786-793
- **TULADHAR, R. et al.** *Nat. Commun.*, 2019, vol. 10, 1-10
- **GILBERT, L. A. et al.** *Cell*, 2013, vol. 154, 442
- **YEO, N. C. et al.** *Nat. Methods*, 2018, vol. 15, 611-616
- **WESSELS, H-H et al.** Efficient combinatorial targeting of RNA transcripts in single cells with Cas13 RNA Perturb-seq. *bioRxiv 2022.02.02.478894*
- **ZHANG, C. et al.** *Cell*, 2018, vol. 175, 212-223
- **DIXIT, A. et al.** Perturb-Seq: Dissecting Molecular Circuits with Scalable Single-Cell RNAProfiling of Pooled Genetic Screens. *Cell*, 2016, vol. 167, 1853-1866
- **DATLINGER, P. et al.** Pooled CRISPR screening with single-cell transcriptome readout. *Nat. Methods.*, 2017, vol. 14, 297-301
- **JAITIN, D. A. et al.** Dissecting Immune Circuits by Linking CRISPR-Pooled Screens with Single-Cell RNA-Seq. *Cell*, 2016, vol. 167, 1883-1896
- **MIMITOU, E. P. et al.** Multiplexed detection of proteins, transcriptomes, clonotypes and CRISPR perturbations in single cells. *Nat. Methods*, 2019, vol. 16, 409-412
- **ADAMSON, B. et al.** A Multiplexed Single-Cell CRISPR Screening Platform Enables Systematic-Dissection of the Unfolded Protein Response. *Cell*, 2016, vol. 167, 1867-1882
- **REPLOGLE, J. M. et al.** Combinatorial single-cell CRISPR screens by direct guide RNA capture andtargeted sequencing. *Nat. Biotechnol.*, 2020, vol. 38, 954-961
- **NORMAN, T. M. et al.** Exploring genetic interaction manifolds constructed from rich single-cell phenotypes. *Science*, 2019, vol. 365, 786-793

- **MICHLITS, G. et al.** Multilayered VBC score predicts sgRNAs that efficiently generate loss-of-function alleles. *Nature Methods*, 2020, vol. 17
- **PAPALEXI, E. et al.** Characterizing the molecular regulation of inhibitory immune checkpoints withmulti-modal single- cell screens. *bioRxiv*, 2020
- **KONERMANN, S. et al.** Transcriptome Engineering with RNA-Targeting Type VI-D CRISPREffectors. *Cell*, 2018, vol. 173, 665-676
- **WESSELS, H. H. et al.** Massively parallel Cas13 screens reveal principles for guide RNA design. *Nat. Biotechnol.*, 2020, vol. 38, 722-727
- **STOECKIUS, M. et al.** Simultaneous epitope and transcriptome measurement in single cells. *Nat.Methods*, 2017, vol. 14, 865-868
- **ANZALONE, A. V.** ; **LIN, A. J.** ; **ZAIRIS, S.** ; **RABADAN, R.** ; **CORNISH, V. W**. Reprogramming eukaryotictranslation with ligand-responsive synthetic RNA switches. *Nat. Methods*, 2016, vol. 13, 453-458
- **NELSON, J. W. et al.** Engineered pegRNAs improve prime editing efficiency. *Nat. Biotechnol.*, 2021
- **TULADHAR, R. et al.** CRISPR-Cas9-based mutagenesis frequently provokes on-target mRNAmisregulation. *Nat. Commun.*, 2019, vol. 10, 1-10
- **GILBERT, L. A. et al.** CRISPR-mediated modular RNA-guided regulation of transcription ineukaryotes. *Cell*, 2013, vol. 154, 442
- **MORRIS, J. A. et al.** Discovery of target genes and pathways of blood trait loci using pooledCRISPR screens and single cell RNA sequencing. *bioRxiv 2021.04.07.438882*, 2021
- **YEO, N. C. et al.** An enhanced CRISPR repressor for targeted mammalian gene regulation. *Nat.Methods*, 2018, vol. 15, 611-616
- **DOENCH, J. G. et al.** Optimized sgRNA design to maximize activity and minimize off-target effectsof CRISPR-Cas9. *Nat. Biotechnol.*, 2016, vol. 34, 184-191
- **SANSON, K. R. et al.** Optimized libraries for CRISPR-Cas9 genetic screens with multiplemodalities. *Nat. Commun.*, 2018, vol. 9, 1-15
- **STOECKIUS, M. et al.** Cell Hashing with barcoded antibodies enables multiplexing and doubletdetection for single cell genomics. *Genome Biol.*, 2018, vol. 19, 1-12
- **WANG, E. et al.** Surface antigen-guided CRISPR screens identify regulators of myeloid leukemiadifferentiation. *Cell Stem Cell*, 2021, vol. 28, 718-731
- **NICOSIA, L. et al.** Pharmacological inhibition of LSD1 triggers myeloid differentiation by targetingGSE1 oncogenic functions in AML. *Oncogene*, 2021
- **FISKUS, W. et al.** Highly effective combination of LSD1 (KDM1A) antagonist and pan-histonedeacetylase inhibitor against human AML cells. *Leukemia*, 2014, vol. 28, 2155-2164
- **LENSCH, S. et al.** Dynamic spreading of chromatin-mediated gene silencing and reactivation between neighboring genes in single cells. *J. Apl. Teknol. Pangan*, 2021
- **SANJANA, N. E.** ; **SHALEM, O.** ; **ZHANG, F**. Improved vectors and genome-wide libraries for CRISPRscreening. *Nat. Methods*, 2014, vol. 11, 783-784
- **GUO, X. et al.** Transcriptome-wide Cas13 guide RNA design for model organisms and viral RNApathogens. *Cell Genomics*, 2021, vol. 1, 100001
- **MACOSKO, E. Z. et al.** Highly parallel genome-wide expression profiling of individual cells usingnanoliter droplets. *Cell*, 2015, vol. 161, 1202-1214
- **LOVE, M. I.** ; **HUBER, W.** ; **ANDERS, S**. Moderated estimation of fold change and dispersion forRNA-seq data with DESeq2. *Genome Biol*, 2014, vol. 15, 550
- **LANGMEAD, B.** ; **TRAPNELL, C.** ; **POP, M.** ; **SALZBERG, S. L**. Ultrafast and memory-efficient alignmentof short DNA sequences to the human genome. *Genome Biol*, 2009, vol. 10
- **LEEK, J. T.** ; **JOHNSON, W. E.** ; **PARKER, H. S.** ; **JAFFE, A. E.** ; **STOREY, J. D**. The sva package forremoving batch effects and other unwanted variation in high-throughput experiments. *Bioinformatics*, 2012, vol. 28, 882-883
- **KOLDE, R.** ; **LAUR, S.** ; **ADLER, P.** ; **VILO, J**. Robust rank aggregation for gene list integration andmeta-analysis. *Bioinformatics*, 2012, vol. 28, 573-580
- **HAO, Y. et al.** Integrated analysis of multi modal single-cell data. *Cell*, 2021, vol. 184, 3573-3587